# EUROPEAN PATENT SPECIFICATION

(11) **EP 0 759 087 B1**
(45) Date of publication and mention of the grant of the patent: **28.03.2001**
(21) Application number: 95920449.6
(22) Date of filing: 15.05.1995
(51) Int. Cl.: C12N 15/86, A61K 48/00

(54) **COMPOSITIONS AND METHODS FOR TARGETING GENE DELIVERY VEHICLES**
VERFAHREN UND ZUSAMMENSETZUNGEN ALS VEHIKEL ZUR ZIELGERICHTETE EINBRINGEN VON GENEN
COMPOSITIONS ET PROCEDES DE CRIBLAGE DE VECTEURS D'APPORT DE GENES

(30) Priority: 13.05.1994 US 242407
(43) Date of publication of application: 26.02.1997
(73) Proprietor: CHIRON CORPORATION, Emeryville, California 94608 (US)
(72) Inventor: JOLLY, Douglas, J., Leucadia, CA 92024 (US); BARBER, Jack, R., San Diego, CA 92129 (US); RESPESS, James, G., San Diego, CA 92109 (US); MOORE, Margaret, Dow, San Diego, CA 92117 (US)
(74) Representative: Goldin, Douglas Michael
(86) International application number: US9506084
(87) International publication number: WO9531566

(56) References cited:
- WO-A-92/06180
- WO-A-93/04701
- WO-A-93/20221

## Description

### Technical Field

The present invention relates generally to compositions and methods for targeting gene delivery vehicles, and more specifically, to compositions and methods which utilize high affinity binding pairs in order to specifically target a gene delivery vehicle to a selected target cell.

### Background of the Invention

Although many bacterial diseases can generally be easily treated with antibiotics, very few effective treatments or prophylactic measures presently exist for many viral, cancerous, and other nonbacterial diseases, such as genetic diseases. Traditional attempts to treat these diseases have employed the use of chemical drugs. In general, however, such drugs have lacked specificity and exhibited high overall toxicity.

Various methods have therefore been developed in order to treat and/or prevent viral, cancerous, and genetic diseases that previously had not been amenable to traditional therapies as well as more recent therapies such as gene therapy. For example, retroviruses, which can replicate and integrate into a host cell's genome through a DNA intermediate, have been utilized in order to deliver a foreign gene into a target cell, in order to therapeutically effect that target cell (*e.g.*, by killing the cell in the case of cancer, or by replacing a gene which is defective or not present in the cell in the case of diseases such as Adenosine Deaminase Deficiency). One difficulty with retroviruses, however, is that they generally only infect rapidly dividing cells, and moreover, are difficult to target to a selected cell type or tissue where it is desired to affect treatment.

A number of methods have been attempted in order to target viral vectors such as retroviral vectors. For example, Neda et al. (*J. Biol. Chem. 266*(22):14143-14146, 1991) chemically coupled α-lactose to viral particles, in order to produce viable viral particles capable of targeting human hepatocytes *in vitro*. Such a method, however, is of limited applicability, and has been only shown to allow the targeting of hepatocytes in tissue cultures.

Others have attempted to link antibodies (Goud et al., *Vir. 163*:251-254, 1988) or antibody fragments (Roux et al., *PNAS* 86:9070-9083, 1989; Etienne-Julan et al., *J. of Gen. Vir. 73*:3251-3255, 1992) with a viral particle, in order to target the viral particle to a specific cell type. Such methods, however, while producing binding of the retrovirus to a specific cell type did not result in the establishment of a proviral state (in Goud et al.) or resulted in only low levels of transduction (Roux et al. and Etienne et al.). Moreover, none of these references described the use of such compositions in order to target cells *in vivo.*

Other attempts have also been made to specifically target a cell type by selecting a vehicle which normally infects that cell type. For example, Shimada et al. (*J. Clin. Invest. 88*:1043-1047, 1991) developed an HIV gene transfer system in order to specifically target CD4+ T cells. One difficulty with such a system, however, is that it produced helper virus (HIV in the above case), which makes such a vector system unsuitable for the treatment of humans.

Other scientists have co-expressed the CD4 protein in-frame with the Avian Leukosis Virus transmembrane protein, or with the transmembrane protein of Murine Leukemia Virus, presumably in an attempt to target HIV infected T cells (Young et al. *Science 250*:1421, 1990). While the CD4 protein was expressed by the virus, no evidence was provided which showed that such viral particles were able to transduce target T cells.

The present invention overcomes previous difficulties of delivering and specifically targeting gene delivery vehicles, and further provides other related advantages.

### Summary of the Invention

Briefly stated, the present invention provides compositions and methods for the targeting of gene delivery vehicles.

Within various embodiments of the invention, a wide variety of targeting elements are provided, including for example, antibody and antibody fragments, bombesin, gastrin-release peptide, cell adhesion peptides, substance P, neuromedin-B, neuromedin-C, metenkephalin, EGF, alpha- and beta-TGF, neurotensin, melanocyte stimulating hormone, follicle stimulating hormone, lutenizing hormone, human growth hormone, cell surface receptors, low density lipoproteins, transferrin, erythropoietin, insulin and fibrinolytic enzymes. Other targeting elements include immune accessory molecules, which include for example, IL-1, IL-2, IL-3, IL-4, IL-5, IL-6, IL-7, IL-8, IL-9, IL-10, IL-11, IL-12, IL-13, IL-14, IL-15, α interferon, β interferon, γ interferon, GM-CSF, G-CSF and M-CSF.

A wide variety of high affinity binding pairs are also provided for use in the above-described methods, including for example, biotin/avidin, cytostatin/papain, val-phosphonate/carboxypeptidase A, 4CABP/RuBisCo, and tobacco hornworm diuretic hormone/tobacco hornworm diuretic hormone receptor, as well as antigen/antibody binding pairs.

Within other embodiments of the invention, a wide variety of gene delivery vehicles are provided. Within one embodiment, the gene delivery vehicle is a retroviral vector construct. Such retroviral vector constructs may be readily constructed from a variety of viruses, including for example, ecotropic, amphotropic, xenotropic and polytropic retroviruses (*see* WO 92/05266). Representative examples of suitable viruses include Avian Leukosis Virus, Bovine Leukemia Virus, Mink-Cell Focus-Inducing Virus, Murine Sarcoma Virus, Reticuloendotheliosis Virus, Gibbon Ape Leukemia Virus, Mason Pfizer Leukemia Virus and Rous Sarcoma Virus. Particularly preferred retroviruses include Murine Leukemia Viruses such as Abelson, Friend, Graffi, Gross, Kirsten, Harvey Sarcoma Virus, Moloney Murine Leukemia Virus and Rauscher. Within other embodiments of the invention, the gene delivery vehicle may be selected from the group consisting of poliovirus vectors, rhinovirus vectors, pox virus vectors, canary pox virus vectors, vaccinia virus vectors, influenza virus vectors, adenovirus vectors, parvovirus vectors, adeno-associated viral vectors, herpesvirus vectors, SV 40 vectors, HIV vectors, measles virus vectors, astrovirus vectors, corona virus vectors, and alphaviral vectors. The present invention also provides a variety of non-viral gene delivery vehicles, including for example, polycation condensed nucleic acids, nucleic acid expression vectors, naked DNA, and certain eukaryotic cells (*e.g.*, producer cells).

Within other embodiments, the gene delivery vehicles described above contain or include a heterologous sequence, such as an antisense or ribozyme sequence, or genes which encode one or more cytotoxic proteins, immune accessory molecules, gene products that activate a compound with little or no cytotoxicity into a toxic product, disease-associated antigens, or replacement proteins. Representative examples of cytotoxic proteins include ricin, abrin, diphtheria toxin, cholera toxin, gelonin, pokeweed, antiviral protein, tritin, Shigella toxin, and Pseudomonas exotoxin A. Representative examples of immune accessory molecules include IL-1, IL-3, IL-4, IL-5, IL-6, IL-7, IL-8, IL-9, IL-10, IL-11, IL-12, IL-13, IL-14, IL-15, α-interferon, γ-interferon, ICAM-1, ICAM-2, β-microglobin, LFA3, and HLA class I and HLA class II molecules. Representative examples of gene products which activate a compound with little or no cytotoxicity into a toxic product include HSVTK and VZVTK. Representative examples of replacement proteins include Factor VIII, ADA, HPRT, CFTCR and the LDL Receptor. Representative examples of disease-associated antigens include immunogenic portions of a virus selected from the group consisting of HBV, HCV, HPV, EBV, FeLV, FIV and HIV.

Within other aspects of the present invention, compositions are provided comprising one or more of the above-described gene delivery vehicles coupled to one member of a high affinity binding pair. Within other aspects of the present invention, compositions are provided comprising a gene delivery vehicle which is coupled to a high affinity bind pair, which is in turn coupled to a targeting element. Within preferred aspects of the present invention, the gene delivery vehicle is coupled to a member of the high affinity binding pair covalently, for example, by chemical methods. Alternatively, a member of the high affinity binding pair may be expressed directly on the exterior of the gene delivery vehicle, or, otherwise incorporated integrally into the exterior surface (*e.g.,* contained within the envelope) of the gene delivery vehicle.

These and other aspects of the present invention will become evident upon reference to the following detailed description and attached drawings. In addition, various references are set forth below which describe in more detail certain procedures or compositions (*e.g.*, plasmids, etc.), and are therefore incorporated by reference in their entirety.

### Brief Description of the Drawings

Figure 1 is a schematic illustration of pKS2+Eco57I-LTR(+).

Figure 2 is a schematic illustration of pKS2+Eco57I-LTR(-).

Figure 3 is a schematic illustration of pKS2+LTR-EcoRI.

Figure 4 is a schematic illustration of pR1.

Figure 5 is a schematic illustration of pR2.

Figure 6 is a schematic illustration of pKT1.

Figure 7 is a schematic illustration of pRI-HIVenv.

Figure 8 is a schematic illustration of pR2-HIVenv.

Figure 9 is a representative "prewobble" sequence for a MoMLV *gag/pol (see also* SEQ I.D. Nos. 11 and 12).

Figure 10 is a representative "wobble" sequence for a MoMLV *gag/pol (see also* SEQ. I.D. Nos. 9 and 10).

Figure 11 is a schematic illustration of pHCMV-PA.

Figure 12 is a schematic illustration of pCMV *gag/po/.*

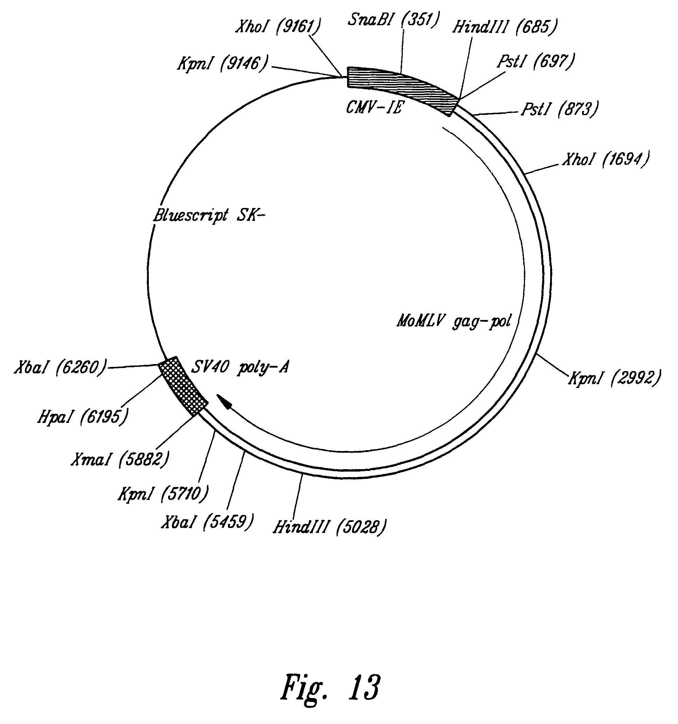

Figure 13 is a schematic illustration of pCMVgpSma.

Figure 14 is a schematic illustration of pCMVgp-X.

Figure 15 is a schematic illustration of pCMV env-X.

Figure 16 is a schematic illustration of pRgpNeo.

Figures 17A, B and C comprise a table which sets forth a variety of retroviruses which may be utilized to construct the retroviral vector constructs, *gag/pol* expression cassettes and *env* expression cassettes of the present invention.

Figure 18 is a schematic illustration of pCMV Envam-Eag-X-less.

Figure 19 is a schematic illustration of the BAG vector.

Figure 20 is a schematic illustration of the BAGΔ vector.

Figure 21 is a schematic illustration of pMLV K.

Figure 22 is a schematic illustration of pNAG1.

Figure 23 is a schematic illustration of ReNEO.

Figure 24 is a schematic illustration of KT-1.

Figure 25 is a schematic illustration of RXEN.

Figure 26 is a schematic illustration of RSEN.

Figure 27 provides a nucleotide and amino acid sequence of chicken avidin.

Figures 28A and 28B provide nucleic acid and amino acid sequences of a Murine Moloney Envelope.

Figure 29 is a schematic illustration of pCRII/N5.

Figure 30 is a schematic illustration of pCRII/A1.

Figure 31 is a schematic illustration of pCRII/B14.

Figure 32 is a schematic illustration of pCRII/C8.

Figure 33 is a schematic illustration of RXEN/N5.

Figure 34 is a schematic illustration of RXEN/A1.

Figure 35 is a schematic illustration of RXEN/B14.

Figure 36 is a schematic illustration of RXEN/C8.

### Detailed Description of the Invention

Prior to setting forth the invention, it may be helpful to an understanding thereof to first set forth definitions of certain terms that will be used hereinafter.

"Gene delivery vehicle" refers to a construct which is capable of delivering, and, within preferred embodiments expressing, one or more gene(s) or sequence(s) of interest in a host cell. Representative examples of such vehicles include viral vectors, nucleic acid expression vectors, naked DNA, and certain eukaryotic cells (*e.g.*, producer cells). Vehicles which are not considered to be within the scope of 'gene delivery vehicles' include liposomes. Preferably, gene delivery vehicles of the present invention have a molecular weight of greater than about x kilodaltons, wherein x is selected from the group consisting of 50, 100, 150, 200, 250, 300, 400, 500, 600, 700, 800, 900, 1,000, 1,500, 2,000, 2,500, 3,000, 4,000, and 5,000. Within particularly preferred embodiments of the invention, the gene delivery vehicle includes a member of the high affinity binding pair (discussed below), either expressed on, or included as, an integral part of the exterior of the gene delivery vehicle.

"High Affinity Binding Pair" refers to a set a molecules which is capable of binding one another with a K_{D} of less than 10^{-y}M, wherein y is selected from the group consisting of 8, 9, 10, 11, 12, 13, 14 and 15. As utilized herein, the "K_{D}" refers to the disassociation constant of the reaction A + B 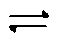 AB, wherein A and B are members of the high affinity binding pair. (In addition, as should be understood by one of ordinary skill in the art, as the affinity of the two molecules increases, K_{D} decreases.) Affinity constants may be readily determined by a variety of techniques, including for example by a Scatchard analysis *(see* Scatchard, *Ann. N.Y. Acad. Sci. 51*:660-672, 1949). Representative examples of suitable affinity binding pairs include biotin/avidin, cytostatin/papain, phosphonate/ carboxypeptidase A, and 4CABP/RuBisCo.

"Targeting element" refers to a molecule which is capable of specifically binding a selected cell type. As utilized within the context of the present invention, targeting elements are considered to specifically bind a selected cell type when a biological effect of the coupled targeting element may be seen in that cell type, or, when there is greater than a 10 fold difference, and preferably greater than a 25, 50 or 100 fold difference between the binding of the coupled targeting element to target cells and non-target cells. Generally, it is preferable that the targeting element bind to the selected cell type with a K_{D} of less than 10⁻⁵M, preferably less than 10⁻⁶M, more preferably less than 10⁻⁷M, and most preferably less than 10⁻⁸M (as determined by a Scatchard analysis, *see* Scatchard, *Ann. N.Y. Acad. Sci. 51*:660-672, 1949). In addition it is generally preferred that the targeting element bind to the selected cell type with an affinity of at least 1 log (*i.e.,* 10 times) less than the affinity constant of the high affinity binding pair. (In other words, the K_{D} value will be at least I log or 10 fold greater.) Suitable targeting elements are preferably non-immunogenic, not degraded by proteolysis, and not scavenged by the immune system. Particularly preferred targeting elements (which are conjugated to a member of the high affinity binding pair) should have a half-life (in the absence of a clearing agent) within an animal of between 10 minutes and 1 week. Representative examples of suitable targeting elements are set forth below in more detail.

"Clearing agent" refers to molecules which can bind and/or cross-link circulating coupled targeting elements. Preferably, the clearing agent is non-immunogenic, specific to the coupled targeting element, and large enough to avoid rapid renal clearance. In addition, the clearing agent is preferably not degraded by proteolysis, and not scavenged by the immune system. Particularly preferred clearing agents for use within the present invention include those which bind to the coupled targeting element at a site other than the affinity binding member, and most preferably, which bind in a manner that blocks the binding of the targeting element to its target. Numerous cleaving agents may be utilized within the context of the present invention, including for example those described by Marshall et al. in *Brit. J. Cancer 69*:502-507, 1994.

"Retroviral vector construct" refers to an assembly which is, within preferred embodiments of the invention, capable of directing the expression of a sequence(s) or gene(s) of interest. Preferably, the retroviral vector construct should include a 5' LTR, a tRNA binding site, a packaging signal, one or more heterologous sequences, an origin of second strand DNA synthesis and a 3' LTR. A wide variety of heterologous sequences may be included within the vector construct, including for example, sequences which encode a protein (*e.g.*, cytotoxic protein, disease-associated antigen, immune accessory molecule, or replacement protein), or which are useful as a molecule itself (*e.g.,* as a ribozyme or antisense sequence). Alternatively, the heterologous sequence may merely be a "stuffer" or "filler" sequence, which is of a size sufficient to allow production of viral particles containing the RNA genome. Preferably, the heterologous sequence is at least 1, 2, 3, 4, 5, 6, 7 or 8 kB in length.

The retroviral vector construct may also include transcriptional promoter/enhancer or locus defining element(s), or other elements which control gene expression by means such as alternate splicing, nuclear RNA export, post-translational modification of messenger, or post-transcriptional modification of protein. Optionally, the retroviral vector construct may also include selectable markers such as Neo, TK, hygromycin, phleomycin, histidinol, or DHFR, as well as one or more specific restriction sites and a translation termination sequence.

"Nucleic Acid Expression Vector" refers to an assembly which is capable of directing the expression of a sequence or gene of interest. The nucleic acid expression vector must include a promoter which, when transcribed, is operably linked to the sequence(s) or gene(s) of interest, as well as a polyadenylation sequence. Within certain embodiments of the invention, the nucleic acid expression vectors described herein may be contained within a plasmid construct. In addition to the components of the nucleic acid expression vector, the plasmid construct may also include a bacterial origin of replication, one or more selectable markers, a signal which allows the plasmid construct to exist as single-stranded DNA (e.g., a M13 origin of replication), a multiple cloning site, and a "mammalian" origin of replication (*e.g.*, a SV40 or adenovirus origin of replication).

### A. TARGETING ELEMENTS

A wide variety of targeting elements may be utilized within the context of the present invention, in order to specifically direct a gene delivery vehicle to a selected cell type. Generally, targeting elements are proteins or peptides, although other non-proteinaceous molecules may also function as targeting elements. For example, within one embodiment of the invention, antibodies may be utilized in order to target a selected cell type (*see* generally, Wilchek and Bayer, *Anal. Biochem 171*:1-32, 1988). Representative examples include anti-CD34 antibodies (*e.g.*, 12.8 (Andrews et al., *Blood 67*:842, 1986), and MylO (Civin et al., *J. Immunol. 133*:157, 1984; commercially available from Becton Dickinson under the designation HPCA-2)) which may be utilized to target the anti-CD34 antigen on stem cells, the anti-CD4 antibody which may be utilized to target CD4+ T-cells, anti-CD8 antibodies to target CD8+ cells, the HER2/neu monoclonal antibody 4D5 (Sarup et al., *Growth Regul. 1*:72-82, 1991) to target ovarian and breast cells, the c-erbB-2 monoclonal antibody GFD-OA-p185-1 (Alper et al., *Cell Growth Differ. 1*:591-9, 1990) to target breast cells, the TAG72 monoclonal Ab: CC49 and B72.3 (King et al., *J. Biochem. 281*:317-23, 1992) to target colon and breast cells, and the carcinoembryonic antigen monoclonal antibody ZCE025 (Nap et al., *Canc. Res. 52*:2329-39, 1992) to target colon carcinoma cells.

Other suitable targeting elements include hormones and hormone receptors. Representative examples include follicle stimulating hormone and lutenizing hormone to ovary and testes cells, melanocyte stimulating hormone and epidermal growth factor to epidermal cells, and human growth hormone to mostly bone cells and skeletal muscle cells.

Within other embodiments, immune accessory molecules may be utilized to target specific receptors on various cells. Examples include interferon targeted to macrophages and natural killer cells, interleukins to T-lymphocytes, and erythropoietin and CSF to bone marrow cells.

Within still other embodiments, peptides such as substance P may target neurons as a mediator of pain signals, neuromedin (Conlon, *J. Neurochem. 51*:988, 1988) may be utilized to target the cells of the uterus for contractile activity and proteins corresponding to ligands for known cell surface receptors such as insulin may be utilized to target insulin receptors on cells for glucose regulation.

Within yet other embodiments, other ligands and antibodies may be utilized to target selected cell types, including for example: monoclonal antibody c-SF-25 to target a 125kD antigen on human lung carcinoma (Takahashi et al, *Science 259*:1460, 1993); antibodies to various lung cancer antigens (Souhami, *Thorax 47*:53-56, 1992); antibodies to human ovarian cancer antigen 14C1 (Gallagher et al., *Br. J*. *Cancer 64*:35-40, 1991); antibodies to H/Le^{y}/Le^{b} antigens to target lung carcinoma (Masayuki et al., *N. Eng. J. Med 327*:14-18, 1992); nerve growth factor to target nerve growth factor receptors on neural tumors (Chao et al., *Science 232*:518, 1986); the Fc receptor to target macrophages (Anderson and Looney, *Immun. Today 1*:264-266, 1987); lectins (Sharon and Lis, *Science* 246:227, 1989); collagen type I to target colon cancer (Pullam and Bodmer, *Nature 356*:529, 1992); Interleukin-1 to target the Interleukin-1 receptor on T cells (Fanslow et al., *Science* 248:739, 1990); acetylated low density lipoproteins ("LDL") to target macrophage scavenger receptors (and atherosclerotic plaques; *see* Brown et al., *Ann. Rev. Biochem 52*:223-261, 1983), as well as other acetylated molecules which target macrophage scavenger receptors (Paulinski et al., *PNAS 86*:1372-1376, 1989); viral receptors (Haywood, *J. Vir. 68*(1):1-5, 1994); transferrin to target transferrin receptors on tumor cells (Huebers et al., *Physio. Rev. 67*:520, 582, 1987); vasoendothelial growth factor ("vegF") to target cells where increased vascularization occurs; and urokinase plasminogen activator receptor (UPAR).

Alternatively, ligands may be selected from libraries created utilizing recombinant techniques (Scott and Smith, *Science 249*:386, 1990; Devlin et al., *Science 249:404,* 1990; Houghten et al., *Nature 354*:84 1991; Matthews and Wells, *Science 260*:1113,1993; Nissim et al., *EMBO J. 13*(3):692-698, 1994), or equivalent techniques utilizing organic compound libraries.

### B. HIGH AFFINITY BINDING PAIRS

In addition to targeting elements, the present invention also provides a wide variety of high affinity binding pairs. Representative examples of suitable affinity binding pairs include biotin/avidin with an affinity (K_{D}) of 10⁻¹⁵ M (Richards, *Meth. Enz. 184*:3-5, 1990; Green, *Adv. in Protein Chem. 29*:85, 1985); cytostatin/papain with an affinity of 10⁻¹⁴ M (Bjork and Ylinenjarvi, *Biochemistry 29*:1770-1776, 1990); val-phosponate/carboxypeptidase A with an affinity of 10⁻¹⁴ M (Kaplan and Bartlett, *Biochemistry 30*:8165-8170, 1991); 4CABP-RuBisCo with an affinity of 10⁻¹³ M, (Schloss, *J. Biol. Chem. 263*:4145-4150, 1988); and tobacco hornworm diuretic hormone/tobacco hornworm diuretic hormone receptor, with an affinity of 10⁻¹¹M (Reagan et *al., Arch. Insect Biochem. Physiol. 23*:135-145, 1993).

A wide variety of other high affinity binding pairs may also be developed, for example, by preparing and selecting antibodies which recognize a selected antigen, and by further screening of such antibodies in order to select those with a high affinity *(see* generally, U.S. Patent Nos. RE 32,011, 4,902,614, 4,543,439, and 4,411,993; *see also, Monoclonal Antibodies, Hybridomas: A New Dimension in Biological Analyses,* Plenum Press, Kennett, McKearn, and Bechtol (eds.), 1980, and *Antibodies: A Laboratory Manual,* Harlow and Lane (eds.), Cold Spring Harbor Laboratory Press, 1988). Alternatively, antibodies or antibody fragments may also be produced and selected utilizing recombinant techniques *(see* William D. Huse et al., "Generation of a Large Combinational Library of the Immunoglobulin Repertoire in Phage Lambda," *Science 246*:1275-1281, December 1989; *see also* L. Sastry et al., "Cloning of the Immunological Repertoire in *Escherichia coli* for Generation of Monoclonal Catalytic Antibodies: Construction of a Heavy Chain Variable Region-Specific cDNA Library," *Proc. Nail. Acad. Sci. USA 86*:5728-5732, August 1989; *see also* Michelle Alting-Mees et al., "Monoclonal Antibody Expression Libraries: A Rapid Alternative to Hybridomas," *Strategies in Molecular Biology 3*:1-9, January 1990; these references describe a commercial system available from Stratacyte, La Jolla, California, which enables the production of antibodies through recombinant techniques).

As will be evident to one of ordinary skill in the art given the disclosure provided herein, either member (or molecule) of the affinity binding pair may be coupled to the gene delivery vehicle (or conversely, the targeting element). Nevertheless, within preferred embodiments of the invention, the larger of the two affinity binding pairs (e.g., avidin of the avidin/biotin pair) is coupled to gene delivery vehicle.

### C. GENE DELIVERY VEHICLES

### 1. Construction of retroviral gene delivery vehicles

Within one aspect of the present invention, retroviral vector constructs are provided which are constructed to carry or express a selected gene(s) or sequence(s) of interest. Numerous retroviral gene delivery vehicles may be utilized within the context of the present invention, including for example EP 0,415,731; WO 90/07936; WO 91/0285, WO 9403622; WO 9325698; WO 9325234; U.S. Patent No. 5,219,740; WO 9311230; WO 9310218; Vile and Hart, *Cancer Res. 53*:3860-3864, 1993; Vile and Hart, *Cancer Res.* *53*:962-967, 1993; Ram et al., *Cancer Res. 55*:83-88, 1993; Takamiya et al., *J. Neurosci. Res.* 33:493-503, 1992; Baba et al., *J. Neurosurg. 79*:729-735, 1993 (U.S. Patent No. 4,777,127, GB 2,200,651, EP 0,345,242 and WO91/02805).

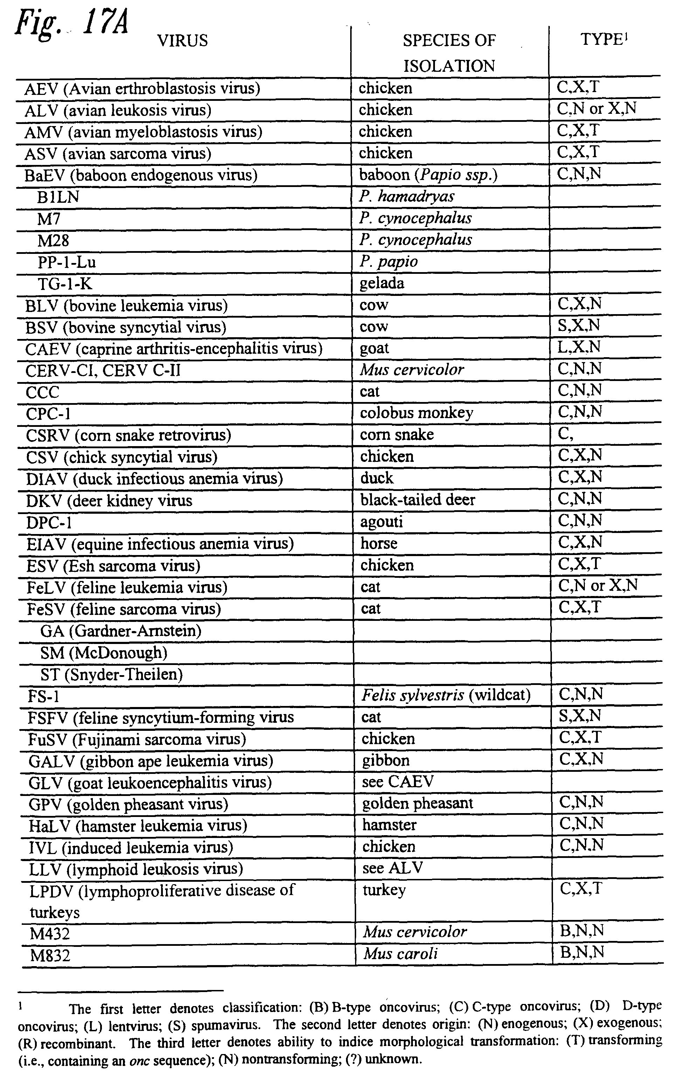

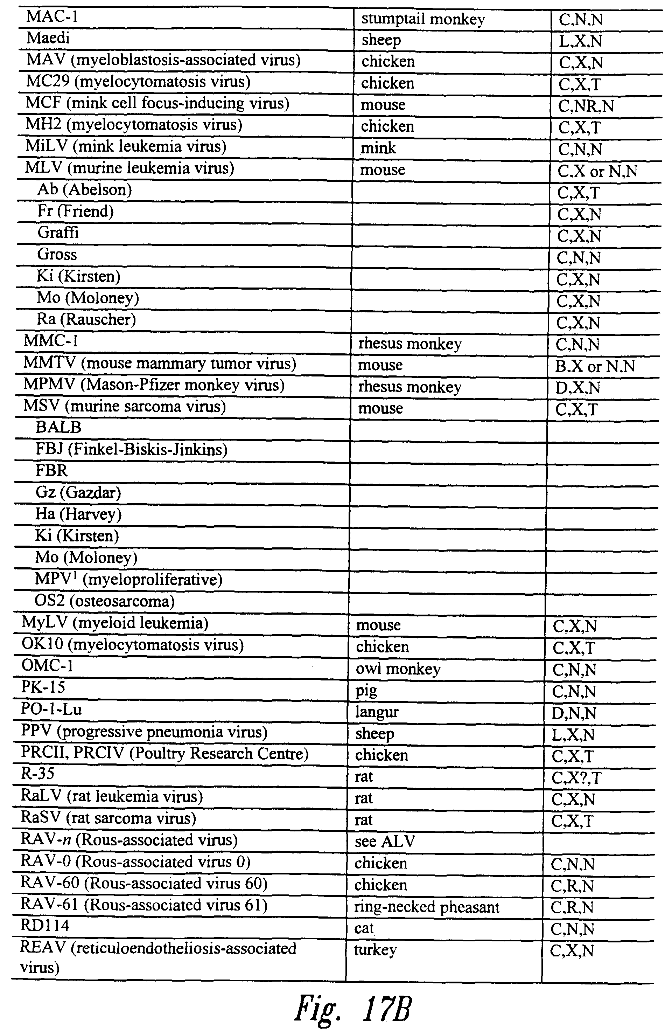

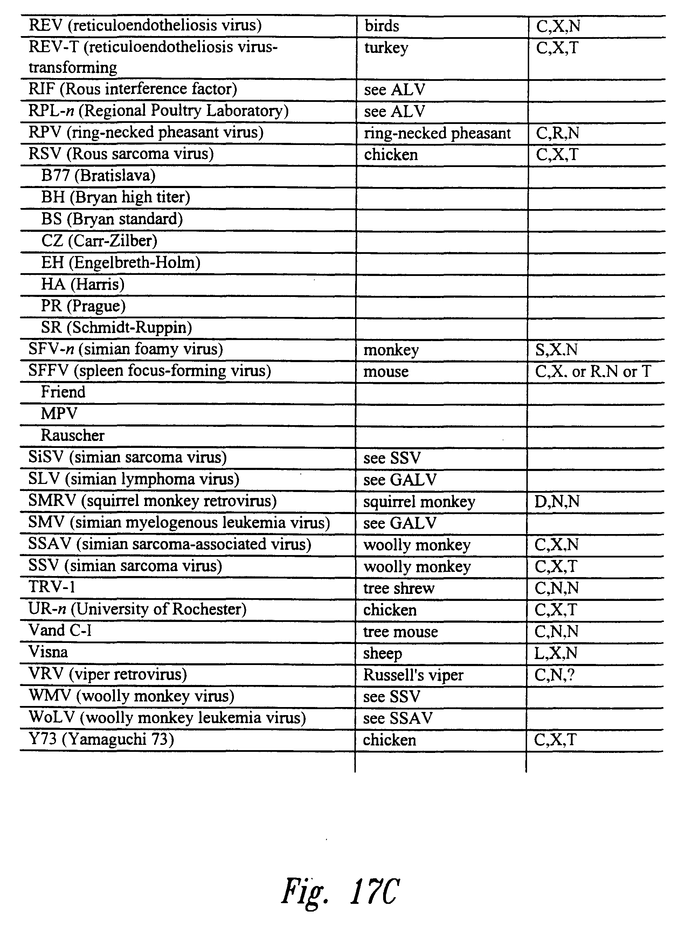

Retroviral gene delivery vehicles of the present invention may be readily constructed from a wide variety of retroviruses, including for example, B, C, and D type retroviruses as well as spumaviruses and lentiviruses (*see* RNA Tumor Viruses, Second Edition, Cold Spring Harbor Laboratory, 1985). Briefly, viruses are often classified according to their morphology as seen under electron microscopy. Type "B" retroviruses appear to have an eccentric core, while type "C" retroviruses have a central core. Type "D" retroviruses have a morphology intermediate between type B and type C retroviruses. Representative examples of suitable retroviruses include those set forth below in Figures 17A, B and C *(see* RNA Tumor Viruses at pages 2-7), as well as a variety of xenotropic retroviruses (e.g., NZB-X1, NZB-X2 and NZB₉₋₁ (*see* O'Neill et al., *J. Vir. 53*:100-106, 1985)) and polytropic retroviruses *(e.g.,* MCF and MCF-MLV (*see* Kelly et al., *J. Vir. 45*(1):291-298, 1983)). Such retroviruses may be readily obtained from depositories or collections such as the American Type Culture Collection ("ATCC"; Rockville, Maryland), or isolated from known sources using commonly available techniques.

Particularly preferred retroviruses for the preparation or construction of retroviral gene delivery vehicles of the present invention include retroviruses selected from the group consisting of Avian Leukosis Virus, Bovine Leukemia Virus, Murine Leukemia Virus, Mink-Cell Focus-Inducing Virus, Murine Sarcoma Virus, Reticuloendotheliosis virus and Rous Sarcoma Virus. Particularly preferred Murine Leukemia Viruses include 4070A and 1504A (Hartley and Rowe, *J. Virol. 19*:19-25, 1976), Abelson (ATCC No. VR-999), Friend (ATCC No. VR-245), Graffi, Gross (ATCC No. VR-590), Kirsten, Harvey Sarcoma Virus and Rauscher (ATCC No. VR-998), and Moloney Murine Leukemia Virus (ATCC No. VR-190). Particularly preferred Rous Sarcoma Viruses include Bratislava, Bryan high titer *(e.g.,* ATCC Nos. VR-334, VR-657, VR-726, VR-659, and VR-728), Bryan standard, Carr-Zilber, Engelbreth-Holm, Harris, Prague (*e.g.*, ATCC Nos. VR-772, and 45033), and Schmidt-Ruppin *(e.g.* ATCC Nos. VR-724, VR-725, VR-354).

Any of the above retroviruses may be readily utilized in order to assemble or construct retroviral gene delivery vehicles given the disclosure provided herein, and standard recombinant techniques (*e.g.*, Sambrook et al, *Molecular Cloning: A Laboratory Manual,* 2d ed., Cold Spring Harbor Laboratory Press, 1989; Kunkle, *PNAS* 82:488, 1985). In addition, within certain embodiments of the invention, portions of the retroviral gene delivery vehicles may be derived from different retroviruses. For example, within one embodiment of the invention, retroviral LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

Within one aspect of the present invention, retroviral vector constructs are provided comprising a 5' LTR, a tRNA binding site, a packaging signal, one or more heterologous sequences, an origin of second strand DNA synthesis and a 3' LTR, wherein the vector construct lacks *gag/pol* or *env* coding sequences. Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTRs may be readily identified in the provirus due to their precise duplication at either end of the genome.

The tRNA binding site and origin of second strand DNA synthesis are also important for a retrovirus to be biologically active, and may be readily identified by one of skill in the art. For example, retroviral tRNA binds to a tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5' LTR. Similarly, the origin of second strand DNA synthesis is, as its name implies, important for the second strand DNA synthesis of a retrovirus. This region, which is also referred to as the poly-purine tract, is located just upstream of the 3' LTR.

In addition to a 5' and 3' LTRs, tRNA binding site, and origin of second strand DNA synthesis, certain preferred retroviral vector constructs which are provided herein also comprise a packaging signal, as well as one or more heterologous sequences, each of which is discussed in more detail below.

Within one aspect of the invention, retroviral vector constructs are provided which lack both *gag/pol* and *env* coding sequences. As utilized within the context of the present invention, a packaging signal should be understood to refer to that sequence of nucleotides which is not required for synthesis, processing or translation of viral RNA or assembly of virions, but which is required in *cis* for encapsidation of genomic RNA *(see* Mann et al., *Cell 33*:153-159, 1983; RNA Tumor Viruses, Second Edition, *supra*). Further, as utilized herein, the phrase "lacks *gag/pol* or *env* coding sequences" should be understood to refer to retrovectors which contain less than 20, preferably less than 15, more preferably less than 10, and most preferably less than 8 consecutive nucleotides which are found *in gag/pol* or *env* genes, and in particular, within *gag/pol* or *env* expression cassettes that are used to construct packaging cell lines for the retroviral vector construct. Representative examples of such retroviral vector constructs are set forth in more detail below and in Example 1.

As an illustration, within one embodiment of the invention construction of retroviral vector constructs which lack *gag/pol* or *env* sequences may be accomplished by preparing retroviral vector constructs which lack an extended packaging signal. As utilized herein, the phrase "extended packaging signal" refers to a sequence of nucleotides beyond the minimum core sequence which is required for packaging, that allows increased viral titer due to enhanced packaging. As an example, for the Murine Leukemia Virus MoMLV, the minimum core packaging signal is encoded by the sequence (counting from the 5' LTR cap site) from approximately nucleotide 144 of SEQ. I.D. No. 1, up through the *Pst* I site (nucleotide 567 of SEQ. I.D. No. 1). The extended packaging signal of MoMLV includes the sequence beyond nucleotide 567 up through the start of the *gag/pol* gene (nucleotide 621), and beyond nucleotide 1040. Thus, within this embodiment retroviral vector constructs which lack extended packaging signal may be constructed from the MoMLV by deleting or truncating the packaging signal downstream of nucleotide 567.

Within other embodiments of the invention, retroviral vector constructs are provided wherein the packaging signal that extends into, or overlaps with, retroviral *gag/pol* sequence is deleted or truncated. For example, in the representative case of MoMLV, the packaging signal is deleted or truncated downstream of the start of the *gag/pol* gene (nucleotide 621 of SEQ ID NO: 1). Within preferred embodiments of the invention, the packaging signal is terminated at nucleotide 570, 575, 580, 585, 590, 595, 600, 610, 615 or 617 of SEQ ID NO: 1.

Within other aspects of the invention, retroviral vector constructs are provided which include a packaging signal that extends beyond the start of the *gag/pol* gene (*e.g.*, for MoMLV, beyond nucleotide 621 of SEQ ID NO: 1). When such retroviral vector constructs are utilized, it is preferable to utilize packaging cell lines for the production of recombinant viral particles wherein the 5' terminal end of the *gag/pol* gene in a *gag/pol* expression cassette has been modified to contain codons which are degenerate for *gag.* Such *gag/pol* expression cassettes are described in more detail below in section 2, and in Example 3.

Within other aspects of the present invention, retroviral vector constructs are provided comprising a 5' LTR, a tRNA binding site, a packaging signal, an origin of second strand DNA synthesis and a 3' LTR, wherein the retrovector plasmid construct does not contain a retroviral nucleic acid sequence upstream of the 5' LTR. As utilized within the context of the present invention, the phrase "does not contain a retroviral nucleic acid sequence upstream of the 5' LTR" should be understood to mean that the retrovector plasmid construct contains less than 20, preferably less than 15, more preferably less than 10, and most preferably less than 8 consecutive nucleotides which are found in a retrovirus, and more specifically, in a retrovirus which is homologous to the retroviral vector construct, upstream of and/or contiguous with the 5' LTR. Within preferred embodiments, the retrovector plasmid constructs do not contain an *env* coding sequence (as discussed below) upstream of the 5' LTR. A particularly preferred embodiment of such retrovector plasmid constructs is set forth in more detail below in Example 1.

Within a further aspect of the present invention, retrovector plasmid constructs are provided comprising a 5' LTR, a tRNA binding site, a packaging signal, an origin of second strand DNA synthesis and a 3' LTR, wherein the retrovector plasmid construct does not contain a retroviral packaging signal sequence downstream of the 3' LTR. As utilized herein, the term "packaging signal sequence" should be understood to mean a sequence sufficient to allow packaging of the RNA genome. A representative example of such a retroviral vector construct is set forth in more detail below in Example 1.

Packaging cell lines suitable for use with the above-described retroviral vector constructs may be readily prepared (*see* U.S. Serial No. 08/240,030, filed May 9, *1994; see also* WO 92/05266), and utilized to create producer cell lines (also termed vector cell lines or "VCLs") for the production of recombinant vector particles.

In particular, a variety of *gag/pol* expression cassettes are provided which, in combination with the retroviral vector constructs and *env* expression cassettes also described herein, enable the construction of packaging cell lines and producer cell lines which preclude the formation of replication competent virus. Briefly, retroviral *gag/pol* genes contain a *gag* region which encodes a variety of structural proteins that make up the core matrix and nucleocapsid, and a pol region which contains genes which encode (1) a protease for the processing of *gag/pol* and *env* proteins, (2) a reverse transcriptase polymerase, (3) an RNase H, and (4) an integrase, which is necessary for integration of the retroviral provector into the host genome. Although retroviral *gag/pol* genes may be utilized to construct the *gag/pol* expression cassettes of the present invention, a variety of other non-retroviral (and non-viral) genes may also be utilized to construct the *gag/pol* expression cassette. For example, a gene which encodes retroviral RNase H may be replaced with genes which encode bacterial (e.g., *E. coli* or *Thermus thermophilus*) RNase H. Similarly, a retroviral integrase gene may be replaced by other genes with similar function (*e.g.*, yeast retrotransposon TY3 integrase).

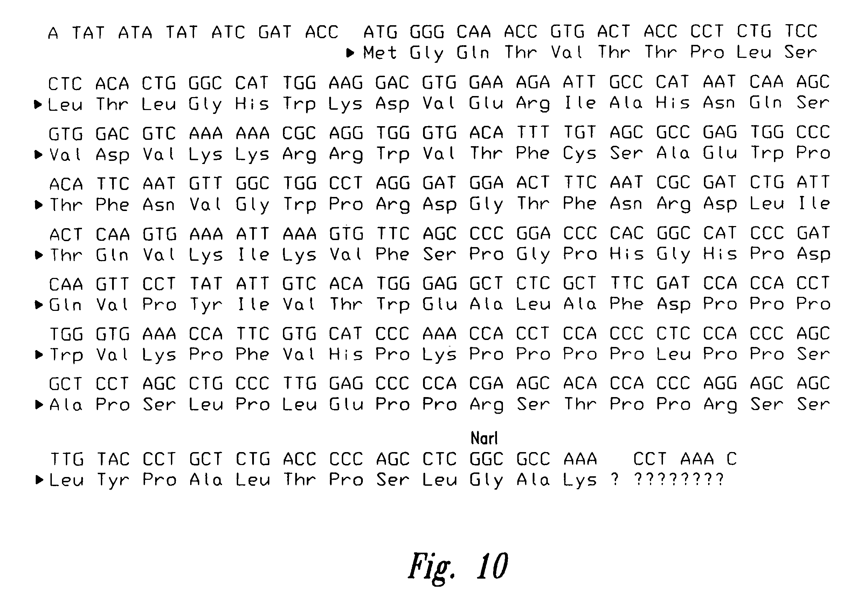

Within one aspect of the invention, *gag/pol* expression cassettes are provided comprising a promoter operably linked to a *gag/pol* gene, and a polyadenylation sequence, wherein the *gag/pol* gene has been modified to contain codons which are degenerate for gag. Briefly, as noted above, in wild-type retrovirus the extended packaging signal of the retrovirus overlaps with sequences which encode gag and pol. Thus, in order to eliminate the potential of crossover between the retroviral vector construct and the *gag/pol* expression cassette, as well as to eliminate the possiblity of co-encapsidation of the *gag/pol* expression cassette and replication competent virus or retroviral vector constructs, sequences of overlap should be eliminated. Within one embodiment of the invention, elimination of such overlap is accomplished by modifying the *gag/pol* gene (and more specifically, regions which overlap with the retroviral vector construct, such as the extended packaging signal) to contain codons that are degenerate *(i.e.,* that "wobble") for gag. In particular, within preferred embodiments of the invention codons are selected which encode biologically active gag/pol protein (*i.e.*, capable of producing a competent retroviral particle, in combination with an *env* expressing element, and a RNA genome), and which lack any packaging signal sequence, including in particular, extended packaging signal sequence. As utilized herein, the phrase "lacks any retroviral packaging signal sequence" should be understood to mean that the *gag/pol* expression cassette contains less than 20, preferably less than 15, more preferably less than 10, and most preferably less than 8 consecutive nucleotides which are identical to a sequence found in a retroviral packaging signal *(e.g.,* in the case of MoMLV, extending up and through the *Xho* I site at approximately nucleotide number 1561). A particularly preferred example of such modified codons which are degenerate for gag is shown in Figure 10, and in Example 3, although the present invention should not be so limited. In particular, within other embodiments, at least 25, 50, 75, 100, 125 or 135 *gag* codons are modified or "wobbled" from the native *gag* sequence within the *gag/pol* expression cassettes of the present invention.

In addition to eliminating overlap between the retroviral vector construct and the *gag/pol* gene, it is also preferable to eliminate any potential overlap between the *gag/pol* gene and the *env* gene in order to prohibit the possibility of homologous recombination. This may be accomplished in at least two principal ways: (1) by deleting a portion of the *gag/pol* gene which encodes the integrase protein, and in particular, that portion of the gene which encodes the integrase protein which overlaps with the *env* coding sequence, or (2) by selecting codons which are degenerate for integrase and/or env.

Thus, within one aspect of the present invention *gag/pol* expression cassettes are provided comprising a promoter operably linked to a *gag/pol* gene, and a polyadenylation sequence or signal, wherein a 3' terminal end of the gene has been deleted without effecting the biological activity of the integrase. (The biological activity of integrase may be readily determined by detection of an integration event, either by DNA analysis or by expression of a transduced gene; *see* Roth et al., *J. Vir. 65*(4):2141-2145, 1991.) As an example, in the Murine Leukemia Virus MoMLV (SEQ ID. NO. 1), the *gag/pol* gene is encoded by nucleotides 621 through 5834. Within this sequence, the protein integrase is encoded by nucleotides 4610 through nucleotide 5834. A portion of the *gag/pol* sequence which encodes integrase also encodes env (which begins at nucleotide 5776). Thus, within one embodiment of the invention, the 3' terminal end of the *gag/pol* gene is deleted or truncated in order to prevent crossover with the *env* gene, without effecting the biological activity of the integrase. Within other preferred embodiments, the *gag/pol* gene is deleted at any nucleotide downstream (3') from the beginning of the integrase coding sequence, and preferably prior to the start of the *env* gene sequence. Within one embodiment, the sequence encoding gag/pol is a MoMLV sequence, and the *gag/pol* gene is deleted at any nucleotide between nucleotides 4610 and 5576 (of SEQ. I.D. No. 1), including for example, at nucleotides 5775, 5770, 5765, 5760, 5755, 5750.

Within other embodiments of the invention, the *gag/pol* expression cassette contains sequences encoding gag/pol (and including integrase), while lacking any sequence found in an *env* gene. The phrase "lacking any sequence found in an *env* gene" should be understood to mean that the *gag/pol* expression cassette does not contain at least 20, preferably at least 15, more preferably at least 10, and most preferably less than 8 consecutive nucleotides which are identical to an *env* sequence, and preferably which are found in an *env* expression cassette which will be utilized along with the *gag/pol* expression cassette to form a packaging cell. Such expression cassettes may be readily prepared by selecting codons which are degenerate for integrase, and which do not encode biologically active env. *(See Morgenstern and Land, Nuc. Acids Res. 18*:3587-3596, 1990.)

Within other embodiments of the invention, the *gag/pol* expression cassette contains a heterologous promoter, and/or heterologous polyadenylation sequence. As utilized herein, "heterologous" promoters or polyadenylation sequences refers to promoters or polyadenylation sequences which are from a different source from which the *gag/pol* gene (and preferably the *env* gene and retroviral vector construct) is derived from. Representative examples of suitable promoters include the Cytomegalovirus Immediate Early ("CMV IE") promoter, the Herpes Simplex Virus Thymidine Kinase ("HSVTK") promoter, the Rous Sarcoma Virus ("RSV") promoter, the Adenovirus major-late promoter and the SV 40 promoter. Representative examples of suitable polyadenylation signals include the SV 40 late polyadenylation signal and the SV40 early polyadenylation signal.

Within preferred aspects of the present invention, *gag/pol* expression cassettes such as those described above will not co-encapsidate along with a replication competent virus.

Within related aspects, *env* expression cassettes are provided which, in combination with *the gag/pol* expression cassettes and retroviral vector constructs described above, preclude formation of replication competent virus by homologous recombination, as well as to confer a particular specificity of the resultant vector particle (*e.g.*, amphotropic, ecotropic, xenotropic or polytropic; *see* Figure 17, as well as the discussion above). Briefly, in a wild-type retrovirus the *env* gene encodes two principal proteins, the surface glycoprotein "SU" and the transmembrane protein "TM", which are translated as a polyprotein, and subsequently separated by proteolytic cleavage. Representative examples of the SU and TM proteins are the gp120 protein and gp41 protein in HIV, and the gp70 protein and pl5e protein in MoMLV. In some retroviruses, a third protein designated the "R" peptide" of undetermined function, is also expressed from the *env* gene and separated from the polyprotein by proteolytic cleavage. In the Murine Leukemia Virus MoMLV, the R peptide is designated "p2".

A wide variety of *env* expression cassettes may be constructed given the disclosure provided herein. Within one aspect, *env* expression cassettes are provided comprising a promoter operably linked to an *env* gene, wherein no more than 6, 8, 10, 15, or 20 consecutive retroviral nucleotides are included upstream (5') of and/or contiguous with said *env* gene. Within other aspects of the invention, *earn* expression cassettes are provided comprising a promoter operably linked to an *env* gene, wherein the *env* expression cassette does not contain a consecutive sequence of greater than 20, preferably less than 15, more preferably less than 10, and most preferably less than 8 or 6 consecutive nucleotides which are found in a *gag/pol* gene, and in particular, in a *gag/pol* expression cassette that will be utilized along with the env expression cassette to create a packaging cell line.

Within another aspect, *env* expression cassettes are provided comprising a promoter operably linked to an *env* gene, and a polyadenylation sequence, wherein a 3' terminal end of the *env* gene has been deleted without effecting the biological activity of env. As utilized herein, the phrase "biological activity of env" refers to the ability of envelop protein to be expressed on the surface of a virus or vector particle, and to allow for a successful infection of a host cell. One practical method for assessing biological activity is to transiently transfect the *env* expression cassette into a cell containing a previously determined functional *gag/pol* expression cassette, and a retroviral vector construct which expresses a selectable marker. A biologically functional *env* expression cassette will allow vector particles produced in that transfected cell, to transmit the selectable marker to a naive sensitive cell such that it becomes resistant to the marker drug selection. Within a preferred embodiment of the invention, the 3' terminal end of the *env* gene is deleted or truncated such that a complete R peptide is not produced by the expression cassette. In the representative example of MoMLV, sequence encoding the R peptide (which begins at nucleotide 7734) is deleted, truncated, or, for example, terminated by insertion of a stop codon at nucleotide 7740, 7745, 7747, 7750, 7755, 7760, 7765, 7770, 7775, 7780, or any nucleotide in between.

Within yet another aspect, *env* expression cassettes are provided which contain a heterologous promoter, and/or heterologous polyadenylation sequence. As utilized herein, "heterologous" promoters or polyadenylation sequences refers to promoters or polyadenylation sequences which are from a different source from which the *gag/pol* gene (and preferably the *env* gene and retroviral vector construct) is derived from. Representative examples of suitable promoters include the CMV IE promoter, the HSVTK promoter, the RSV promoter, the Adenovirus major-late promoter and the SV 40 promoters. Representative examples of suitable polyadenylation signals include the SV 40 late polyadenylation signal and the SV40 early polyadenylation signal.

### 2. Alphavirus delivery vehicles

The present invention also provides a variety of Alphavirus vectors which may function as gene delivery vehicles. For example, the Sindbis virus is the prototype member of the alphavirus genus of the togavirus family. The unsegmented genomic RNA (49S RNA) of Sindbis virus is approximately 11,703 nucleotides in length, contains a 5' cap and a 3' polyadenylated tail, and displays positive polarity. Infectious enveloped Sindbis virus is produced by assembly of the viral nucleocapsid proteins onto the viral genomic RNA in the cytoplasm and budding through the cell membrane embedded with viral encoded glycoproteins. Entry of virus into cells is by endocytosis through clatharin coated pits, fusion of the viral membrane with the endosome, release of the nucleocapsid, and uncoating of the viral genome. During viral replication the genomic 49S RNA serves as template for synthesis of the complementary negative strand. This negative strand in turn serves as template for genomic RNA and an internally initiated 26S subgenomic RNA. The Sindbis viral nonstructural proteins are translated from the genomic RNA while structural proteins are translated from the subgenomic 26S RNA. All viral genes are expressed as a polyprotein and processed into individual proteins by post translational proteolytic cleavage. The packaging sequence resides within the nonstructural coding region, therefore only the genomic 49S RNA is packaged into virions.

Several different Alphavirus vector systems may be constructed and utilized within the present invention. Representative examples of such systems include those described within U.S. Patent Nos. 5,091,309 and 5,217,879, PCT Publication WO 92/10578, and U.S. Serial Nos. 08/405,627 and 08/404,796.

Particularly preferred Alphavirus vectors for use within the present invention include those which are described within WO 94/10469. Briefly, within one embodiment, Alphavirus constructs are provided comprising a 5' sequence which is capable of initiating transcription of an Alphavirus, a nucleotide sequence encoding Alphavirus non-structural proteins, an Alphavirus viral junction region which may, in certain embodiments, be inactivated such that viral transcription of the subgenomic fragment is prevented or modified such that viral transcription is reduced, and a Sindbis RNA polymerase recognition sequence.

Within yet another aspect, eukaryotic layered vector initiation systems may be utilized as a gene delivery vehicle. Such systems generally comprise a 5' promoter, a construct which is capable of expressing a heterologous nucleotide sequence that is capable of replication in a cell either autonomously or in response to one or more factors, and a transcription termination sequence.

In still further embodiments, the vector constructs described above contain no Alphavirus structural proteins in the vector constructs the selected heterologous sequence may be located downstream from the viral junction region; in the vector constructs described above having a second viral junction, the selected heterologous sequence may be located downstream from the second viral junction region, where the heterologous sequence is located downstream, the vector construct may comprise a polylinker located between the viral junction region and said heterologous sequence, and preferably the polylinker does not contain a wild-type Alphavirus restriction endonuclease recognition sequence.

### 3. Other viral gene delivery vehicles

In addition to retroviral vectors and Alphavirus vectors, numerous other viral vectors systems may also be utilized as a gene delivery vehicle. Representative examples of such gene delivery vehicles include poliovirus (Evans et al., *Nature 339*:385-388, 1989; and Sabin, *J. Biol. Standardization 1*:115-118, 1973); rhinovirus; pox viruses, such as canary pox virus or vaccinia virus (Fisher-Hoch et al., *PNAS 86*:317-321, 1989; Flexner et al., *Ann*. *N.Y. Acad. Sci. 569*:86-103, 1989; Flexner et al., *Vaccine 8*:17-21, 1990; U.S. Patent Nos. 4,603,112, 4,769,330 and 5,017,487; WO 89/01973; WO 95/0924); SV40 (Mulligan et al., *Nature 277*:108-114, 1979); influenza virus (Luytjes et al., *Cell 59*:1107-1113, 1989; McMicheal *et al., N. Eng. J. Med. 309*:13-17, 1983; and Yap et al., *Nature* 273:238-239, 1978); adenovirus (Berkner, *Biotechniques 6*:616-627, 1988; Rosenfeld et al., *Science* 252:431-434, 1991; WO 93/9191; Kolls et al., *PNAS 91*(1):215-219, 1994; Kass-Eisler et al., *PNAS 90*(24):11498-502, 1993; Guzman et al., *Circulation 88*(6):2838-48, 1993; Guzman et al., *Cir. Res. 73*(6):1202-1207, 1993; Zabner et al., *Cell 75*(2);207-216, 1993; Li et al., *Hum. Gene Ther. 4*(4):403-409, 1993; Caillaud et al., *Eur. J. Neurosci. 5*(10):1287-1291, 1993; Vincent et al., *Nat. Genet. 5*(2):130-134, 1993; Jaffe et al., *Nat. Genet. 1*(5):372-378, 1992; and Levrero et al., *Gene 101*(2):195-202, 1991); parvovirus such as adeno-associated virus (Samulski et *al., J. Vir. 63*:3822-3828, 1989; Mendelson et al., *Virol. 166*:154-165, 1988; PA 7/222,684; Flotte et al., *PNAS 90*(22):10613-10617, 1993); herpes (Kit, *Adv. Exp. Med. Biol. 215*:219-236, 1989; U.S. Patent No. 5,288,641); SV40; HIV (Poznansky, *J. Virol. 65*:532-536, 1991); measles (EP 0 440,219); astrovirus (Munroe, S.S. et al., *J. Vir. 67*:3611-3614, 1993); Semliki Forest Virus, and coronavirus, as well as other viral systems *(e.g.,* EP 0,440,219; WO 92/06693; U.S. Patent No. 5,166,057). In addition, viral carriers may be homologous, non-pathogenic(defective), replication competent virus (e.g., Overbaugh et al., *Science 239*:906-910,1988), and nevertheless induce cellular immune responses, including CTL.

### 4. Non-viral gene delivery vehicles

In addition to the above viral-based vectors, numerous non-viral gene delivery vehicles may likewise be utilized within the context of the present invention. Representative examples of such gene delivery vehicles include direct delivery of nucleic acid expression vectors, naked DNA alone (WO 90/11092), polycation condensed DNA linked or unlinked to killed adenovirus (Curiel et al., *Hum. Gene Ther.* 3:147-154, 1992), DNA ligand linked to a ligand with or without one of the high affinity pairs described above (Wu et al., J. *of Biol. Chem 264*:16985-16987, 1989), and certain eukaryotic cells *(e.g.,* producer cells - *see* U.S. Serial No. 08/240,030, filed May 9, 1994, and WO 92/05266).

### D. COUPLING OF A GENE DELIVERY VEHICLE OR TARGETING ELEMENT TO A MEMBER OF THE AFFINITY BINDING PAIR

As noted above, the present invention provides gene delivery vehicles which have been coupled to a member of a high affinity binding pair (also referred to as the "coupled gene delivery vehicle"), as well as targeting elements which have coupled to a member of a high affinity binding pair (also referred to as the "coupled targeting element"). As utilized within the context of the present invention, the term "coupled" may refer to either noncovalent or covalent interactions, although generally covalent bonds are preferred. Numerous methods may be utilized in order to couple one member of a high affinity binding pair to either a gene delivery vehicle or a targeting element, including for example use of crosslinking agents such as N-succinimidyl-3-(2-pyridyl dithio) propionate ("SPDP"; Carlson et al., *J. Biochem. 173:723,* 1978); Sulfosuccinimidyl 4-N-maleimidomethyl) cyclohexane-1-carboxylate ("SulfoSMCC"); 1-ethyl-3 (3-dimethylaminopropyl) carbodiimide ("EDC"); Bis-diazobenzidine ("BDB"); and Periodic acid / Schiffs base.

Within certain embodiments of the invention, a member of the high affinity binding pair is either expressed on, or included as an integral part of, the exterior (e.g., envelope) of the gene delivery vehicle. For example, within one embodiment of the invention a member of the affinity binding pair is co-expressed along with the envelope protein of a viral gene delivery vehicle, as a hybrid protein. More particularly, within certain embodiments the coding region of all or a portion of an affinity binding agent may be fused in-frame with a sequence which encodes an envelope gene. Such affinity binding agent coding regions may be fused to either the amino or carboxyl termini of an envelope gene, or placed within an envelope gene (either by replacement of a portion of the envelope gene, or in addition to the envelope gene). Of particular interest are variable regions A and B, which contain the wild-type receptor binding determinants for ecoptropic and amphotropic envelopes, respectively. A representative example of such fusion proteins is described in more detail below in Example 11.

### HETEROLOGOUS SEQUENCES

Any of the gene delivery vehicles described above may include, contain (and/or express) one or more heterologous sequences. A wide variety of heterologous sequences may be utilized within the context of the present invention, including for example, cytotoxic genes, disease-associated antigens, antisense sequences, sequences which encode gene products that activate a compound with little or no cytotoxicity *(i.e.,* a "prodrug") into a toxic product, sequences which encode immunogenic portions of disease-associated antigens, sequences which encode immune accessory molecules and sequences which encode a desired protein *(e.g.,* a therapeutic or replacement gene such as Factor VIII, U.S. Serial No. 08/366,851). Representative examples of cytotoxic genes include the genes which encode proteins such as ricin (Lamb et al., *Eur. J. Biochem. 148*:265-270, 1985), abrin (Wood et al:, *Eur. J. Biochem. 198*:723-732, 1991; Evensen, et al., *J. of Biol. Chem. 266*:6848-6852, 1991: Collins et al., *J. of Biol. Chem. 265*:8665-8669, 1990; Chen et al., *Fed. of Eur. Biochem Soc. 309*:115-118, 1992), diphtheria toxin (Tweten et al., *J. Biol. Chem. 260*:10392-10394, 1985), cholera toxin (Mekalanos et al., *Nature* 306:551-557, 1983; Sanchez & Holmgren, *PNAS* 86:481-485, 1989), gelonin (Stirpe et al., *J. Biol. Chem. 255*:6947-6953, 1980), pokeweed (Irvin, *Pharmac. Ther.* 21:371-387, 1983), antiviral protein (Barbieri et al., *Biochem. J.* 203:55-59, 1982; Irvin et al., *Arch. Biochem. & Biophys. 200*:418-425, 1980; Irvin, *Arch. Biochem. & Biophys. 169*:522-528, 1975), tritin, Shigella toxin (Calderwood et al., *PNAS 84*:4364-4368, 1987; Jackson et al., *Microb. Path. 2*:147-153, 1987), and Pseudomonas exotoxin A (Carroll and Collier, *J. Biol. Chem. 262*:8707-8711, 1987).

Within further embodiments of the invention, antisense RNA may be utilized as a cytotoxic gene in order to induce a potent Class I restricted response. Briefly, in addition to binding RNA and thereby preventing translation of a specific mRNA, high levels of specific antisense sequences may be utilized to induce the increased expression of interferons (including gamma-interferon), due to the formation of large quantities of double-stranded RNA. The increased expression of gamma interferon, in turn, boosts the expression of MHC Class I antigens. Preferred antisense sequences for use in this regard include actin RNA, myosin RNA, and histone RNA. Antisense RNA which forms a mismatch with actin RNA is particularly preferred.

Within other embodiments of the invention, antisense sequences are provided which inhibit, for example, tumor cell growth, viral replication, or a genetic disease by preventing the cellular synthesis of critical proteins needed for cell growth. Examples of such antisense sequences include antisense thymidine kinase, antisense dihydrofolate reductase (Maher and Dolnick, *Arch. Biochem. & Biophys. 253*:214-220, 1987; Bzik et al., *PNAS 84*:8360-8364, 1987), antisense HER2 (Coussens et al., *Science 230*:1132-1139, 1985), antisense ABL (Fainstein, et al., *Oncogene 4*:1477-1481, 1989), antisense Myc (Stanton et al., *Nature 310*:423-425, 1984) and antisense *ras*, as well as antisense sequences which block any of the enzymes in the nucleotide biosynthetic pathway.

Within other aspects of the invention, gene delivery vehicles are provided which direct the expression of a gene product that activates a compound with little or no cytotoxicity *(i.e.,* a "prodrug") into a toxic product. Representative examples of such gene products include varicella zoster virus thymidine kinase (VZVTK), herpes simplex virus thymidine kinase (HSVTK) (Field et al., *J. Gen. Virol. 49*:115-124, 1980), and E. *coli.* guanine phosphoribosyl transferase (*see* WO 94/13304, entitled "Compositions and Methods for Utilizing Conditionally Lethal Genes;" *see also* WO 93/10218 entitled "Vectors Including Foreign Genes and Negative Selection Markers;" WO 93/01281 entitled "Cytosine Deaminase Negative Selection System for Gene Transfer Techniques and Therapies;" WO 93/08843 entitled "Trapped Cells and Use Thereof as a Drug;" WO 93/08844 entitled "Transformant Cells for the Prophylaxis or Treatment of Diseases Caused by Viruses, Particularly Pathogenic Retroviruses;" and WO 90/07936 entitled "Recombinant Therapies for Infection and Hyperproliferative Disorders;" Field et al., *J. Gen*. *Virol. 49*:115-124, 1980; Munir et al., *Protein Engineering 7*(1):83-89, 1994; Black and Loeb, *Biochem 32*(43):11618-11626, 1993). Within preferred embodiments of the invention, the gene delivery vehicle directs the expression of a gene product that activates a compound with little or no cytotoxicity into a toxic product in the presence of a pathogenic agent, thereby affecting localized therapy to the pathogenic agent (*see* WO 94/13304).

Within one embodiment of the invention, gene delivery vehicles are provided which direct the expression of a HSVTK gene downstream, and under the transcriptional control of an HIV promoter (which is known to be transcriptionally silent except when activated by HIV tat protein). Briefly, expression of the tat gene product in human cells infected with HIV and carrying the gene delivery vehicle causes increased production of HSVTK. The cells (either *in vitro* or *in vivo*) are then exposed to a drug such as ganciclovir, acyclovir or its analogues (FIAU, FIAC, DHPG). Such drugs are known to be phosphorylated by HSVTK (but not by cellular thymidine kinase) to their corresponding active nucleotide triphosphate forms. Acyclovir and FIAU triphosphates inhibit cellular polymerases in general, leading to the specific destruction of cells expressing HSVTK in transgenic mice (*see* Borrelli et al., *Proc. Natl. Acad. Sci. USA 85*:7572, 1988). Those cells containing the gene delivery vehicle and expressing HIV tat protein are selectively killed by the presence of a specific dose of these drugs.

Within further aspects of the present invention, gene delivery vehicles of the present invention may also direct the expression of one or more sequences which encode immunogenic portions of disease-associated antigens. As utilized within the context of the present invention, antigens are deemed to be "disease-associated" if they are either associated with rendering a cell (or organism) diseased, or are associated with the disease-state in general but are not required or essential for rendering the cell diseased. In addition, antigens are considered to be "immunogenic" if they are capable, under appropriate conditions, of causing an immune response (either cell-mediated or humoral). Immunogenic "portions" may be of variable size, but are preferably at least 9 amino acids long, and may include the entire antigen.

A wide variety of "disease-associated" antigens are contemplated within the scope of the present invention, including for example immunogenic, non-tumorigenic forms of altered cellular components which are normally associated with tumor cells *(see* U.S. Serial No. 08/104,424). Representative examples of altered cellular components which are normally associated with tumor cells include ras∗ (wherein "∗" is understood to refer to antigens which have been altered to be non-tumorigenic), p53∗, Rb∗, altered protein encoded by Wilms' tumor gene, ubiquitin∗, mucin, protein encoded by the DCC. APC, and MCC genes, as well as receptors or receptor-like structures such as neu, thyroid hormone receptor, Platelet Derived Growth Factor ("PDGF") receptor, insulin receptor, Epidermal Growth Factor ("EGF") receptor, and the Colony Stimulating Factor ("CSF") receptor.

"Disease-associated" antigens should also be understood to include all or portions of various eukaryotic (including for example, parasites), prokaryotic (*e.g.*, bacterial) or viral pathogens. Representative examples of viral pathogens include the Hepatitis B Virus ("HBV"; *see* WO 93/15207) and Hepatitis C Virus ("HCV"; *see* WO 93/15207), Human Papiloma Virus ("HPV"; *see* WO 92/05248; WO 90/10459; EPO 133,123), Epstein-Barr Virus ("EBV"; *see* EPO 173,254; JP 1,128,788; and U.S. Patent Nos. 4,939,088 and 5,173,414), Feline Leukemia Virus ("FeLV"; *see* WO 93/09070; EPO 377,842; WO 90/08832; WO 93/09238), Feline immunodeficiency Virus ("FIV"; U.S. Patent No. 5,037,753; WO 92/15684; WO 90/13573; and JP 4,126,085), HTLV I and II, and Human Immunodeficiency Virus ("HIV"; *see* WO 91/02805).

Within other aspects of the present invention, the gene delivery vehicles described above may also direct the expression of one or more immune accessory molecules. As utilized herein, the phrase "immune accessory molecules" refers to molecules which can either increase or decrease the recognition, presentation or activation of an immune response (either cell-mediated or humoral). Representative examples of immune accessory molecules include IL-1, IL-2, IL-3, IL-4, IL-5, IL-6, IL-7 (U.S. Patent No. 4,965,195); IL-8, IL-9, IL-10, IL-11, IL-12 (Wolf et al., *J. Immun. 46*:3074, 1991; Gubler et al., *PNAS 88*:4143, 1991; WO 90/05147; EPO 433,827), IL-13 (WO 94/04680), IL-14, IL-15, GM-CSF, M-CSF-1, G-CSF, CD3 (Krissanen et al., *Immunogenetics 26*:258-266, 1987), CD8, ICAM-1 (Simmons et al., *Nature 331*:624-627, 1988), ICAM-2 (Singer, *Science 255*:1671, 1992), β-microglobulin (Parnes et al., *PNAS* 78:2253-2257, 1981), LFA-1 (Altmann et al., *Nature 338:* 521, 1989), LFA3 (Wallner et al., *J. Exp. Med. 166*(4):923-932, 1987), HLA Class I, HLA Class II molecules B7 (Freeman et al., *J. Immun. 143*:2714, 1989), and B7-2. Within a preferred embodiment, the heterologous gene encodes gamma-interferon.

Within preferred aspects of the present invention, the gene delivery vehicles described herein may direct the expression of more than one heterologous sequence. Such multiple sequences may be controlled either by a single promoter, or preferably, by additional secondary promoters (*e.g.*, Internal Ribosome Binding Sites or "IRBS"). Within preferred embodiments of the invention, a gene delivery vehicle directs the expression of heterologous sequences which act synergistically. For example, within one embodiment retroviral vector constructs are provided which direct the expression of a molecule such as IL-15, IL-12, IL-2, gamma interferon, or other molecule which acts to increase cell-mediated presentation in the T_{H}1 pathway, along with an immunogenic portion of a disease-associated antigen. In such embodiments, immune presentation and processing of the disease-associated antigen will be increased due to the presence of the immune accessory molecule.

Within other aspects of the invention, gene delivery vehicles are provided which direct the expression of one or more heterologous sequences which encode "replacement" genes. As utilized herein, it should be understood that the term "replacement genes" refers to a nucleic acid molecule which encodes a therapeutic protein that is capable of preventing, inhibiting, stabilizing or reversing an inherited or noninherited genetic defect. Representative examples of such genetic defects include disorders in metabolism, immune regulation, hormonal regulation, and enzymatic or membrane associated structural function. Representative examples of diseases caused by such defects include Cystic Fibrosis (due to a defect in the Cystic Fibrosis Transmembrane Conductance Regulator ("CFTCR"), *see* Dorin et al., *Nature 326*:614, ), Parkinson's Disease, Adenosine Deaminase deficiency ("ADA"; Hahma et al., *J. Bad. 173*:3663-3672, 1991), β-globin disorders, Hemophilia A & B (Factor VIII-deficiencies; *see* Wood et al., *Nature 312*:330, 1984), Gaucher disease, diabetes, forms of gouty arthritis and Lesch-Nyhan disease (due to "HPRT" deficiencies; *see* Jolly et al., *PNAS 80*:477-481, 1983) Duchennes Muscular Dystrophy and Familial Hypercholesterolemia (LDL Receptor mutations; *see* Yamamoto et al., *Cell 39*:27-38, 1984).

Sequences which encode the above-described heterologous genes may be readily obtained from a variety of sources. For example, plasmids which contain sequences that encode immune accessory molecules may be obtained from a depository such as the American Type Culture Collection (ATCC, Rockville, Maryland), or from commercial sources such as British Bio-Technology Limited (Cowley, Oxford England). Representative sources sequences which encode the above-noted immune accessory molecules include BBG 12 (containing the GM-CSF gene coding for the mature protein of 127 amino acids), BBG 6 (which contains sequences encoding gamma interferon), ATCC No. 39656 (which contains sequences encoding TNF), ATCC No. 20663 (which contains sequences encoding alpha interferon), ATCC Nos. 31902, 31902 and 39517 (which contains sequences encoding beta interferon), ATCC No 67024 (which contains a sequence which encodes Interleukin-1), ATCC Nos. 39405, 39452, 39516, 39626 and 39673 (which contains sequences encoding Interleukin-2), ATCC Nos. 59399, 59398, and 67326 (which contain sequences encoding Interleukin-3), ATCC No. 57592 (which contains sequences encoding Interleukin-4), ATCC Nos. 59394 and 59395 (which contain sequences encoding Interleukin-5), and ATCC No. 67153 (which contains sequences encoding Interleukin-6). It will be evident to one of skill in the art that one may utilize either the entire sequence of the protein, or an appropriate portion thereof which encodes the biologically active portion of the protein.

Alternatively, known cDNA sequences which encode cytotoxic genes or other heterologous genes may be obtained from cells which express or contain such sequences. Briefly, within one embodiment mRNA from a cell which expresses the gene of interest is reverse transcribed with reverse transcriptase using oligo dT or random primers. The single stranded cDNA may then be amplified by PCR (*see* U.S. Patent Nos. 4,683,202, 4,683,195 and 4,800,159. *See also* PCR Technology: Principles and Applications for DNA Amplification, Erlich (ed.), Stockton Press, 1989 all of which are incorporated by reference herein in their entirety) utilizing oligonucleotide primers complementary to sequences on either side of desired sequences. In particular, a double stranded DNA is denatured by heating in the presence of heat stable Taq polymerase, sequence specific DNA primers, ATP, CTP, GTP and TTP. Double-stranded DNA is produced when synthesis is complete. This cycle may be repeated many times, resulting in a factorial amplification of the desired DNA.

Sequences which encode the above-described genes may also be synthesized, for example, on an Applied Biosystems Inc. DNA synthesizer *(e.g.,* ABI DNA synthesizer model 392 (Foster City, California)).

### COMPOSITIONS

Within another aspect of the invention, compositions are provided comprising one or more of the above-described gene delivery vehicles, coupled to one member of a high affinity binding pair. Within other aspects of the present invention, compositions are provided comprising a gene delivery vehicle which is coupled to a high affinity binding pair, which is in turn coupled to a targeting element. Within preferred aspects of the present invention, the gene delivery vehicle is coupled to the member of the high affinity binding pair covalently, for example, by the chemical methods described above. Alternatively, a member of the high affinity binding pair may be expressed directly on the exterior or surface of the gene delivery vehicle, or, otherwise incorporated integrally into the exterior surface (*e.g.*, contained within the envelope or lipid bilayer) of the gene delivery vehicle.

Within other aspects of the present invention, any of the above compositions are provided in combination with a pharmaceutically acceptable carrier or diluent. Such pharmaceutical compositions may be prepared either as a liquid solution, or as a solid form (*e.g.*, lyophilized) which is suspended in a solution prior to administration. In addition, the composition may be prepared with suitable carriers or diluents for topical administration, injection, or nasal, oral, vaginal, sub-lingual, inhalant or rectal administration.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A particularly preferred composition comprises a retroviral vector construct or recombinant viral particle in 10 mg/ml mannitol, 1 mg/ml HSA, 20 mM Tris, pH 7.2, and 150 mM NaCl. In this case, since the recombinant vector represents approximately 1 mg of material, it may be less than 1% of high molecular weight material, and less than 1/100,000 of the total material (including water). This composition is stable at -70°C for at least six months.

Pharmaceutical compositions of the present invention may also additionally include factors which stimulate cell division, and hence, uptake and incorporation of a gene delivery vehicle. Representative examples include Melanocyte Stimulating Hormone (MSH), for melanomas or epidermal growth factor for breast or other epithelial carcinomas. In addition, pharmaceutical compositions of the present invention may be placed within containers or kits (*e.g.*, one container for the coupled targeting element, and a second container for the coupled gene delivery vehicle), along with packaging material which provides instructions regarding the use of such pharmaceutical compositions. Generally, such instructions will include a tangible expression describing the reagent concentration, as well as within certain embodiments, relative amounts of excipient ingredients or diluents (*e.g.*, water, saline or PBS) which may be necessary to reconstitute the pharmaceutical compositions.

Particularly preferred methods and compositions for preserving certain of the gene delivery vehicles provided herein, such as recombinant viruses, are described in WO 94/11414.

### EXAMPLE 1

### CONSTRUCTION OF RETROVECTOR BACKBONES

### A. PREPARATION OF A RETROVIRAL VECTOR CONSTRUCT THAT DOES NOT CONTAIN AN EXTENDED PACKAGING SEQUENCE (Ψ)

This example describes the construction of a retroviral vector construct using site-specific mutagenesis. Within this example, a MoMLV retroviral vector construct is prepared wherein the packaging signal "Ψ" of the retrovector is terminated at basepair 617 of SEQ ID NO: 1, thereby eliminating the ATG start of *gag*. Thus, no crossover can occur between the retroviral vector construct and the *gag/pol* expression cassette which is described below in Example 3.

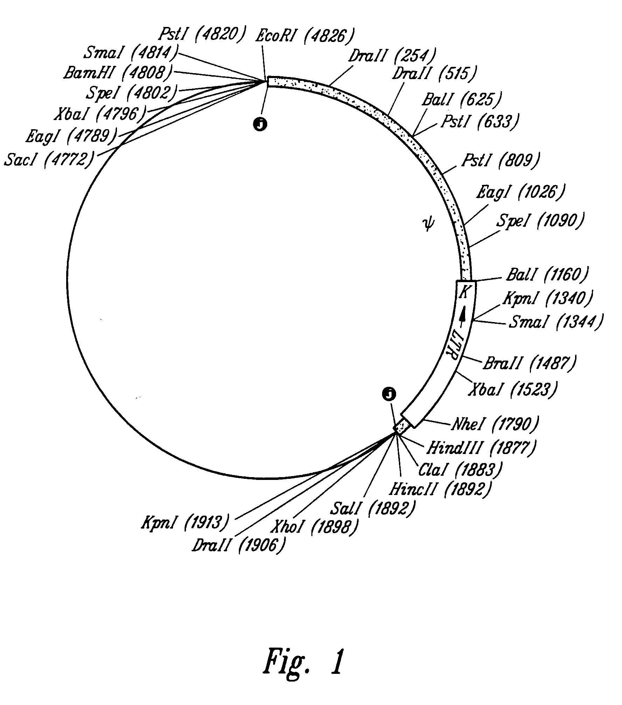

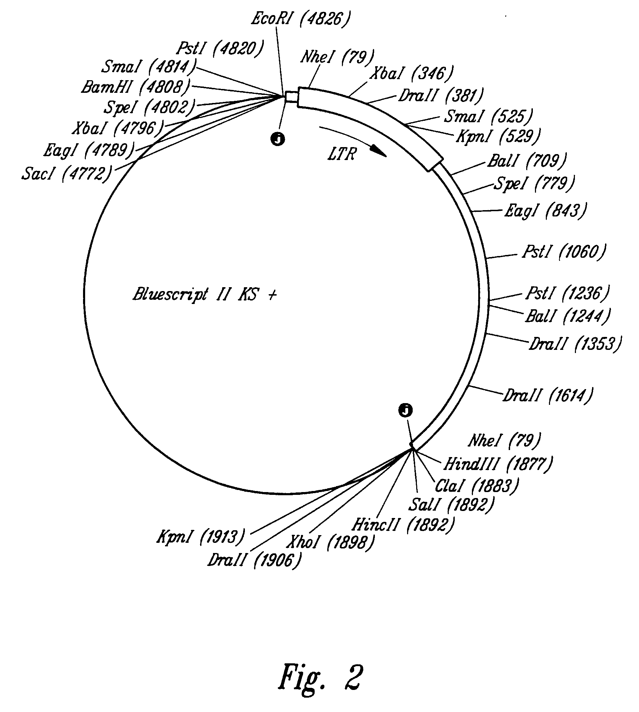

Briefly, pMLV-K (Miller, *J. Virol* 49:214-222, 1984 - an infectious clone derived from pMLV-1 Shinnick et al., *Nature, 293*:543-548, 1981) is digested with *Eco*57I, and a 1.9kb fragment is isolated. (*Eco*57I) cuts upstream from the 3' LTR, thereby removing all *env* coding segments from the retroviral vector construct.) The fragment is then blunt ended with T4 polymerase (New England Biolabs), and all four deoxynucleotides, and cloned into the *EcoRV* site of phagemid pBluescript II KS+ (Stratagene, San Diego, Calif.). This procedure yields two constructs, designated pKS2+Eco57I-LTR(+) (Figure 1) and pKS2+Eco57I-LTR(-) (Figure 2), which are screened by restriction analysis. When the (+) single stranded phagemid is generated, the sense sequence of MoMLV is isolated.

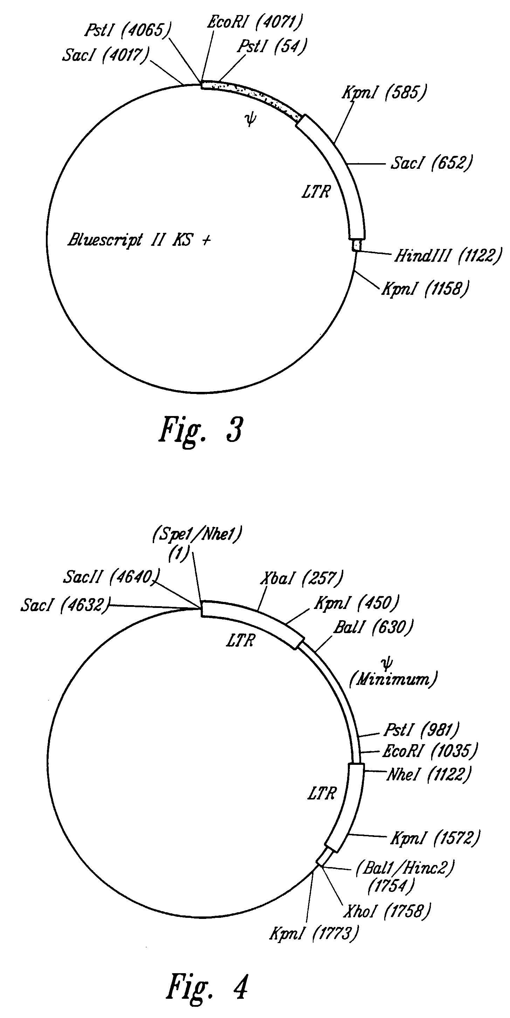

A new *Eco*RI site is then created in construct pKS2+*Eco*57I-LTR(+) in order to remove the ATG start codon of *gag*. In particular, an *Eco*RI site is created using the single stranded mutagenesis method of Kunkle (*PNAS 82*:488, 1985). pKS2+*Eco*57I-LTR(+) is a pBluescript™ II + phagemid (Strategene, San Diego, Calif.) containing an *Eco*57I fragment from pMLV-K. It includes the MoMLV LTR and downstream sequence to basepair 1378. When single stranded phagemid is generated the sense sequence of MoMLV is isolated. The oligonucleotide, 5'-GGT AAC AGT CTG GCC CGA ATT CTC AGA CAA ATA CAG (SEQ ID NO: 2), is created and used to generate an *Eco*RI site at basepairs 617-622. This construct is designated pKS2+LTR-EcoRI (Figure 3).

### B. SUBSTITUTION OF NONSENSE CODONS IN THE EXTENDED PACKAGING SEQUENCE (Ψ+)

This example describes modification of the extended packaging signal (Ψ+) by site-specific mutagenesis. In particular, the modification will substitute a stop codon, TAA, at the normal ATG start site of *gag* (position 631-633 of SEQ ID NO: 1), and an additional stop codon TAG at position 637-639 of SEQ ID NO: 1.

Briefly, an *Eco*571 - *Eco*RI fragment (MoMLV basepairs 7770 to approx. 1040) from pN2 (Amentano et al., J. Virol. 61:1647-1650, 1987) is first cloned into pBluescript II KS+ phagemid at the *Sac*II and *Eco*RI sites (compatible). Single stranded phagemid containing antisense MoMLV sequence, is generated using helper phage M13K07 (Stratagene, San Diego, Calif.). The oligonucleotide 5'-CTG TAT TTG TCT GAG AAT TAA GGC TAG ACT GTT ACC AC (SEQ ID NO: 3) is synthesized, and utilize according to the method of Kunkle as described above, in order to modify the sequence within the Ψ region to encode stop codons at nucleotides 631-633 and 637-639.

### C. REMOVAL OF RETROVIRAL PACKAGING SEQUENCE DOWNSTREAM FROM THE 3' LTR

Retroviral packaging sequence which is downstream from the 3' LTR is deleted essentially as described below. Briefly, pKS2+Eco57I-LTR(-) (Figure 2) is digested with *Ball* and *Hinc*II, and relegated excluding the *Ball* to *Hinc*II DNA which contains the packaging region of MoMLV.

### D. CONSTRUCTION OF VECTOR BACKBONES

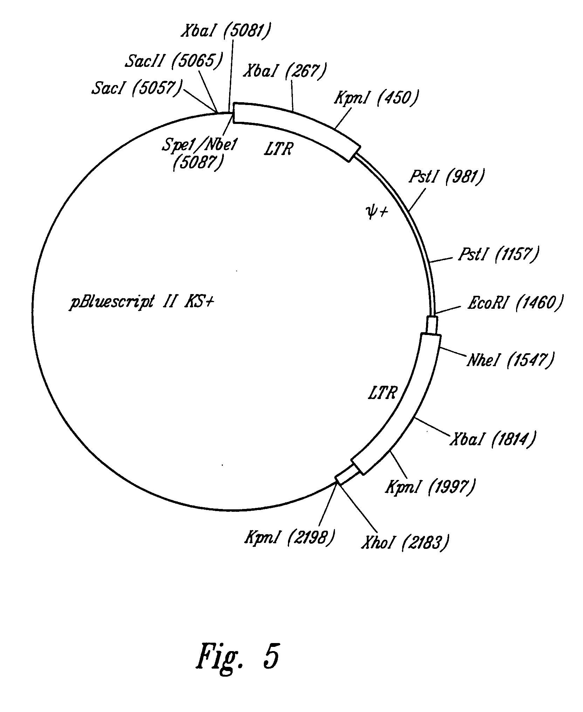

Constructs prepared in sections A and C above, or alternatively from sections B and C above, are combined with a plasmid vector as described below, in order to create a retrovector backbone containing all elements required *in cis*, and excluding all sequences of 8 nucleic acids or more contained in the retroviral portion of the *gag-pol* and *env* expression elements (*see* Examples 3 and 4).
1. Parts A and C are combined as follows: The product of A is digested with *Nhe*I and *Eco*RI, and a 1034 basepair fragment containing the LTR and minimal Ψ is isolated. The fragment is ligated into the product of part C at the unique (compatible) restriction sites *Spe*I and *Eco*RI. The resultant construct is designated pR1 (Figure 4)
2. Parts B and C are combined as follows: The product of B is digested with *Nhe*I and *Eco*RI and a 1456 basepair fragment containing the LTR and modified Ψ+ region is isolated. The fragment is ligated into the product of C at the unique (compatible) restriction sites *Spe*I and *Eco*RI. The resultant construct is designated pR2 (Figure 5).

### EXAMPLE 2

### INSERTION OF A GENE OF INTEREST INTO PR1 AND PR2

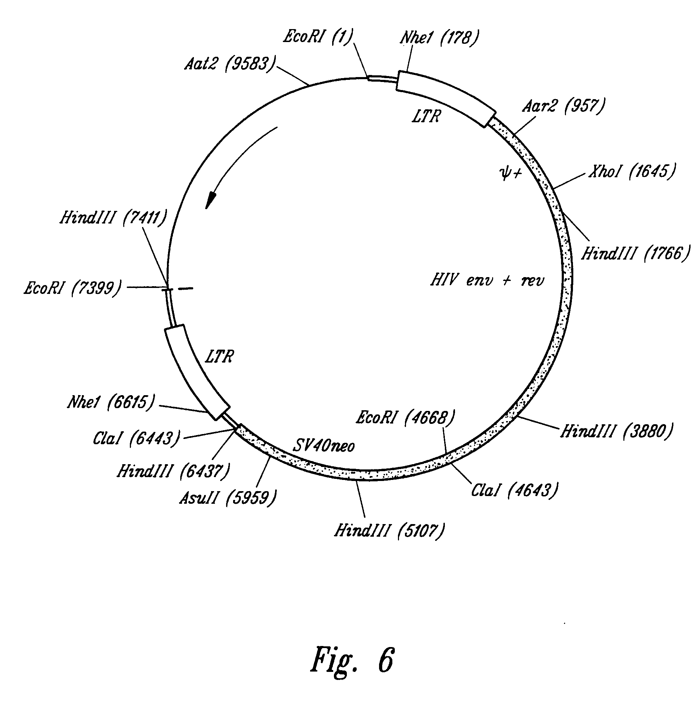

This example describes the insertion of a gene of interest, gp120, gp41, and rev along with a selectable marker into either pR1 or pR2. Briefly, the sequence encoding gp120, gp41 and rev is taken from construct pKTl (Figure 6; *see also* Chada et al., J. *Vir. 67*:3409-3417, 1993); note that this vector is also referred to as N2IIIBenv. In particular, pKT1 is first digested at the unique *Asu*II site (position 5959). The ends are blunted, and an *Xho* I linker is ligated at that site. (New England Biolabs). The construct is then digested with *Xho* I, and a 4314 bp fragment containing HIV envelope (gp120 and gp41), rev, SV40 early promoter and G418 resistance genes is isolated.

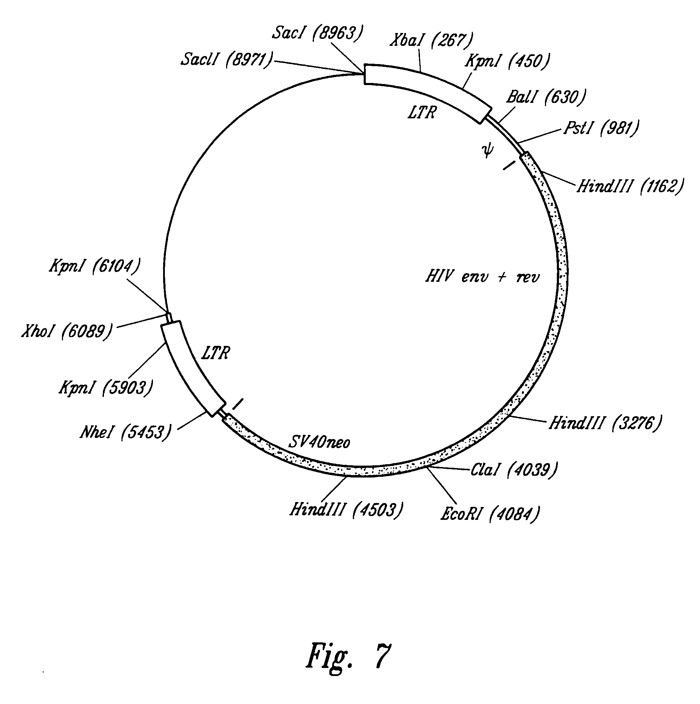

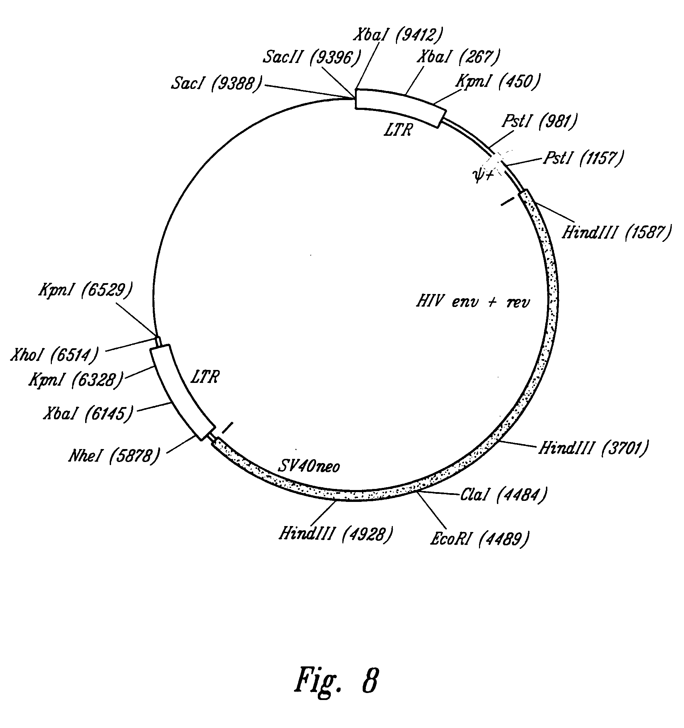

pR1 or pR2 is digested at the unique *Eco* Rl restriction site, blunted, and *Sal I* linkers (New England Biolabs) are ligated in. The 4314 bp KT1 fragment is then ligated into pR1 or pR2 at the *new Sal* I sites, and the correct orientation is determined (*see* Figures 7 and 8). In both of these constructs, (pRl-HlVenv and pR2-HIVenv) the HIV genes are expressed from the MLV LTR, and G418 resistance is expressed from the SV40 promoter.

### EXAMPLE 3

### CONSTRUCTION OF GAG-POL EXPRESSION CASSETTES

### A. CONSTRUCTION OF AN EXPRESSION CASSETTE BACKBONE, PHCMU-PA

A vector is first created in order to form the backbone for both the *gag/pol* and *env* expression cassettes. Briefly, pBluescript SK- phagemid (Stratagene, San Diego, Calif; GenBank accession number 52324; referred to as "SK-") is digested with *Spe*I and blunt ended with Klenow. A blunt end *Dra*I fragment of SV40 (Fiers et al., "Complete nucleotide sequence of SV40 DNA" *Nature 273*:113-120, 1978) from Dral (bp 2366) to Dral (bp2729) is then inserted into SK-, and a construct isolated in which the SV40 late polyadenylation signal is oriented opposite to the LacZ gene of SK-. This construct is designated SK-SV40A.

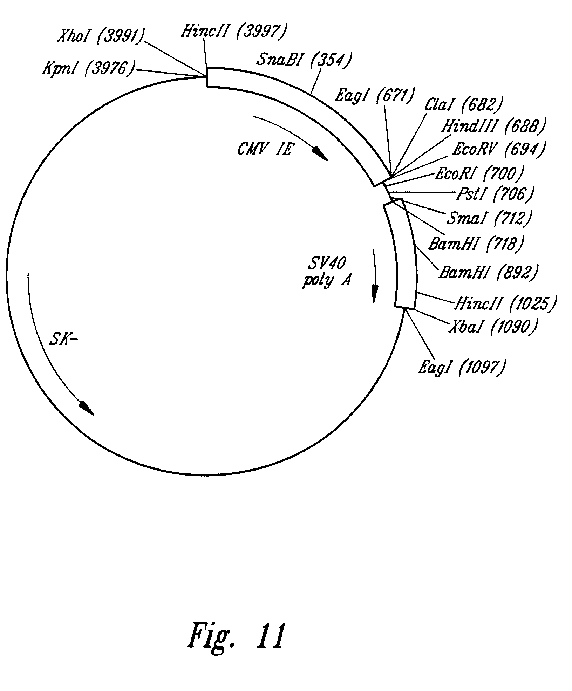

A Human Cytomegalovirus Major Immediate Early Promoter ("HCMV-IE"; Boshart et al., *Cell 41*:521-530, 1985) *(Hinc*II, bp 140, to *Eag*I, bp814) is isolated after digestion with *Hinc*II and *Eag*I*,* and the *Eag*I site blunt ended. The 674 blunt ended fragment is ligated into SK-SV40A. The final construct, designated pHCMV-PA is then isolated *(see* Figure 11). This construct contains the HCMV promoter oriented in opposite orientation to the LacZ gene, and upstream from the late polyadenylation signal of SV40.

### B. CREATION OF NEW CODONS FOR THE 5' GAG

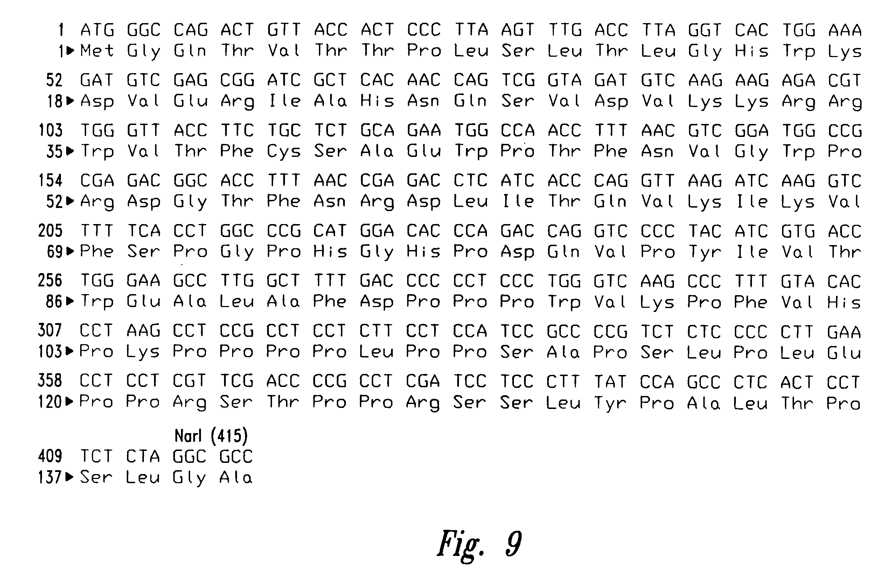

This example describes *gag/pol* expression cassettes that lack non-coding sequences upstream from the *gag* start, thereby reducing recombination potential between the *gag-pol* expression element and Ψ+ sequence of a retroviral vector construct, and inhibiting co-packaging of the *gag-pol* expression element along with the retrovector. In order to construct such an expression cassette, 448 bp of DNA is synthesized with the following features: 5' ATATATATATATCGAT(*Cla*I site)ACCATG(start codon, position 621) (SEQ ID NO: 4), followed by 410 bp encoding 136+ amino acid residues using alternative codons *(see* Figures 9 and 10), followed by GGCGCC(*Nar*I site)AAACCTAAAC 3' (SEQ ID NO: 5).

Briefly, each of oligos 15 through 24 (set forth below in Table 1) are added to a PCR reaction tube such that the final concentration for each is 1 µM. Oligos 25 and 26 are added to the tube such that the final concentration for each is 3 µM. 1.2 µL of 2.5 mM stock deoxynucleotide triphosphates (dG, dA, dT, dC) are added to the tube. 5 µL of 10X PCR buffer (Perkin Elmer). Water is added to a final volume of 50 µL. Wax beads are added and melted over the aqueous layer at 55°C and then cooled to 22°C. A top aqueous layer is added as follows: 5 µL 10X PCR buffer, 7.5 µL dimethylsulfoxide, 1.5 µL Taq polymerase (Perkin-Elmer) and 36 µL water. Forty cycles of PCR are then performed as follows: 94°C, 30 seconds; 56°C, 30 seconds; and 72°C, 30 seconds. The PCR product is stored at -20°C until assembly of the *gag/pol* expression cassette.

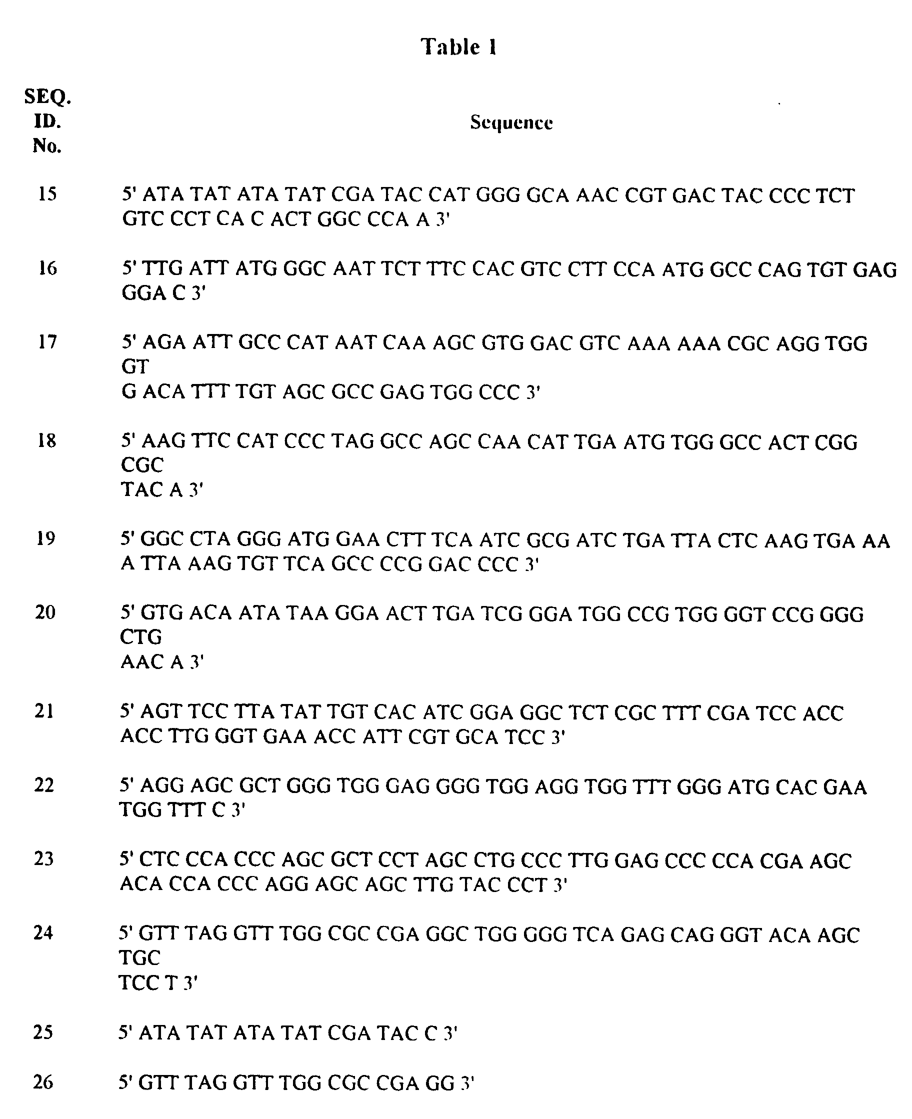

### C. CREATION OF A NEW 3' END FOR POL

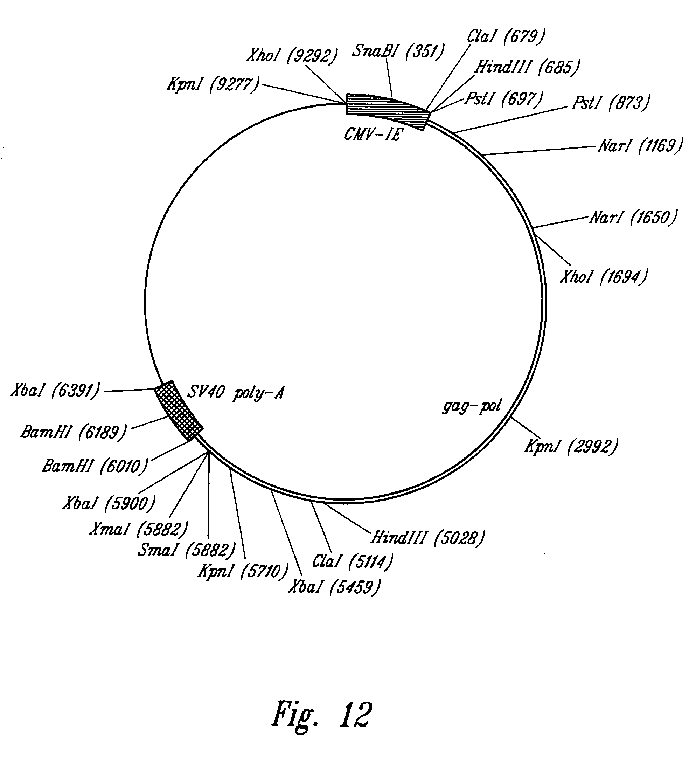

In order to prepare a *gag/pol* expression cassette which expresses full length *gag/pol,* pCMV*gag/pol* is constructed. Briefly, MoMLV sequence from *Pst*1 (BP567) to *Nhel* (bp 7847) is cloned into the *Pst*1-*Xha*1 sites of pUC19 (New England Biolabs). The resultant intermediate is digested with *Hind*III and *Xho*l, and a 1008 bp fragment containing the *gag* leader sequence is isolated. The same intermediate is also digested with *Xho*I and *Sca*I, and a 4312 bp fragment containing the remaining *gag* and *pol* sequences is isolated. The two isolated fragments are then cloned into the *Hind*III and *Sma*I sites of pHCMV-PA, described above. The resultant construct, designated CMV *gag/pol* (Figure 12) expresses MoMLV *gag* and *pol* genes.

In order to truncate the 3' end *of the pol* gene found in pCMV *gag-pol,* a 5531 basepair *SnaBI - Xma*I fragment containing a portion of the CMV IE promoter and all of *gag-pol* except the final 28 codons, is isolated from pCMV *gag-pol*. This fragment is cloned into the *Sna*BI and *Xma*I sites of pHCMV-PA. This construct expresses five new amino acids at the carboxy-terminus (Ser-Lys-Asn-Tyr-Pro) (SEQ ID NO: 6) (pCMV gpSma).

Alternatively, these five amino acids may be eliminated by digesting pCMVgp *Sma*I with *Sma*I and adding an *Nhe*I (termination codons in three phases) linker (5' - CTA GCT AGC TAG SEQ ID NO: 14; New England Biolabs) at the end of the truncated *pol* sequence. This construct is designated pCMV gp Nhe. Both of these constructs eliminates potential crossover between *gag/pol* and *env* expression cassettes.

### D. GAG-POL EXPRESSION CASSETTE

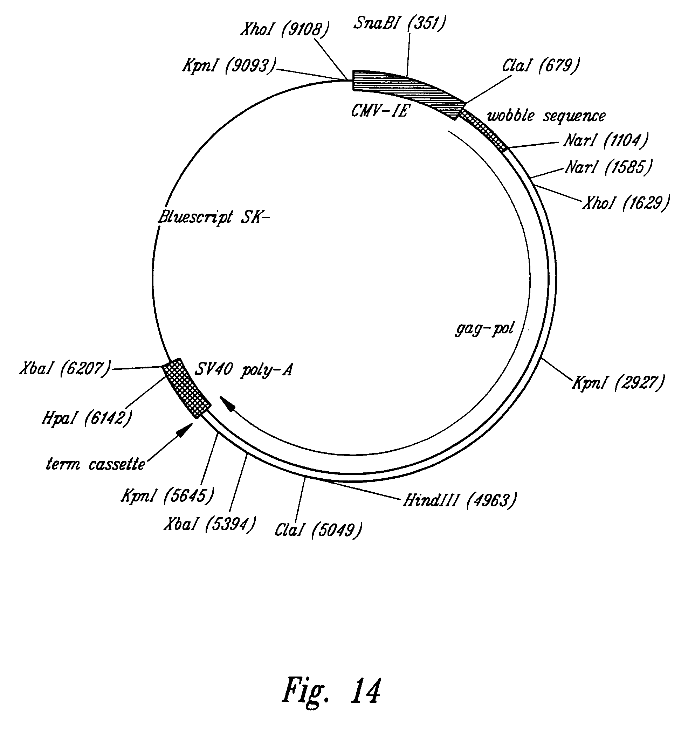

Parts B and C from above are combined to provide an expression vector containing a CMV IE promoter, *gag-pol* sequence starting from the new *Cla*I site (followed by ACC ATG and 412 bp of alternative or "wobble" *gag* coding sequence) and terminating at the *Sma*I site (MoMLV position 5750) followed by an SV40 polyadenylation signal, essentially as described below. Briefly, the approximately 451 bp double stranded wobble fragment from part A is ligated into PCR™II TA cloning vector (Invitrogen Corp.). The wobble PCR product naturally contains a 3' A-overhang at each end, allowing for cloning into the 3' T-overhang of pCR™II. The 422 bp *Cla*I *-Nar*I wobble fragment from the pCRT™II clone is removed and is ligated into the *Cla*I (Position 679, Figure pCMV gp Sma) and *Nar*I (Position 1585) sites of pCMVgp Smal (Part B) (or pCMV gp Nhe). (The *Cla*I site at position 5114 is methylated and not cut with *Cla*I). The product of that ligation is digested with *Nar*I, and the MLV-K *Narl* fragment (positions 1035 to 1378) is inserted (SEQ ID NO: 1). This construct is designated pCMVgp -X (Figure 14).

### EXAMPLE 4

### CONSTRUCTION OF ENV EXPRESSION CASSETTES

### A. CREATION OF A NEW 5' EAGI RESTRICTION SITE

Starting with an *Eag*I- *Eco*RI 626 bp subfragment from a 4070A amphotropic envelope (Chattopodhyay et al., *J. Vir. 39*:777, 1981; GenBank accession # MLV4070A, and #MLVENVC; SEQ ID NO: 12) cloned in a pBluescript II Ks+ vector (containing the start codon), site directed mutagenesis is performed upstream of the translation start site in order to change ACCATCCTCTGGACGGACATG... (SEQ ID NO: 7; positions 20 - 40 of Genebank sequence # MLVENVC) to ACCCGGCCGTGGACGGACATG... (SEQ ID NO: 8) and create a new *Eag*I site at position 23. This modification allows cloning of the amphotropic envelope sequence into an expression vector eliminating upstream 4070A sequence homologous to the *gag-pol* expression element as described in Example 2A.

### B. CREATION OF A NEW 3' END FOR ENV

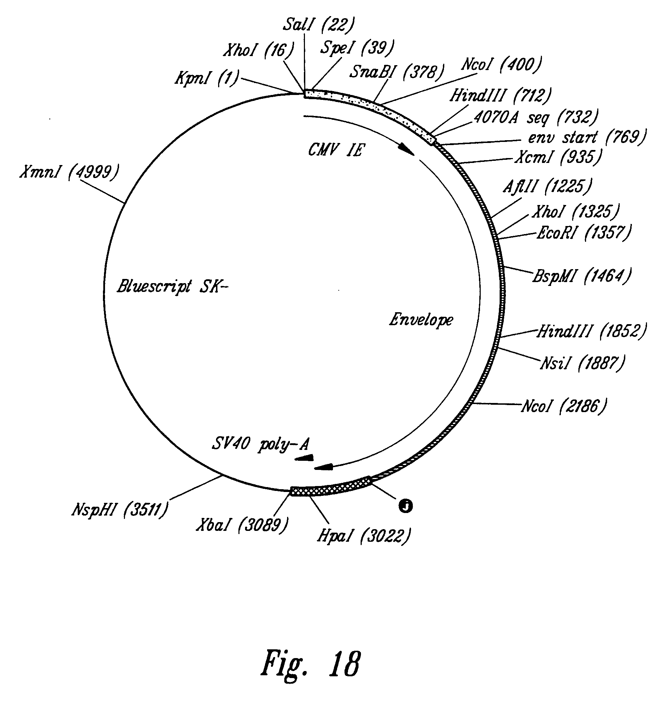

A new 3' end of the envelope expression element is created by terminating the sequence which encodes the R-peptide downstream from the end of the transmembrane region (p15E). Briefly, construct pHCMV-PA, described above, is first modified by digestion with *Not*I (position 1097), blunted and relegated to obliterate the overlapping Bluescript *Eag*I site at the same position. pCMV Envam-Eag-X-less is then constructed by digesting the modified pHCMV-PA with *Eag*I (position 671 and *Sma*I (position 712) and ligating in two fragments. The first is an *EagI-NcoI* fragment from 4070A (positions 1-1455) (SEQ ID NO: 12). The second is an MLV-K envelope fragment, *NcoI - Pvu*II (positions 7227-7747) (SEQ ID NO: 12). The resultant construct from the three-way ligation contains the HCMV promoter followed by the SU (GP70) coding sequence of the 4070A envelope, the TM (p15e) coding sequence of MoMLV, and sequence encoding 8 residues of the R-peptide. In addition, this envelope expression cassette (pCMV Env am-Eag-X-less) (Figure 18) shares no sequence with crossless retrovector backbones described in Example 1.

### C. ENVELOPE EXPRESSION ELEMENT

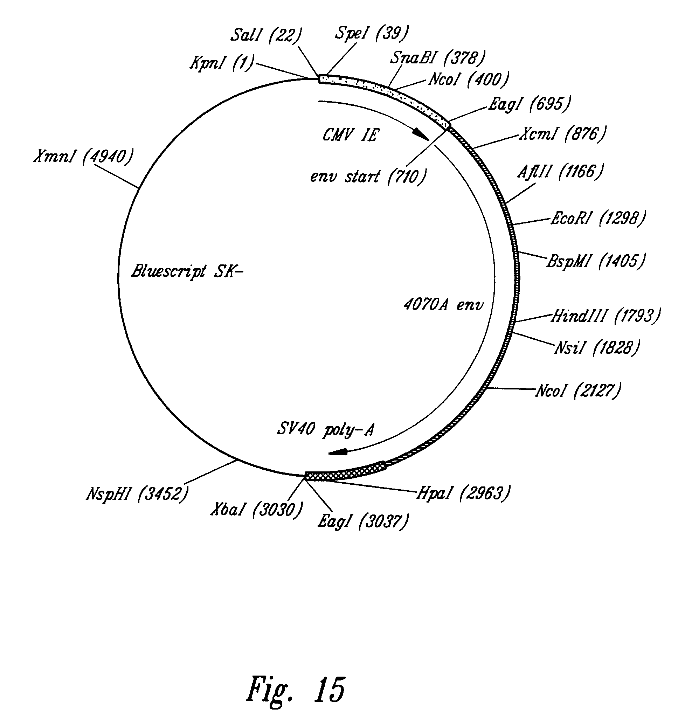

Parts A and B from above are combined to complete an amphotropic expression element containing the CMV promoter, 4070A SU, MoMLV TM and SV40 polyadenylation signal in a Bluescript SK- plasmid vector. This construct is called pCMVenv-X (Figure 15). Briefly, the construct described in part A with a new *Eag*I restriction site is digested with *Eag*I and *Xho*I, and a 571 bp fragment is isolated. pCMV Envam-Eag-X-less (from part B) is digested with *Kpn*I and *Eag*I and the 695 bp fragment is reserved. pCMV Envam-Eag-X-less (from part B) is digested with *Kpn*I and *Xho*I and the 4649 bp fragment is reserved. These two fragments are ligated together along with the 571 bp *Eag*I to *Xho*I fragment digested from the PCR construct from part A. pCMVenv-X shares no sequence with crossless retrovector backbones nor the *gag-pol* expression element pCMVgp-X.

### EXAMPLE 5

### FUNCTIONALITY TESTS FOR GAG-POL AND ENV EXPRESSION CASSETTES

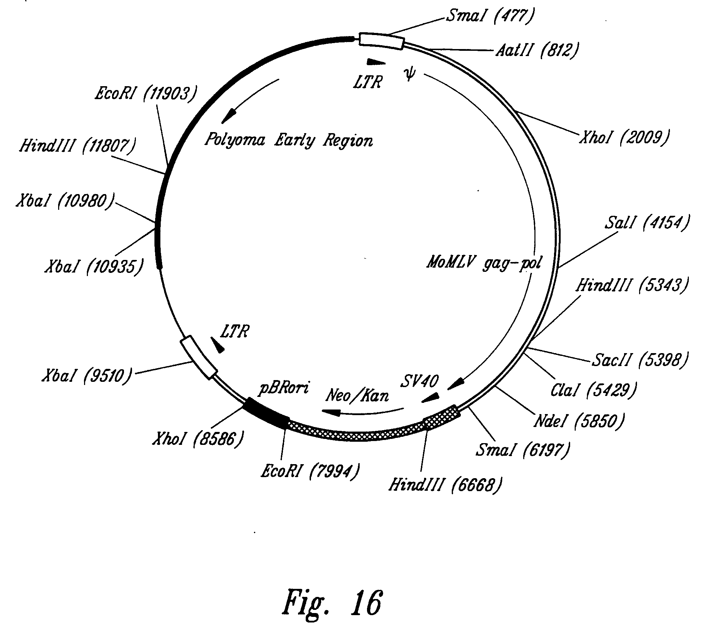

Rapid tests have been developed in order to ensure that the *gag-pol* and *env* expression cassettes are biologically active. The materials for these tests consist of a cell line used for transient expression (typically 293 cells, ATCC #CRL 1573), a target cell line sensitive to infection (typically HT 1080 cells, ATCC #CCL 121) and either pRgpNeo (Figure 16) or pLARNL (Emi et al., *J. Virol 65*:1202-1207, 1991). The two later plasmids express rescuable retrovectors that confer G418 resistance and also express *gag-pol,* in the case of RgpNeo or *env*, in the case of pLARNL. For convenience, the organization of RgpNeo (Figure 16) is set forth below.

In order to test expression cassettes such as pCMVgp-X for functionality of *gag/pol,* the plasmid is co-transfected with pLARNL at a 1:1 ratio into 293 cells. After 12 hours, the media is replaced with normal growth media. After an additional 24 hours, supernatant fluid is removed from the 293 cells, filtered through a 0.45 µm filter, and placed on HT 1080 target cells. Twenty-four hours after that treatment, the media is replaced with growth media containing 800 ug/ml G418. G418 resistant colonies are scored after one week. The positive appearance of colonies indicates that all elements are functional and active in the original co-transfection.
For convenience, the organization of RgpNeo (Figure 16) is set forth below:
Position 1 = left end of 5' LTR; Positions 1-6320 = MoMLV sequence from 5'LTR to Sca 1 restriction site; Positions 6321 - 6675 = SV40 early promoter; Positions 6676-8001 = Neo resistance gene from Tn 5 (including prokaryotic promoter); and Positions 8002 - 8606 = pBR origin of replication.

### EXAMPLE 6

### PACKAGING CELL LINE AND PRODUCER CELL LINE DEVELOPMENT

This example describes the production of packing and producer cell lines utilizing the above described retroviral vector constructs, *gag/pol* expression cassettes, and *env* expression cassettes, which preclude the formation of replication competent virus.

Briefly, for amphotropic MoMLV-based retroviral vector constructs, a parent cell line is selected which lacks sequences which are homologous to Murine Leukemia Viruses, such as the dog cell line D-17 (ATCC No. CCL 183). The *gag/pol* expression cassettes are then introduced into the cell by electroporation, along with a selectable marker plasmid such as DHFR (Simonsen et al., *PNAS 80*:2495-2499, 1983). Resistant colonies are then selected, expanded in 6 well plates to confluency, and assayed for expression of gag/pol by Western Blots. Clones are also screened for the production of high titer vector particles after transduction with pLARNL.

The highest titer clones are then electroporated with an *env* expression cassette and a selectable marker plasmid such as hygromycin (*see* Gritz and Davies, *Gene* 25:179-188, 1983). Resistant colonies are selected, expanded in 6 well plates to confluency, and assayed for expression of env by Western Blots. Clones are also screened for the production of high titer vector particles after transduction with a retroviral vector construct.

Resultant packaging cell lines may be stored in liquid Nitrogen at 10 x 10⁶ cells per vial, in DMEM containing 10% irradiated Fetal Bovine Serum, and 8% DMSO. Further testing may be accomplished in order to confirm sterility, and lack of helper virus production. Preferably, both an S+L- assay and a *Mus dunni* marker rescue assay should be performed in order to confirm a lack of helper virus production.

In order to construct a producer cell line, retroviral vector construct as described above in Example 1 is electroporated into a xenotropic packaging cell line made utilizing the methods described above. After 24-48 hours, supernatant fluid is removed from the xenotropic packaging cell line, and utilized to transduce a second packaging cell line, thereby creating the final producer cell line.

### EXAMPLE 7

### HELPER DETECTION ASSAY COCULTIVATION, AND MARKER RESCUE

This example describes a sensitive assay for the detection of replication competent retrovirus ("RCR"). Briefly, 5 x 10⁵ vector-producing cells are cocultivated with an equal number of *Mus dunni* cells (Lander and Chattopadhyay, *J. Virol. 52*:695, 1984). *Mus dunni* cells are particularly preferred for helper virus detection because they are sensitive to nearly all murine leukemia-related viruses, and contain no known endogenous viruses. At three, six, and nine days after the initial culture, the cells are split approximately 1 to 10, and 5 x 10⁵ fresh *Mus dunni* cells are added. Fifteen days after the initial cocultivation of *Mus dunni* cells with the vector-producing cells, supernatant fluid is removed from cultures, filtered through a 0.45 µm filter, and subjected to a marker rescue assay.

Briefly, culture fluid is removed from a MdH tester cell line (*Mus dunni* cells containing pLHL (a hygromycin resistance marker retroviral vector; *see* Palmer et al., *PNAS* 84(4):1055-1059, 1987) and replaced with the culture fluid to be tested. Polybrene is added to a final concentration of 4 µg/ml. On day 2, medium is removed and replaced with 2 ml of fresh DMEM containing 10% Fetal Calf Serum. On day 3, supernatant fluid is removed, filtered, and transferred to HT1080 cells. Polybrene is added to a final concentration of 4µg/ml. On day 4, medium in the HT1080 cells is replaced with fresh DMEM containing 10% Fetal Calf Serum, and 100 µg/ml hygromycin. Selection is continued on days 5 through 20 until hygromycin resistant colonies can be scored, and all negative controls (*e.g.*, mock infected MdH cells) are dead.

### EXAMPLE 8

### RETROVIRAL VECTOR-AVIDIN COUPLED GENE DELIVERY VEHICLES, AND MELANOCYTE STIMULATING HORMONE-BIOTIN COUPLED TARGETING ELEMENTS

The following example describes the use of the coupled targeting element melanocyte stimulating hormone-biotin to target the coupled retroviral vector particle-biotin to a specific cell type. Generally, biotinylated melanocyte stimulating hormone (MSH) is first injected into the patient. After a period of time (up to 3 days) after which non-specific binding has decayed and only specific ligand complexes remain, a vector expressing avidin on its surface is injected. The high affinity of avidin for biotin focuses the vector to the target tissue.

Briefly, melanocyte-stimulating hormone (MSH) is a 13 amino acid peptide that is specifically recognized by a receptor on melanocytes. MSH has a receptor affinity (K_{D}) in the range of 10⁻⁸M.

### A. CONSTRUCTION OF PCMV-ENV^{ECO}

pCMV-env^{eco} is created by inserting the XbaI-NheI fragment of MoMLV (bp 5766 through bp 7845 of MoMLV) into pCMV-PA (example 3A) expression vector. Briefly the XbaI-NheI envelope fragment is isolated from pMLV-K (Miller et al., *J. Vir. 49*:214-222,1988) on an agarose gel. The fragment is then blunt-ended with T4 polymerase using standard methods, ligated into pCMV-PA (example 3), and digested at the EcoRV and SmaI sites. The product in the correct orientation has a CMVIE promoter followed by the complete ecotropic envelope coding sequence and an SV40 polyadenylation signal.

### B. CREATION OF AVIDIN-ENVELOPE CHIMERA

A portion of avidin DNA (GenBank # CHKAVIR) from bp 116 through bp 499 is incorporated into the MoMLV ecotropic envelope construct pCMV-env^{eco}. Briefly, the following oligonucleotide is generated as follows: The oligonucleotide is used to modify single stranded pCMV-env^{eco} by the method of Kunkle *(PNAS* 82:488, 1985). This modification replaces a portion of the variable A region of envelope (Battini et al., *J. Virol 66*:1468-1475, 1992) with the sequence of the oligonucleotide. The product is then digested with EcoRI and partially digested with FspI. The EcoRI-FspI fragment of avidin (bp 198 through bp 485) is ligated into the vector. The final product is a plasmid containing CMV promoter, hybrid eco-avidin envelope and SV40 polyadenylation signal, called pCMV-env^{eco-avidin}.

### C. BIOTINYLATED MSH

The MSH peptide S-Y-S-M-E-H-F-R-W-G-L-P-V-NH2 (Sequence I.D. No. 28) is synthesized (Chiron Corp., Emeryville, CA), and biotinylated with NHS-Biotin (Pierce) according to the manufacturer's instructions.

### D. GENERATION OF MARKER RETROVECTOR DISPLAYING AVIDIN

The beta galactosidase encoding marker retrovector, CBβ-gal is cotransfected into cell line 293 2-3 (WO 92/05266) along with pCMV-env^{eco-avidin}. Alternatively, equivalent vectors encoding luciferase, green fluorescent protein (GFP) or other markers can be used. Clones are selected (with G418) and screened for high production of RNA containing particles and screened for surface expression of avidin using ³H-biotin binding. Vector particles containing avidin are tested utilizing ¹⁴C-biotin (Amersham) and a sucrose gradient.

### E. IN VITRO TARGETING

Human melanoma cells, DM252, DM6, DM92 are grown in appropriate medium. The specificity of biotinylated MSH binding to target cells is tested by addition of avidin-fluorescein and fluorescence microscopy. Transduction of eco-avidin CBβ-gal is tested either by staining or by G418 selection (*see* WO 94/21792), and the efficiency of tranduction compared to non-melanoma cells such as HT 1080 human fibrosarcoma cells.

### F. IN VIVO TARGETING

Nude mice are implanted with one or more of the following human melanoma cell lines: DM252, DM6, DM92 (*see* WO 94/21792) in the peritoneal cavity. Targeting is determined by first injecting biotin-MSH into the mouse, followed by injection of 10⁵-10⁸ colony forming units eco-avidin CBβ-gal retrovectors. Targeting is assessed by subsequently dissecting the melanoma tissue, and staining for β-gal, or assaying for luciferase activity in the melanoma and mouse tissue. As a control, the same vectors encapsidated in the pCMV-env^{eco} transfection of 293-2-3 cells, and with no added envelope plasmids, are injected into mice in parallel, and the tissues of these mice are assayed.

### EXAMPLE 9

### CONJUGATION OF CARBOXYPEPTIDASE A WITH LACTOSE

### A. PREPARATION OF OXIDIZED LACTOSE

A solution of 0.01 M Na borate pH 4.0, with 0.1 M Na m-periodate (Sigma Chemical Company, St. Louis) and 0.1 M in lactose is prepared and incubated 1 hour at room temperature in the dark (C.J. Sanderson and D.V. Wilson, *Immunology 20:* 1061-1065, 1971). The solution is then adjusted with 0.2 M Na phosphate buffer to pH 7.

### B. CONJUGATION WITH CPA

10 mg of bovine pancreatic carboxypeptidase A (CpA) (Sigma Chemical, St. Louis, MO) is dialyzed in a large excess of 0.1 M Na phosphate buffer overnight. The solution is concentrated to 2 ml (5 mg/ml) in Centricon 10,000 dt cutoff centrifugal ultrafiltration units, according to manufacturer's directions (Amicon). Lactose is added to the CpA solution at a 1000-fold molar excess (285 mmoles), and incubated at room temperature for one hour. The reaction is terminated by the reduction of the Schiff's base by the addition of cyanoborohydride (Sigma) (Borch et al., *J. Am. Chem. Soc. 93*: 2897-2904 (1971) and Fagnani et al., *Cancer Research 50*: 3638-3645 (1990)) at a ratio of 1:5 relative to the lactose, and incubating another 2 hours with stirring. The conjugate is dialyzed against phosphate buffered saline pH 7.5 and stored at 4°C prior to injection.

### C. PREPARATION OF SINDBIS VECTOR PARTICLES CARRYING THE β-GALACTOSIDASE GENE

Preparation of Sindbis vector particles carrying the β-galactosidase gene involves four primary steps:
(1) construction ofa Sindbis vector;
(2) insertion of the β-galactosidase gene into the Sindbis vector;
(3) packaging of the Sindbis/β-galactosidase vector by transfection/ infection of a cell line expressing the Sindbis virus structural proteins; and
(4) purification of Sindbis vector particles. Other marker genes such as luciferase or green fluorescent protein (GFP) can be used in an equivalent fashion, except for the assay of the gene product.

Briefly, the configuration of Sindbis is that of a replacement vector, wherein the heterologous genetic material is substituted for the viral structural genes. The remaining portion of the viral genome is unmodified. Thus, on a linear map, the expression vector is comprised of the following ordered elements: Sindbis nonstructural genes; Sindbis junction region; β-galactosidase gene; 40 3' end Sindbis nucleotides; a consecutive tract of 40 dA:dT residues; and a restriction endonuclease recognition sequence which is unique to the vector construction. The signal for genome packaging is contained within the nonstructural protein region.

The construction of the basic Sindbis vector from a genomic Sindbis virus cDNA clone is described in Example 2, WO 94/10469. Briefly, construction of the Sindbis β-galactosidase vector is performed by assembling together components of 3 independent plasmids, pSKII5'SIN and pKSII3'SIN, and pSV-β-galactosidase, Promega (Madison, WI). The β-galactosidase gene is first inserted into the pKSII3'SIN plasmid between the Hind III and Bam HI sites. The β-galactosidase gene is then isolated from the pSV-β-galactosidase plasmid by digestion with Bam HI and Hind III, and electrophoresed on a 1% agarose/TBE gel. An approximately 3,737 bp fragment is then excised from the gel, and purified with Gene Clean II kit (Bio 101, San Diego, CA). Insertion into pKS3'SIN is then accomplished by ligation of the 3737 bp β-galactosidase fragment with a gel purified 3008 bp fragment resulting from digestion with Bam HI and Hind III and treatment with CIAP of pKSII3'SIN. This construction is designated as pKSII3'SIN-β-Gal.

Final assembly of the Sindbis β-galactosidase vector is accomplished by first digesting pSK5'SIN with Xho I and Sac I, then treating with CIAP, and gel purifying the large 10,533 bp fragment. The pSK5'SIN 10,533 bp fragment is then ligated together with the 2854 bp small fragment resulting from digestion of pKSII3'SIN-β-Gal with Xho I and Sac I. This construction contains the entire Sindbis nonstructural gene coding region, and 3' viral elements necessary for genome replication; the β-galactosidase gene is placed between these two viral 5' and 3' elements. This vector is designated as pSKSINBV-β-Gal.

In the application described above, the pSKSINBV-β-Gal vector is defective and is unable to complete a full infection cycle including cell lysis when introduced to monolayers known to support the permissive infection of Sindbis virus.

In order to construct a Sindbis vector particle which is capable of expressing the β-galactosidase gene after infection of cells permissive for supporting infection characteristic of wild type Sindbis virus, vector RNA from the pSKSINBV-β-Gal clone is first transcribed *in vitro,* then transfected onto a cell line which expresses the Sindbis structural proteins. The pSKSINBV-β-Gal clone is linearized by digestion with Sac I, and the 3' overhang ends generated by digestion with the enzyme are made blunt by inclusion of the T4 DNA polymerase enzyme from *E. coli* during the last 15 minutes of digestion. RNA corresponding to the Sindbis-β-Gal vector is transcribed *in vitro* from purified linearized pSKSINBV-β-Gal DNA, using the mMessage mMachine kit (Ambion Inc., Austin TX) according to the directions of the supplier.

Packaging of the Sindbis vector is accomplished by transfection of the *in vitro* transcribed Sindbis-β-Gal RNA with Lipofectin (Gibco-BRL, Gaithersberg, MD) onto cells which express the Sindbis structural proteins. These cells are known as Sindbis vector packaging cell lines, and their construction is described in Example 7 of WO 94/10469. The Sindbis vector packaging cell lines can be derived from several possible hosts, including for example, mosquito, quail, and hamster cells.

In order to generate a high titer preparation of the Sindbis-β-Gal vector particle, supernatants from the transfected Sindbis vector packaging cell lines are harvested at 24 hours post transfection, and used to infect fresh Sindbis packaging cell line monolayers. One ml of transfection supernatant is used per 10 cm plate, which contain, typically, 5 X 10⁶ cells.

Packaged Sindbis-β-Gal vector particles are purified and concentrated from the infected Sindbis vector packaging cells at 48 hours post infection, or when the cells demonstrate substantial cytopathic effects (CPE). The vector particle is purified and concentrated by the following steps: (1) removal of cell debris by centrifugation at 1000 g for 15 min.; (2) two-fold purification by gradient centrifugation through linear 15-35% potassium tartrate gradients in phosphate-buffered saline lacking calcium, for 12 hours at 24,000 r.p.m.; and (3) dialysis overnight at 4°C, with 12,000 MW cut-off bags, against a buffer comprised of 10 mM HEPES and 100 mM NaCI, pH 7.4. Wild type Sindbis virus particles purified in this manner typically have titers of 1 x 10¹⁰ to 1 x 10¹¹ PFU/ml.

### D. SYNTHESIS OF PEPTIDE ANALOG CYS-PHE-VAL^{P}-(O)PHE

The transitional state analog inhibitor, [[L-Cysteinyl-L-phenylalanyl-L-valinyl-1-aminoethyl] hydroxyphosphinyl]-L-phenylalanine, is synthesized from commercially available reagents as described in Hanson et al. (Hanson et al., *Biochemistry 28:* 6294-6305 (1989) or Kaplan and Bartlett (Kaplan and Bartlett, *Biochemistry 30:* 8165-8170 (1991).

### E. CONJUGATION OF SINDBIS TO A HIGH AFFINITY MOLECULE

1. Reaction of Sindbis Vector with SulfoSMCC: Purified Sindbis vector is dialysed in 0.1 M Na phosphate buffer pH 7.5. Sulfosuccinimidyl 4-(N-maleimidomethyl) cyclohexane-1-carboxylate (SulfoSMCC) (Pierce, Rockford, IL) is dissolved in water (Carlsson et al., *Biochem. J. 173*: 723-737 (1978) and Hashida et *al. J. Appl. Biochem. 6* :56-63 (1984)). Sindbis reactor is added to the SulfoSMCC solution, and incubated overnight at 4 °C on a rocker. The virus is then separated from the cross-linking agent by dialysis in degassed 0.1 M Na phosphate buffer, 1 mM EDTA pH 7.0.
2. Reaction of the Peptide Derivative with the Sindbis Vector: The peptide analog described in Section D above is reduced in 10 mM dithiothreitol, and desalted in degassed 0.1 M Na phosphate buffer, 1 mM EDTA pH 7.0 on a P6 column (BioRad, Melville, NY). The peptide is then added to the Sindbis vector conjugate and allowed to react for one hour at 4°C on a rocker. The virus is separated from the unconjugated peptide by dialysis in phosphate buffered saline.

### F. TARGETING β-GAL EXPRESSION TO THE LIVER IN RATS

A solution containing 100 µg of the carboxypeptidase A conjugate is injected into the tail vein of adult male Sprague-Dawley rats. After 24 hours, the rats are anesthetized with ketamine (110 mg/kg, ip) and 10⁷ units of the Sindbis vector are injected into the hepatic portal vein. After 24 or 48 hours, the rat is dissected and tissue sections are examined for βgal expression or the expression of other marker genes. The results are compared to those obtained with unmodified control Sindbis vectors.

### EXAMPLE 10

### TARGETING POLYCATION-DNA COMPLEXES TO THE LIVER WITH CYTOSTATIN AND PAPAIN

### A. CONJUGATION OF TRANSFERRIN WITH CYSTATIN

Cystatin and transferrin are obtained commercially (Sigma). Briefly, the transferrin and cystatin are combined in a 1:1 molar ratio (approx. 5:1 w/w) at total of 30 mg/ml in water and dialyzed in 0.001 M Na phosphate pH 7.5. Solid 1-ethyl-3-(3-dimethylaminopropyl) carbodiimide HCl (Pierce) is added to a final concentration of 20 mM. The reaction is incubated at room temperature (approx. 22°C) on a rocker and quenched after 16 hours by the addition of solid Na acetate to 200 mM. This reaction is described in more detail in M.R. Mauk and A.G. Mauk, *Eur. J. Biochem.* 186: 473-486 (1989). The reaction is diluted to 4 mg/ml and dialyzed into phosphate buffered saline. The extent of the reaction is checked by SDS gel electrophoresis.

### B. CONJUGATION OF PAPAIN TO POLYCATION-DNA COMPLEX, AND PREPARATION OF PAPAIN-POLYCATION-DNA/βGAL EXPRESSION REPORTER PLASMID MIXTURES

The papain-polycation conjugate is formed by mixing commercially available papain (Sigma Chemical Co., St. Louis, MO) with poly-L-lysine, Mᵣ = 41,000 dt (Sigma) at a 1:1 weight ratio in 5 ml deionized water, pH 7.4. The reactants are conjugated with 1-ethyl-3-(3-dimethylaminopropyl) carbodiimide HCI (Pierce) in a 140 fold excess over papain and stirred 16 hours at 25°C. The reaction mixture is then dialyzed against deionized water at 4°C for 72 hours (G.Y. Wu, P. Zhan, L.L. Sze, A.R. Rosenberg, and C.H. Wu, *J. Biol. Chem. 269*: 11542-11546 (1994)). The extent of cross-linking is determined by SDS gel electrophoresis. A CMV promoter, 13-gal expression vector designated pcDNA3 (Invitrogen) is mixed at a 1:1.3 w/w ratio of plasmid to polylysine conjugate in 150 mM NaCI, 20 mM HEPES pH 7.4, at 4 µg plasmid/ml and incubated at room temperature for 30 minutes (Wagner et al., *Proc. Natl. Acad. Sci. 88*: 4255-4259 (1991)). Other marker plasmids (e.g., luciferase) can also be used.

The transferrin receptor is elevated in most tumor types. Thus, nude mice bearing several types of human tumor xenografts either in the peritoneum or as liver metastases are injected with 100 ng to I mg of conjugated transferrin-cystatin, intraperitoneally, or in the tail vein, respectively. At 2 hours to 21 days later, the DNA complex carrying 100 ng to 100 µg of DNA is injected into the same site, or into the hepatic portal vein. After 24-72 hours, mice are dissected and tumors and tissues tested for marker gene activity in order to determine targeting of the polycation-DNA complex.

### EXAMPLE 11

### TARGETING WITH ECOTROPIC ENVELOPE FUSED WITH AVIDIN

### A. CONSTRUCTION OF RXEN AND RSEN

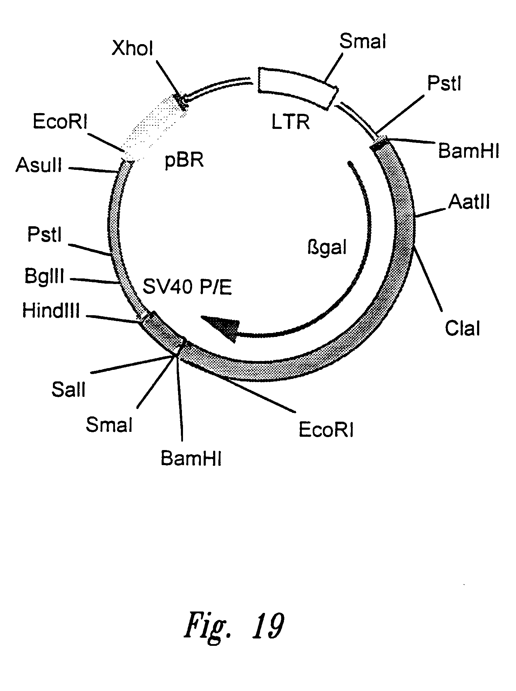

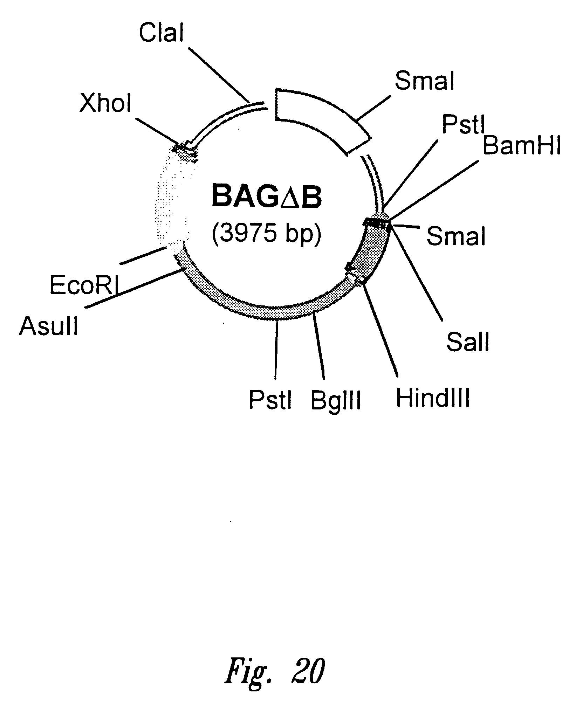

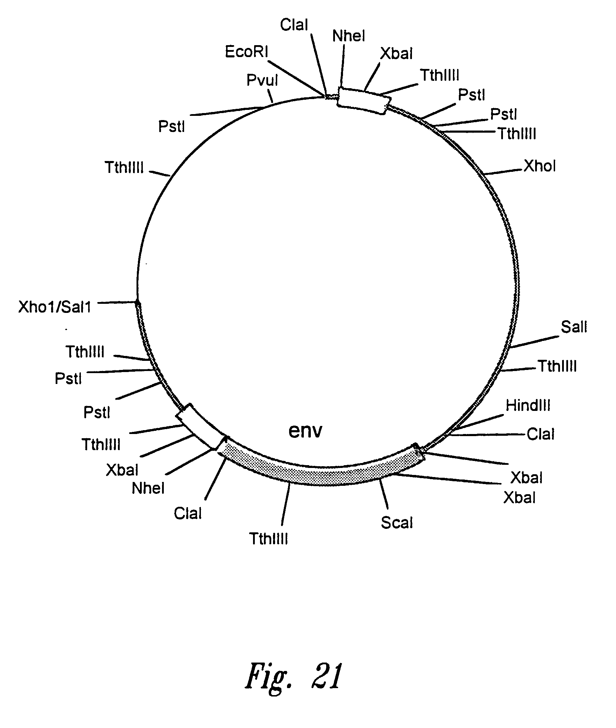

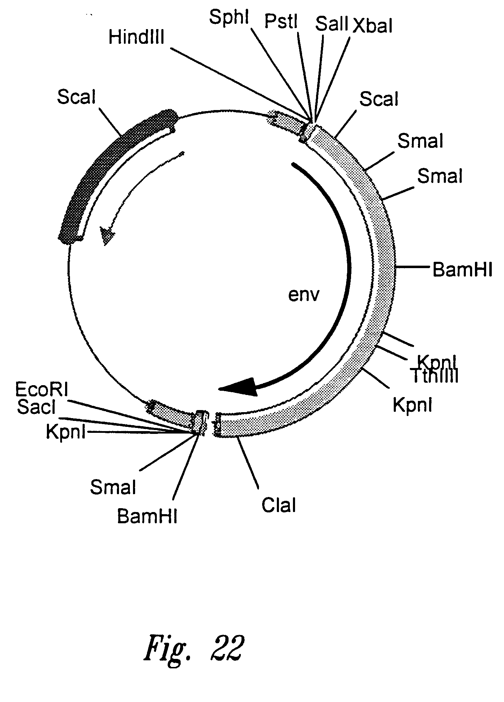

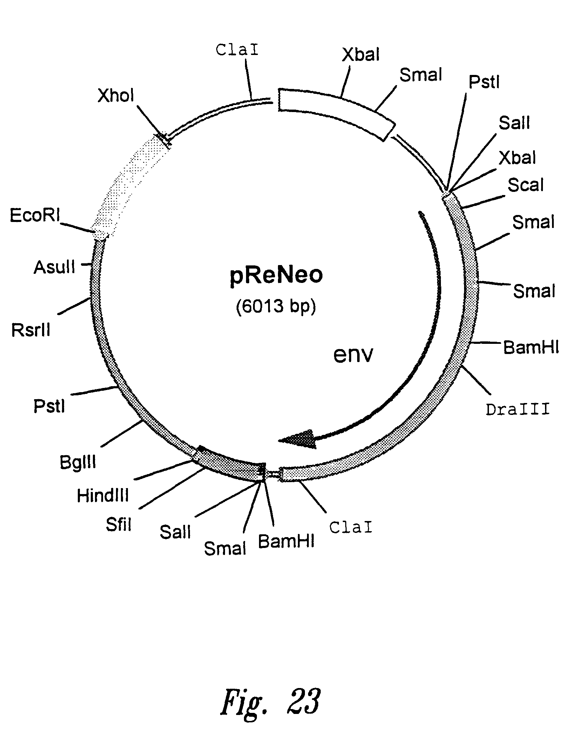

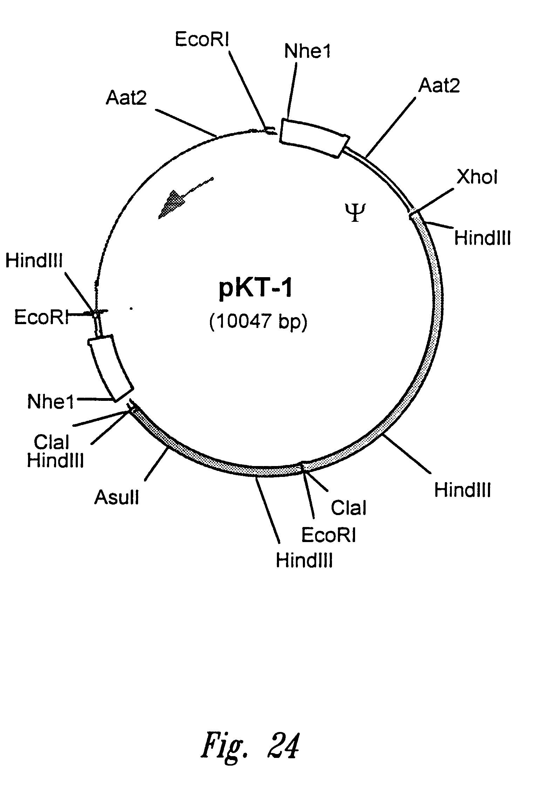

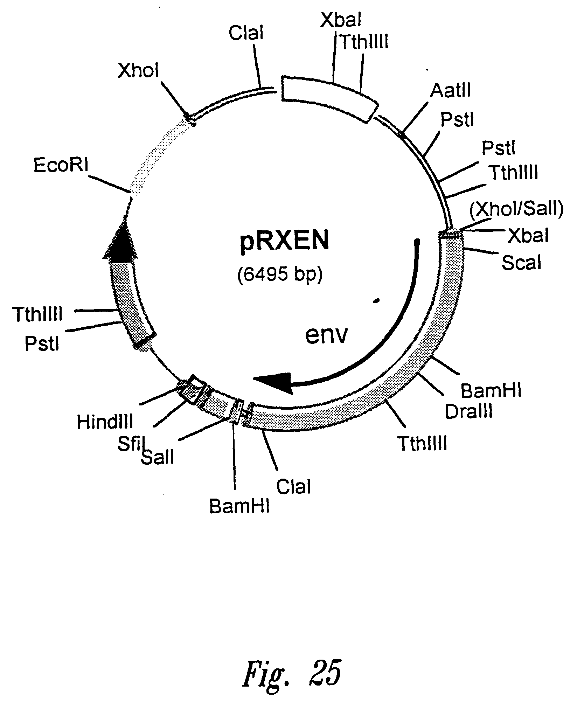

The first retroviral vector, ReNeo, designed as a base for modifying the ecotropic envelope, was made by replacing the β galactosidase gene of the BAG vector (Figure 19) (Price et al., *PNAS 84*:156-160, 1987) with the ecotropic envelope gene of MLV-K (Miller et al., *J. Virol. 49*:214, 1984). The BAG vector consists of one murine Moloney leukemia virus LTR, β galactosidase gene, SV40 promoter/enhancer, neomycin/kanamycin resistance gene, and the pBR322 origin of replication. The β galactosidase gene was removed by digesting the BAG vector with *Bam*HI and religating it, to make the intermediate construct, BAGΔB (Figure 20). The ecotropic envelope gene was taken from pMLV-K (Figure 21) by digestion with *Xba*I and *Nhe*I and ligated into the *Xha*I site of pUC18 in such a way as to put the 5' end of envelope next to the *Pst*I site of the polylinker and the 3' end near the *Bam*HI site (Figure 22). The sequence of the envelope insert of this intermediate vector, pNAG1, was verified by standard DNA sequencing methods. The ecotropic envelope was removed from pNAGI by partial digestion with *Bam*HI and complete digestion with *Pst*I. The BAGΔB vector was digested with *Eco*RI and *Bam*HI for one fragment of a three piece ligation, and the smaller, 1600 bp fragment was isolated. The BAGΔB vector was then digested with *Eco*RI and *Pst*I and the larger, 2000 bp fragment was isolated. The ecotropic envelope PstI-BamHI fragment was ligated with the two BAGΔB fragments, *Bam*HI-*Eco*RI and *Eco*RI-*Pst*I to make ReNeo (Figure 23). The extended packaging sequence was taken from the KT1 vector (Figure 24) by digestion with *Aat*II and *Xho*I, isolating the 600 bp fragment. This was ligated to ReNeo in a three piece ligation, *Aat*II-*Dra*III, 5 Kb fragment and *Dra*III-*Sal*I, 700 bp fragment. The resulting retroviral vector, RXEN, (Figure 25) contains the extended packaging sequence as well as the rest of the features of ReNeo.

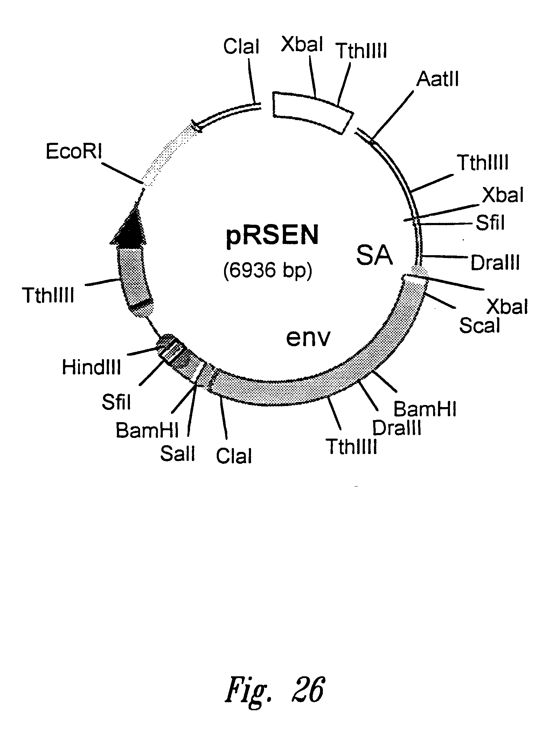

The envelope gene in ReNeo and RXEN was removed without the native splice acceptor from Moloney MLV. There is a strong splice donor in the extended packaging sequence of RXEN, and only a cryptic splice acceptor upstream of the envelope gene. This may impair the efficiency of envelope mRNA production by ReNeo and RXEN by allowing the coding sequence to be spliced out of the transcript. The native splice acceptor of the Moloney envelope was excised from MLV-K as a 400 bp Xba I fragment and inserted upstream of the envelope gene of RXEN, partially digested with Xbal, to make RSEN (Figure 26).

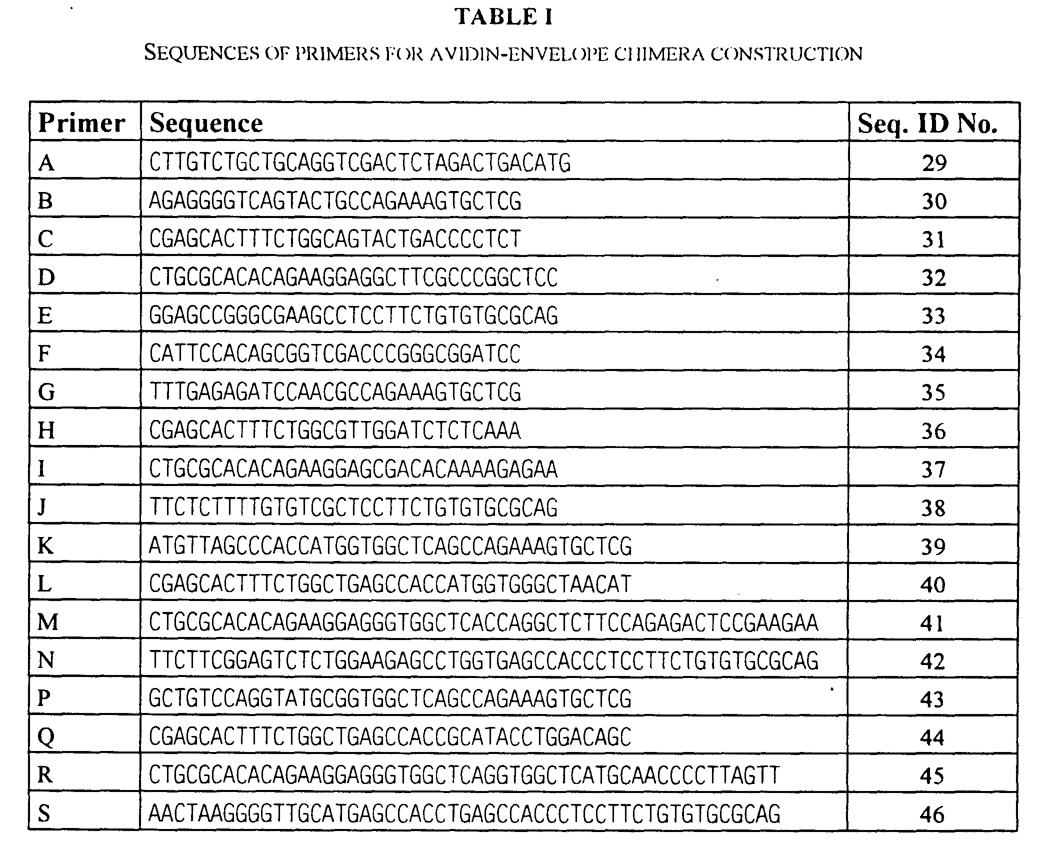

**TABLE II**

| INSERTION OF AVIDIN INTO MOLONEY ECOTROPIC ENVELOPE BY DOUBLE OVERLAP PCR | | | | |
|---|---|---|---|---|
| **Fusion Point** | **Template** | **Primer 1*** | **Primer 2*** | **Product** |
| Amino | ReNeo** | A | C | AC |
| Amino | Avidin | B | E | BE |
| Amino | ReNeo | D | F | DF |
| Amino | AC and BE | A | E | AE |
| Amino | AE and DF | A | F | Final Insert |
| Var.A | ReNeo | A | L | AL |
| Var. A | Avidin | K | N | KN |
| Var. A | ReNeo | M | F | MF |
| Var. A | AL and KN | A | N | AN |
| Var. A | AN and MF | A | F | Final Insert |
| Var. B | ReNeo | A | Q | AQ |
| Var. B | Avidin | P | S | PS |
| Var. B | ReNeo | R | F | RF |
| Var. B | AQ and PS | A | S | AS |
| Var. B | AS and RF | A | F | Final Insert |
| Carboxyl | ReNeo | A | H | AH |
| Carboxyl | Avidin | G | J | GJ |
| Carboxyl | ReNeo | I | F | IF |
| Carboxyl | AH and GJ | A | J | AJ |
| Carboxyl | AJ and IF | A | F | Final Insert |

| | | | | |
|---|---|---|---|---|
| * Primer Sequences are shown in Table I above. * Template Sequences are shown in Figures 27, 28A and 28B. | | | | |

### B. CONSTRUCTION OF AVIDIN CHIMERAE BY PCR

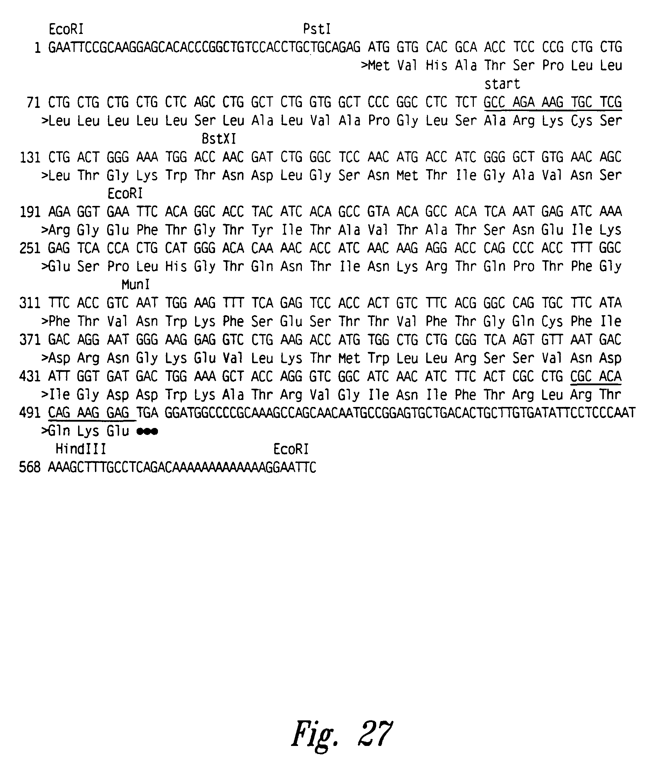

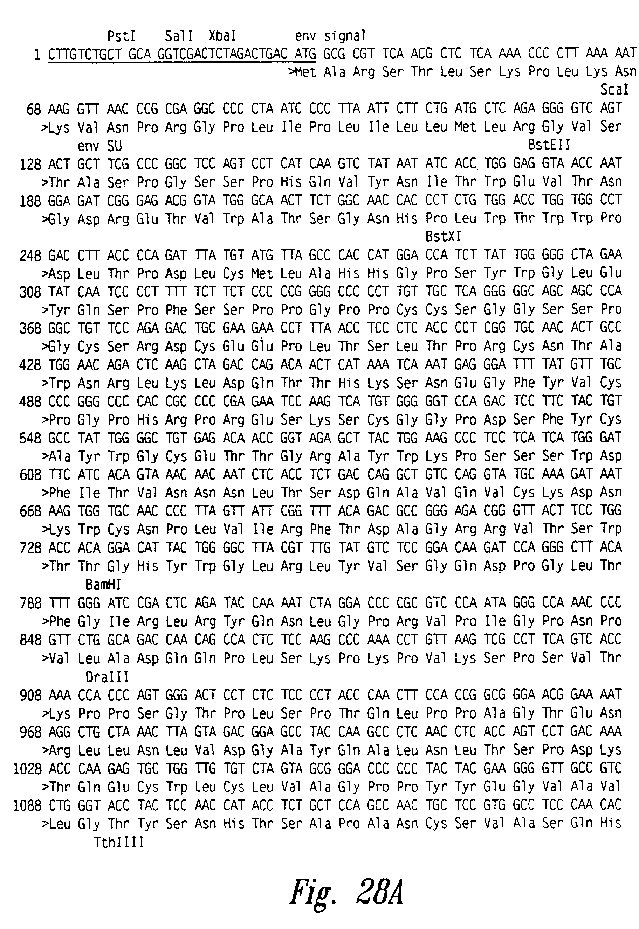

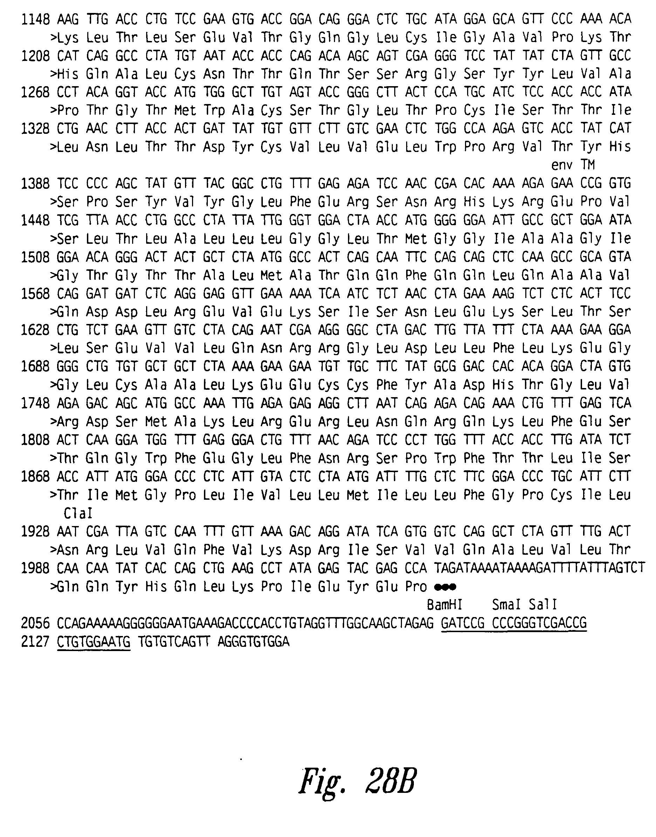

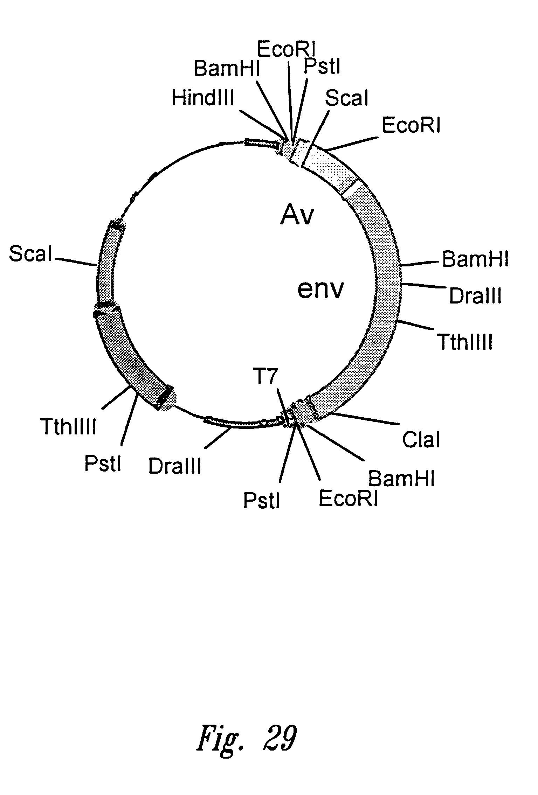

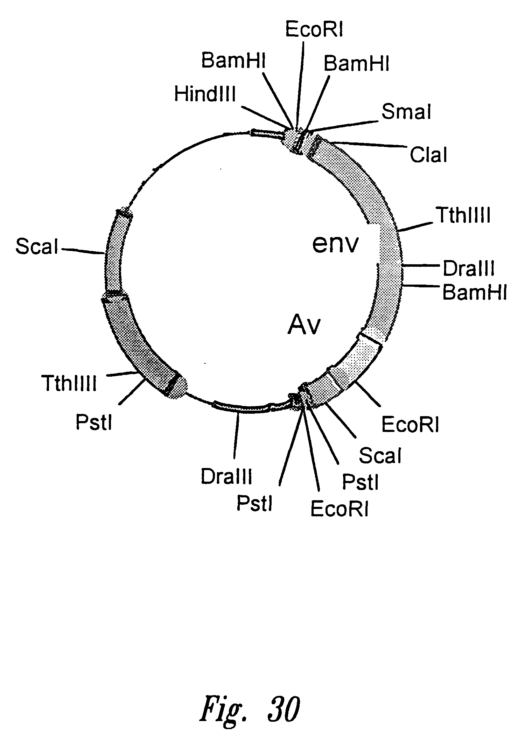

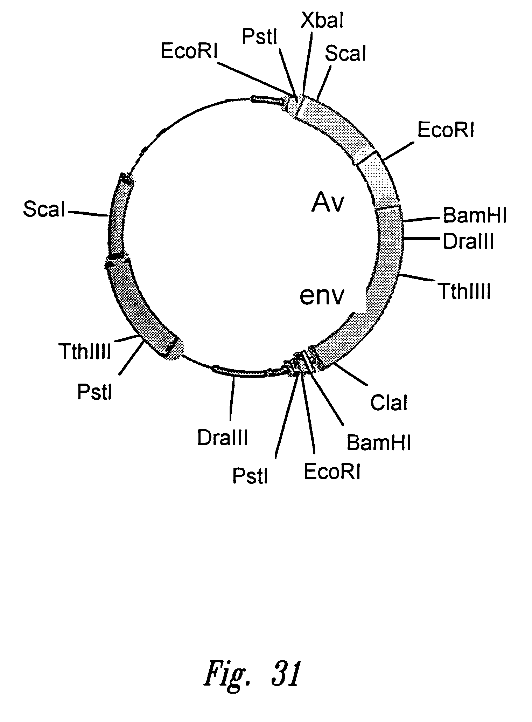

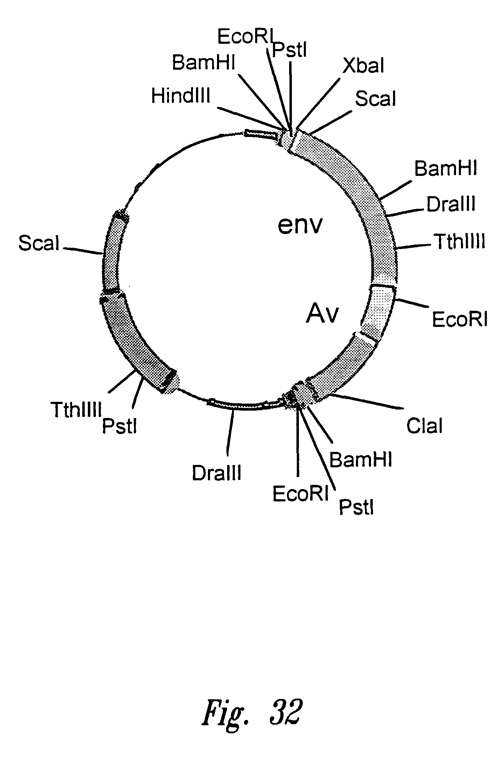

The avidin envelope chimerae were constructed by double overlap PCR using ReNeo and chicken avidin cDNA as templates (Horton et al., *Biotechnique*s *8*:528-535, 1990). The primers A and F in Table I correspond to outside sequences flanking the envelope gene and the remaining 16 primers are designed in sets of four for the insertion of avidin into four differents sites in the envelope gene. The four sites used for fusion with avidin were: 1) the amino terminus, between thr33 and ala34; 2) the variable region A, replacing the sequence from gly85 through ser111 and changing cys114 and cysl 18 each to serine; 3) the variable region B, replacing sequence from lys210 through trp214. The PCR reactions were carried out as indicated in Table II on template sequences listed in Figures 27, 28A and 28B, as recommended by the manufacturer's instructions using a GeneAmp PCR kit (Perkin Elmer/Cetus). The final chimeric PCR products containing avidin inserted at the specified sites were each cloned into the vector, pCRII according to the manufacturer's instruction using the TA Cloning kit (Invitrogen, San Diego, CA). The sequences of the avidin inserts and the envelope region flanking them were verified by standard DNA sequencing methods. The clones that were found to be correct and were used in all further constructions were: pCRII/N5 (Figure 29) for the amino terminal fusion, pCRII/A1 (Figure 30) for the variable region A fusion, pCRII/B14 (Figure 31) for the variable region B fusion, and pCRII/C8 (Figure 32) for the carboxyl terminal fusion.

### C. CONSTRUCTION OF RXEN/AVIDIN RETROVIRAL VECTORS

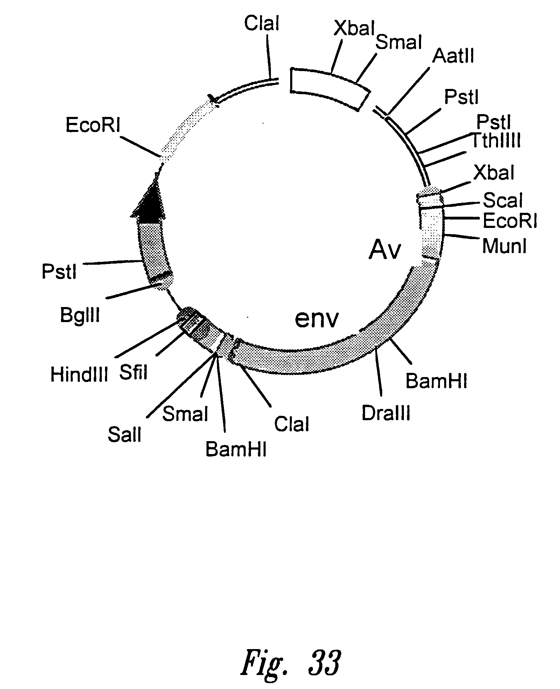

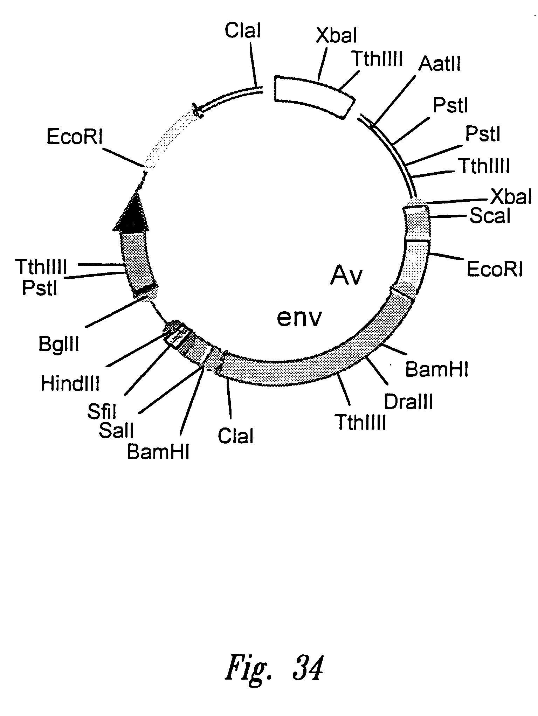

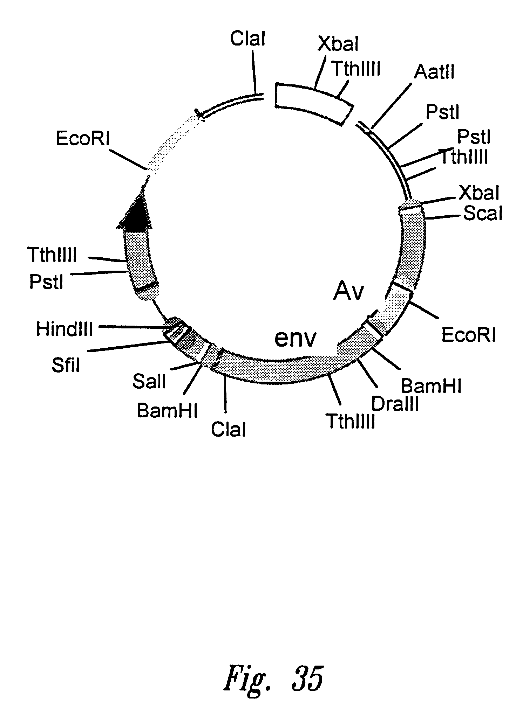

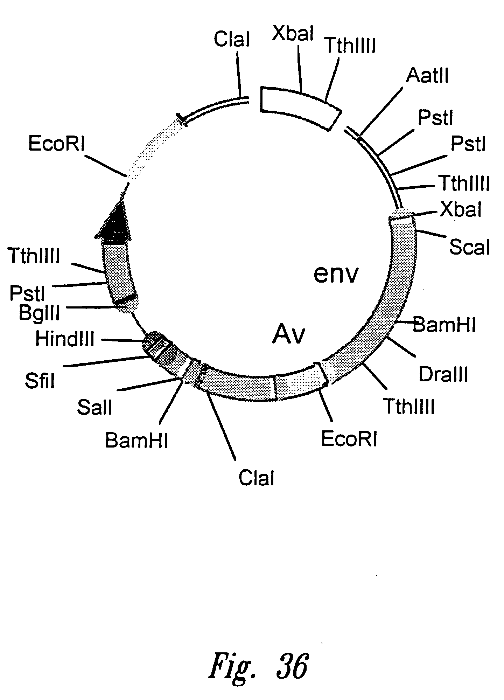

The avidin-containing regions of the pCRII clones were removed by digestion with appropriate restriction enzymes and inserted into the corresponding sites of RXEN. Three of the four avidin fusions, pCRII/N5, pCRII/A1 and pCRII/B14, were cloned into RXEN by ligation of each ScaI-DraIII, 1.1 Kb fragment to RXEN cut with Scal and DraIII to make RXEN/N5 (Figure 33), RXEN/A1 (Figure 34), and RXEN/B14 (Figure 35). The carboxy terminal avidin fusion pCRII/C8 was cut with DraIII and Clal, 1.5 Kb, and ligated to RXEN, partially digested with Clal and completely with DraIII, 5.0 Kb to make RXEN/C8 (Figure 36).

### D. TRANSDUCTION OF PACKAGING CELL LINES WITH RXEN/AVIDIN RETROVIRAL VECTORS

The retroviral vectors containing chimeric avidin-envelope genes were introduced into the packaging cell lines, 293 2-3 and HX (WO 92/05266) and GP+E (Markowitz et *al., J. Virol. 62*:1120-1124, 1988) by G pseudotyping (Burns et al., *PNAS 90*:8033-8037, 1993). This method consists of cotransfection of 293 2-3 with 10 µg of each of RXEN, RXEN/N5, RXEN/A1, RXEN/B14, and RXEN/C8 retroviral vectors with 10 µg of the VSV G protein vector, MLPG by CaPO₄ transfection with the ProFection kit according to the manufacturer's instructions (Promega, Madison, WI). This was followed by transduction of 293 2-3, HX and GP+E each with the resulting vector-containing supernatants. These cells were subjected to selection with geneticin and the resulting pooled transductants were raised to confluency. The supernatants of these cell lines were harvested, passed through 0.45 µm filters and stored at -80°C in aliquots until use.

### E. IN VITRO TARGETING ASSAY USING BIOTINYLATED LIGANDS

Target cells, either HCT116 human colon carcinoma cells (ATCC No. CCL 247) or murine SC-1 cells (ATCC No. CRL 1404), were seeded at 1 x 10⁵ cells per well of a six well plate in two ml of fresh DMEM containing 10% Fetal Calf Serum (FCS). On day 2, plates were removed from the 37°C. incubator and set on an ice bed for fifteen minutes prior to addition of biotinylated ligand, to slow cellular metabolism and reduce surface membrane capping. Each biotin conjugated reagent was then added to a single culture well in the following concentrations: Transferrin-biotin, 5 mg/well; Low Density Lipoprotein-biotin, 2.5 mg/well; Wheat germ Agglutinin-biotin, 2 mg/well; Phytohemagglutinin-L-biotin, 2 mg/well; or Concanavalin-A-biotin, 2 mg/well. This mixture was incubated on ice for thirty minutes, then each well was washed twice with cold DMEM media and brought to one ml with DMEM plus 10% FCS.

Retrovector with avidin fused to envelope: RXEN, RXEN/N5, RXEN/B14, RXEN/C8, and RXEN/A1, packaged in HX, GP+E and 293 2-3 packaging cell lines were collected from the supernatant of 24 hour confluent packaging cell line cultures, and filtered through a 0.45µm filter. Equal volumes of this material are added to each culture well, and the final volume brought to 2 ml with DMEM plus 10% FCS. Control wells included target cells with no biotinylated ligand and no retroviral vector, and target cells with retroviral vector only. Polybrene was added to each well at a final concentration of 4 mg/ml. Plates were held on ice for another thirty minutes, then incubated at 37°C.

On day 4 post-transfection, medium on the HCT116 cells was replaced with fresh DMEM containing 10% FCS and 400 mg/ml G418. Selection was continued from days 5 through 14 until G418 resistant colonies could be detected and scored, and until all cells in control wells lacking retroviral vector were dead.

## Claims

1. A combination of:
(i) a targeting element coupled to a first member of a high affinity binding pair, said targeting element being capable of specifically binding to a selected cell type in a warm-blooded animal; and
(ii) a gene delivery vehicle presenting the second member of said high affinity binding pair,
for sequential or simultaneous administration to target said gene delivery vehicle to cells of said selected cell type.

2. A combination as claimed in claim 1 wherein said gene delivery vehicle is covalently coupled to said second member of said high affinity binding pair.

3. A combination as claimed in claim 1 wherein said gene delivery vehicle contains or expresses said second member of said high-affinity binding pair.

4. A combination as claimed in any one of claims 1 to 3 wherein said gene delivery vehicle is coupled to said targeting element by binding of said first member of said high affinity binding pair coupled to said targeting element to said second member of said high affinity binding pair presented by said gene delivery vehicle.

5. A combination according to any one of claims 1 to 4 wherein said targeting element is an antibody or antibody fragment.

6. A combination according to any one of claims 1 to 4 wherein said targeting element is selected from the group consisting of bombesin, gastrin-release peptide, cell adhesion peptides, substance P, neuromedin-B, neuromedin-C and metenkephalin.

7. A combination according to any one of claims 1 to 4 wherein said targeting element is selected from the group consisting of EGF, alpha- and beta-TGF, neurotensin, melanocyte stimulating hormone, follicle stimulating hormone, lutenizing hormone and human growth hormone.

8. A combination according to any one of claims 1 to 4 wherein said targeting element is a ligand for a cell surface receptor selected from the group consisting of low density lipoproteins, transferrin and insulin.

9. A combination according to any one of claims 1 to 4 wherein said targeting element is a fibrinolytic enzyme.

10. A combination according to any one of claims 1 to 4 wherein said targeting element is a immune accessory molecule selected from the group consisting of IL-1, IL-2 IL-3, IL-4, IL-5, IL-6, IL-7, IL-8, IL-9, IL-10, IL-11, IL-12, IL-13, α interferon, β interferon, γ interferon, GM-CSF, G-CSF, M-CSF and erythropoietin.

11. A combination according to any one of claims 1 to 10 wherein said high affinity binding pair is selected from the group consisting of biotin/avidin, cytostatin/papain, val-phosphonate/carboxypeptidase A, 4-chlorophyll a/b binding protein/ribulose-1,5-diphosphate carboxylase (4CABP/RuBisCo) and tobacco hornworm diuretic hormone/tobacco hornworm diuretic hormone receptor.

12. A combination according to any one of claims 1 to 10 wherein said high affinity binding pair is an antigen/antibody binding pair.

13. A combination according to any one of claims 1 to 12 wherein said gene delivery vehicle is a retroviral vector construct.

14. A combination according to claim 13 wherein said retroviral vector construct is constructed from a virus selected from the group consisting of Avian Leukosis Virus, Bovine Leukemia Virus, Murine Leukemia Virus, Mink-Cell Focus-Inducing Virus, Murine Sarcoma Virus, Reticuloendotheliosis virus, Gibbon Ape Leukemia Virus, Mason Pfizer Leukemia Virus and Rous Sarcoma Virus.

15. A combination according to claim 13 wherein said retroviral vector construct is constructed from a Murine Leukemia Virus selected from the group consisting of Abelson, Friend, Graffi, Gross, Kirsten, Harvey Sarcoma Virus, Moloney Murine Leukemia Virus and Rauscher.

16. A combination according to any one of claims 1 to 12 wherein said gene delivery vehicle is selected from the group consisting of poliovirus vectors, rhinovirus vectors, pox virus vectors, canary pox virus vectors, vaccinia virus vectors, influenza virus vectors, adenovirus vectors, parvoirus vectors, adeno-associated viral vectors, herpesvirus vectors, SV 40 vectors, HIV vectors, measles virus vectors, astrovirus vectors, corona virus vectors and Sindbis viral vectors.

17. A combination according to any one of claims 1 to 12 wherein said gene delivery vehicle is selected from the group consisting of polycation condensed nucleic acids, naked DNA and producer cell lines.

18. A combination according to any one of claims 1 to 17 wherein said gene delivery vehicle contains a heterologous sequence.

19. A combination according to claim 18 wherein said heterologous sequence is a gene encoding a cytotoxic protein.

20. A combination according to claim 19, where said cytotoxic protein is selected from the group consisting of ricin, abrin, diphtheria toxin, cholera toxin, gelonin, pokeweed, antiviral protein, tritin, Shigella toxin and Pseudomonas exotoxin A.

21. A combination according to claim 18 wherein said heterologous sequence is an antisense sequence.

22. A combination according to claim 18 wherein said heterologous sequence encodes an immune accessory molecule.

23. A combination according to claim 22 wherein said immune accessory molecule is selected from the group consisting of α interferon, β interferon, IL-1, IL-3, IL-4, IL-5, IL-6, IL-7, IL-8, IL-9, IL-10, IL-11 and IL-13.

24. A combination according to claim 22 wherein said immune accessory molecule is selected from the group consisting of IL-2, IL-12, IL-15 and gamma-interferon.

25. A combination according to claim 22 wherein said immune accessory molecule is selected from the group consisting of ICAM-1, ICAM-2, β-microglobin, LFA3, and HLA class I and HLA class II molecules.

26. A combination according to claim 18 wherein said heterologous sequence encodes a gene product that activates a compound with little or no cytotoxicity into a toxic product.

27. A combination according to claim 26 wherein said gene product is selected from the group consisting of HSVTK and VZVTK.

28. A combination according to claim 18 wherein said heterologous sequence is ribozyme.

29. A combination according to claim 18 wherein said heterologous sequence is a replacement gene.

30. A combination according to claim 29 wherein said replacement gene encodes a protein selected from the group consisting of Factor VIII, ADA, HPRT, CFTCR and the LDL Receptor.

31. A combination according to claim 18 wherein said heterologous sequence encodes an immunogenic portion of a virus selected from the group consisting of HBV, HCV, HPV, EBV, FeLV, FIV and HIV.

32. A gene delivery vehicle which presents a member of a high affinity binding pair selected from the binding pairs biotin/avidin, cytostatin/papain, val-phosphonate/carboxypeptidase A, 4-chlorophyll a/b binding protein/ribulose-1,5-diphosphate carboxylase (4CABP/RuBisCo) and tobacco hornworm diuretic hormone/tobacco hornworm diuretic hormone receptor.

33. A gene delivery vehicle as claimed in claim 32 wherein said gene delivery vehicle is covalently coupled to said binding pair member.

34. A gene delivery vehicle as claimed in claim 32 which contains or expresses said binding pair member.

35. A gene delivery vehicle according to any one of claims 32 to 34 which is a retroviral vector construct.

36. A gene delivery vehicle according to claim 35 which is constructed from a virus selected from the group consisting of Avian Leukosis Virus, Bovine Leukemia Virus, Murnie Leukemia Virus, Mink-Cell Focus-Inducing Virus, Murine Sarcoma Virus, Reticuloendotheliosis Virus, Gibbon Ape Leukemia Virus, Mason Pfizer Leukemia Virus and Rous Sarcoma Virus.

37. A gene delivery vehicle according to claim 35 wherein said retroviral vector construct is constructed from a Murine Leukemia Virus selected from the group consisting of Abelson, Friend, Graffi, Gross, Kirsten, Havey Sarcoma Virus, Moloney Murine Leukemia Virus and Rauscher.

38. A gene delivery vehicle according to any one of claims 32 to 34 wherein said gene delivery vehicle is selected from the group consisting of poliovirus vectors, rhinovirus vectors, pox virus vectors, canary pox virus vectors, vaccinia virus vectors, influenza virus vectors, adenovirus vectors, parovirus vectors, adeno-associated viral vectors, herpesvirus vectors, SV 40 vectors, HIV vectors, measles virus vectors, corona virus vectors, astrovirus vectors and Sindbis viral vectors.

39. A gene delivery vehicle according to any one of claims 32 to 34 wherein said gene delivery vehicle is selected from the group consisting of poycation condensed nucleic acids, naked DNA and producer cells.

40. A gene delivery vehicle according to any one of claims 32 to 39 which includes a heterologous sequence.

41. A gene delivery vehicle according to claim 40 wherein said heterologous sequence is a gene encoding a cytotoxic protein.

42. A gene delivery vehicle according to claim 41 wherein said cytotoxic protein is selected from the group consisting of ricin, abrin, diphtheria toxin, cholera toxin, gelonin, pokeweed, antiviral protein, tritin, Shigella toxin and Pseudomonas exotoxin A.

43. A gene delivery vehicle according to claim 40 wherein said heterologous sequence is an antisense sequence.

44. A gene delivery vehicle according to claim 40 wherein said heterologous sequence encodes an immune accessory molecule.

45. A gene delivery vehicle according to claim 44 wherein said immune accessory molecule is selected from the group consisting of α interferon, β interferon, IL-1, IL-3, IL-4, IL-5, IL-6, IL-7, IL-8, IL-9, IL-10, IL-11, and IL-13.

46. A gene delivery vehicle according to claim 44 wherein said immune accessory molecule is selected from the group consisting of IL-2, IL-12, IL-15 and gamma-interferon.

47. A gene delivery vehicle according to claim 44 wherein said immune accessory molecule is selected from the group consisting of ICAM-1, ICAM-2, b-microglobin, LFA3, and HLA class I and HLA class II molecules.

48. A gene delivery vehicle according to claim 40 wherein said heterologous sequence encodes a gene product that activates a compound with little or no cytotoxicity into a toxic product.

49. A gene delivery vehicle according to claim 48 wherein said gene product is selected from the group consisting of HSVTK and VZVTK.

50. A gene delivery vehicle according to claim 40 wherein said heterologous sequence is a ribozyme.

51. A gene delivery vehicle according to claim 40 wherein said heterologous sequence is a replacement gene.

52. A gene delivery vehicle according to claim 51 wherein said replacement gene encodes a protein selected from the group consisting of Factor VIII, ADA, HPRT, CFTCR and the LDL Receptor.

53. A gene delivery vehicle according to claim 40 wherein said heterologous sequence encodes an immunogenic portion of a virus selected from the group consisting of HBV, HCV, HPV, EBV, FeLV, FIV and HIV.

## Patentansprüche

1. Kombination aus:
(i) einem Targetingelement (Zielelement), das an ein erstes Mitglied eines Bindungspaars mit hoher Affinität gebunden ist, wobei das Targetingelement in der Lage ist, in spezifischer Weise mit einem ausgewählten Zelltyp in einem warmblütigen Tier zu binden, und
(ii) einem Genabgabevehikel, welches das zweite Mitglied des Bindungspaars mit hoher Affinität darstellt,
zur aufeinanderfolgenden oder gleichzeitigen Verabreichung, um das Genabgabevehikel auf Zellen des genannten ausgewählten Zelltyps zu richten.

2. Kombination nach Anspruch 1, dadurch **gekennzeichnet**, dass das Genabgabevehikel covalent mit dem zweiten Mitglied des Bindungspaars hoher Affinität verbunden ist.

3. Kombination nach Anspruch 1, dadurch **gekennzeichnet**, dass das Genabgabevehikel das zweite Mitglied des Bindungspaars mit hoher Affinität enthält oder exprimiert.

4. Kombination nach einem der Ansprüche 1 bis 3, dadurch **gekennzeichnet**, dass das Genabgabevehikel an das Targetingelement durch Bindung des ersten Mitglieds des Bindungspaars mit hoher Affinität, das an das Targetingelement gebunden ist, mit dem zweiten Mitglied des Bindungspaars mit hoher Affinität, das von dem Genabgabevehikel dargestellt wird, gebunden ist.

5. Kombination nach einem der Ansprüche 1 bis 4, dadurch **gekennzeichnet**, dass das Targetingelement ein Antikörper oder ein Antikörperfragment ist.

6. Kombination nach einem der Ansprüche 1 bis 4, dadurch **gekennzeichnet**, dass das Targetingelement ausgewählt ist aus der Gruppe bestehend aus Bombesin, Gastrin-releasing Peptid, Zelladhäsionspeptiden, Substanz P, Neuromedin-B, Neuromedin-C und Methenkephalin.

7. Kombination nach einem der Ansprüche 1 bis 4, dadurch **gekennzeichnet**, dass das Targetingelement ausgewählt ist aus der Gruppe bestehend aus EGF, alpha- und beta-TGF, Neurotensin, Melanozyten-stimulierendem Hormon, Follikel-stimulierendem Hormon, luteinisierendem Hormon und humanem Wachstumshormon.

8. Kombination nach einem der Ansprüche 1 bis 4, dadurch **gekennzeichnet**, dass das Targetingelement ein Ligand für einen Zelloberflächenrezeptor, ausgewählt aus der Gruppe bestehend aus Lipoproteinen niedriger Dichte, Transferrin und Insulin, ist.

9. Kombination nach einem der Ansprüche 1 bis 4, dadurch **gekennzeichnet**, dass das Targetingelement ein fibrinolytisches Enzym ist.

10. Kombination nach einem der Ansprüche 1 bis 4, dadurch **gekennzeichnet**, dass das Targetingelement ein akzessorisches Immunmolekül, ausgewählt aus der Gruppe bestehend aus IL-1, IL-2, IL-3, IL-4, IL-5, IL-6, IL-7, IL-8, IL-9, IL-10, IL-11, IL-12, IL-13, α-Interferon, β-Interferon, γ-Interferon, GM-CSF, G-CSF, M-CSF und Erythropoietin, ist.

11. Kombination nach einem der Ansprüche 1 bis 10, dadurch **gekennzeichnet**, dass das Bindungspaar mit hoher Affinität ausgewählt ist aus der Gruppe bestehend aus Biotin/Avidin, Cytostatin/Papain, Val-Phosphonat/Carboxypeptidase A, 4-Chlorophyll a/b-Bindungsprotein/Ribulose-1,5-diphosphatcarboxylase (4CABP/RuBisCo) und dem diuretischen Hormon des Tabakhornwurms/dem diuretischen Hormonrezeptor des Tabakhornwurms.

12. Kombination nach einem der Ansprüche 1 bis 10, dadurch **gekennzeichnet**, dass das Bindungspaar mit hoher Affinität ein Antigen/Antikörper-Bindungspaar ist.

13. Kombination nach einem der Ansprüche 1 bis 12, dadurch **gekennzeichnet**, dass das Genabgabevehikel ein retrovirales Vektorkonstrukt ist.

14. Kombination nach Anspruch 13, dadurch **gekennzeichnet**, dass das retrovirale Vektorkonstrukt ausgehend von einem Virus, ausgewählt aus der Gruppe bestehend aus dem Vogel-Leukosevirus, dem Rinder-Leukämievirus, dem Maus-Leukämievirus, dem Nerz-Zellfokus-induzierenden Virus, dem Maus-Sarkomvirus, dem Reticuloendotheliosevirus, dem Gibbonaffe-Leukämievirus, dem Mason-Pfizer-Leukämievirus und dem Rous-Sarkomvirus, konstruiert wird.

15. Kombination nach Anspruch 13, dadurch **gekennzeichnet**, dass das retrovirale Vektorkonstrukt ausgehend von einem Maus-Leukämievirus, ausgewählt aus der Gruppe bestehend aus Abelson, Friend, Graffi, Gross, Kirsten, Harvey-Sarkomvirus, dem Moloney-Maus-Leukämievirus und Rauscher, konstruiert ist.

16. Kombination nach einem der Ansprüche 1 bis 12, dadurch **gekennzeichnet**, dass das Genabgabevehikel ausgewählt ist aus der Gruppe bestehend aus Poliovirus-Vektoren, Rhinovirus-Vektoren, Pockenvirus-Vektoren, Kanarienvogelpockenvirus-Vektoren, Vacciniavirus-Vektoren, Influenzavirus-Vektoren, Adenovirus-Vektoren, Parvovirus-Vektoren, Adeno-assoziierten viralen Vektoren, Herpesvirus-Vektoren, SV-40-Vektoren, HIV-Vektoren, Masernvirus-Vektoren, Astrovirus-Vektoren, Coronavirus-Vektoren und viralen Sindbis Vektoren.

17. Kombination nach einem der Ansprüche 1 bis 12, dadurch **gekennzeichnet**, dass das Genabgabevehikel ausgewählt ist aus der Gruppe bestehend aus Polykation-kondensierten Nukleinsäuren, nackter DNA und Produzenten-Zelllinien.

18. Kombination nach einem der Ansprüche 1 bis 17, dadurch **gekennzeichnet**, dass das Genabgabevehikel eine heterologe Sequenz enthält.

19. Kombination nach Anspruch 18, dadurch **gekennzeichnet**, dass die heterologe Sequenz ein Gen ist, das für ein cytotoxisches Protein codiert.

20. Kombination nach Anspruch 19, dadurch **gekennzeichnet**, dass das cytotoxische Protein ausgewählt ist aus der Gruppe bestehend aus Ricin, Abrin, Diphtherietoxin, Choleratoxin, Gelonin, Pokeweed, antiviralem Protein, Tritin, Shigellatoxin und Pseudomonas-Exotoxin A.

21. Kombination nach Anspruch 18, dadurch **gekennzeichnet**, dass die heterologe Sequenz eine Antisensesequenz ist.

22. Kombination nach Anspruch 18, dadurch **gekennzeichnet**, dass die heterologe Sequenz für ein akzessorisches Immunmolekül codiert.

23. Kombination nach Anspruch 22, dadurch **gekennzeichnet**, dass das akzessorische Immunmolekül ausgewählt ist aus der Gruppe bestehend aus α-Interferon, β-Interferon, IL-1, IL-3, IL-4, IL-5, IL-6, IL-7, IL-8, IL-9, IL-10, IL-11 und IL-13.

24. Kombination nach Anspruch 22, dadurch **gekennzeichnet**, dass das akzessorische Immunmolekül ausgewählt ist aus der Gruppe bestehend aus IL-2, IL-12, IL-15 und gamma-Interferon.

25. Kombination nach Anspruch 22, dadurch **gekennzeichnet**, dass das akzessorische Immunmolekül ausgewählt ist aus der Gruppe bestehend aus ICAM-1, ICAM-2, β-Mikroglobin, LFA3 und HLA-Klasse-I- und HLA-Klasse-II-Molekülen.

26. Kombination nach Anspruch 18, dadurch **gekennzeichnet**, dass die heterologe Sequenz für ein Genprodukt codiert, das eine Verbindung mit geringer oder keiner Cytotoxizität in ein toxisches Produkt aktiviert.

27. Kombination nach Anspruch 26, dadurch **gekennzeichnet**, dass das Genprodukt ausgewählt ist aus der Gruppe bestehend aus HSVTK und VZVTK.

28. Kombination nach Anspruch 18, dadurch **gekennzeichnet**, dass die heterologe Sequenz ein Ribozym ist.

29. Kombination nach Anspruch 18, dadurch **gekennzeichnet**, dass die heterologe Sequenz ein Austauschgen ist.

30. Kombination nach Anspruch 29, dadurch **gekennzeichnet**, dass das Austauschgen für ein Protein, ausgewählt aus der Gruppe bestehend aus Faktor VIII, ADA, HPRT, CFTCR und dem LDL-Rezeptor, codiert.

31. Kombination nach Anspruch 18, dadurch **gekennzeichnet**, dass die heterologe Sequenz für einen immunogenen Teil eines Virus, ausgewählt aus der Gruppe bestehend aus HBV, HCV, HPV, EBV, FeLV, FIV und HIV, codiert.

32. Genabgabevehikel, das ein Mitglied eines Bindungspaars mit hoher Affinität, ausgewählt aus den Bindungspaaren Biotin/Avidin, Cytostatin/Papain, Val-Phosphonat/Carboxypeptidase A, 4-Chlorophyll a/b-Bindungsprotein/Ribulose-1,5-diphosphatcarboxylase (4CABP/RuBisCo) und dem diuretische Hormon des Tabakhornwurms/dem diuretischem Hormonrezeptor des Tabakhornwurms, codiert.

33. Genabgabevehikel nach Anspruch 32, dadurch **gekennzeichnet**, dass das Genabgabevehikel covalent mit dem Bindungspaarmitglied verbunden ist.

34. Genabgabevehikel nach Anspruch 32, das das Bindungspaarmitglied enthält oder exprimiert.

35. Genabgabevehikel nach einem der Ansprüche 32 bis 34, nämlich ein retrovirales Vektorkonstrukt.

36. Genabgabevehikel nach Anspruch 35, das ausgehend von einem Virus, ausgewählt aus der Gruppe bestehend aus dem Vogel-Leukosevirus, dem Rinder-Leukämievirus, dem Maus-Leukämievirus, dem Nerz-Zellfokus-induzierenden Virus, dem Maus-Sarkomvirus, dem Reticuloendotheliosevirus, dem Gibbonaffe-Leukämievirus, dem Mason-Pfizer-Leukämievirus und dem Rous-Sarkomvirus, konstruiert wird.

37. Genabgabevehikel nach Anspruch 35, dadurch **gekennzeichnet**, dass das retrovirale Vektorkonstrukt, ausgehend von einem Maus-Leukämievirus, ausgewählt aus der Gruppe bestehend aus Abelson, Friend, Graffi, Gross, Kirsten, Havey Sarkomvirus, Moloney Maus-Leukämievirus und Rauscher, konstruiert wird.

38. Genabgabevehikel nach einem der Ansprüche 32 bis 34, dadurch **gekennzeichnet**, dass das Genabgabevehikel ausgewählt ist aus der Gruppe bestehend aus Poliovirus-Vektoren, Rhinovirus-Vektoren, Pockenvirus-Vektoren, Kanarienvögelpockenvirus-Vektoren, Vacciniavirus-Vektoren, Influenzavirus-Vektoren, Adenovirus-Vektoren, Parovirus-Vektoren, Adeno-assoziierten viralen Vektoren, Herpesvirus-Vektoren, SV-40-Vektoren, HIV-Vektoren, Masernvirus-Vektoren, Coronavirus-Vektoren, Astrovirus-Vektoren und viralen Sindbis Vektoren.

39. Genabgabevehikel nach einem der Ansprüche 32 bis 34, dadurch **gekennzeichnet**, dass das Genabgabevehikel ausgewählt ist aus der Gruppe bestehend aus Polykation-kondensierten Nukleinsäuren, nackter DNA und Produzentenzellen.

40. Genabgabevehikel nach einem der Ansprüche 32 bis 39, das eine heterologe Sequenz beinhaltet.

41. Genabgabevehikel nach Anspruch 40, dadurch **gekennzeichnet**, dass die heterologe Sequenz ein Gen ist, das für ein cytotoxisches Protein codiert.

42. Genabgabevehikel nach Anspruch 41, dadurch **gekennzeichnet**, dass das cytotoxische Protein ausgewählt ist aus der Gruppe bestehend aus Ricin, Abrin, Diphtherietoxin, Choleratoxin, Gelonin, Pokeweed, antiviralem Protein, Tritin, Shigella-Toxin und Pseudomonas-Exotoxin A.

43. Genabgabevehikel nach Anspruch 40, dadurch **gekennzeichnet**, dass die heterologe Sequenz eine Antisensesequenz ist.

44. Genabgabevehikel nach Anspruch 40, dadurch **gekennzeichnet**, dass die heterologe Sequenz für ein akzessorisches Immunmolekül codiert.

45. Genabgabevehikel nach Anspruch 44, dadurch **gekennzeichnet**, dass das immunakzessorische Molekül ausgewählt ist aus der Gruppe bestehend aus α-Interferon, β-Interferon, IL-1, IL-3, IL-4, IL-5, IL-6, IL-7, IL-8, IL-9, IL-10, IL-11 und IL-13.

46. Genabgabevehikel nach Anspruch 44, dadurch **gekennzeichnet**, dass das akzessorische Immunmolekül ausgewählt ist aus der Gruppe bestehend aus IL-2, IL-12, IL-15 und gamma-Interferon.

47. Genabgabevehikel nach Anspruch 44, dadurch **gekennzeichnet**, dass das akzessorische Immunmolekül ausgewählt ist aus der Gruppe bestehend aus ICAM-1, ICAM-2, β-Mikroglobin, LFA3 und HLA-Klasse-I- und HLA-Klasse-II-Molekülen.

48. Genabgabevehikel nach Anspruch 40, dadurch **gekennzeichnet**, dass die heterologe Sequenz für ein Genprodukt codiert, das eine Verbindung mit geringer oder keiner Cytotoxizität in ein toxisches Produkt aktiviert.

49. Genabgabevehikel nach Anspruch 48, dadurch **gekennzeichnet**, dass das Genprodukt ausgewählt ist aus der Gruppe bestehend aus HSVTK und VZVTK.

50. Genabgabevehikel nach Anspruch 40, dadurch **gekennzeichnet**, dass die heterologe Sequenz ein Ribozym ist.

51. Genabgabevehikel nach Anspruch 40, dadurch **gekennzeichnet**, dass die heterologe Sequenz ein Austauschgen ist.

52. Genabgabevehikel nach Anspruch 51, dadurch **gekennzeichnet**, dass das Austauschgen für ein Protein, ausgewählt aus der Gruppe bestehend aus Faktor VIII, ADA, HPRT, CFTCR und dem LDL-Rezeptor, codiert.

53. Genabgabevehikel nach Anspruch 40, dadurch **gekennzeichnet**, dass die heterologe Sequenz für einen immunogenen Teil eines Virus, ausgewählt aus der Gruppe bestehend aus HBV, HCV, HPV, EBV, FeLV, FIV und HIV, codiert.

## Revendications

1. Combinaison de :
(i) un élément de ciblage couplé à un premier membre d'une paire de liaison à affinité élevée, ledit élément de ciblage étant capable de se lier spécifiquement à un type de cellule choisi chez un animal à sang chaud ; et
(ii) un véhicule d'apport de gène présentant le second membre de ladite paire de liaison à affinité élevée,
pour une administration séquentielle ou simultanée afin de cibler ledit véhicule d'apport de gène aux cellules dudit type de cellule choisi.

2. Combinaison selon la revendication 1, dans laquelle ledit véhicule d'apport de gène est couplé par covalence audit second membre de ladite paire de liaison à affinité élevée.

3. Combinaison selon la revendication 1, dans laquelle ledit véhicule d'apport de gène contient ou exprime ledit second membre de ladite paire de liaison à affinité élevée.

4. Combinaison selon l'une quelconque des revendications 1 à 3, dans laquelle ledit véhicule d'apport de gène est couplé audit élément de ciblage par liaison dudit premier membre de ladite paire de liaison à affinité élevée couplé audit élément de ciblage audit second membre de ladite paire de liaison à affinité élevée présentée par ledit véhicule d'apport de gène.

5. Combinaison selon l'une quelconque des revendications 1 à 4, dans laquelle ledit élément de ciblage est un anticorps ou un fragment d'anticorps.

6. Combinaison selon l'une quelconque des revendications 1 à 4, dans laquelle ledit élément de ciblage est choisi dans le groupe constitué par bombésine, peptide de libération de la gastrine, peptides d'adhésion cellulaire, substance P, neuromédine-B, neuromédine-C et metenképhaline.

7. Combinaison selon l'une quelconque des revendications 1 à 4, dans laquelle ledit élément de ciblage est choisi dans le groupe constitué par EGF, alpha- et bêta-TGF, neurotensine, hormone de stimulation de mélanocyte, hormone de stimulation folliculaire, hormone lutéinisante, et hormone de croissance humaine.

8. Combinaison selon l'une quelconque des revendications 1 à 4, dans laquelle ledit élément de ciblage est un ligand pour un récepteur de surface cellulaire choisi dans le groupe constitué par lipoprotéines de faible densité, transferrine et insuline.

9. Combinaison selon l'une quelconque des revendications 1 à 4, dans laquelle ledit élément de ciblage est une enzyme fibrinolytique.

10. Combinaison selon l'une quelconque des revendications 1 à 4, dans laquelle ledit élément de ciblage est une molécule accessoire immune choisie dans le groupe constitué par IL-1, IL-2, IL-3, IL-4, IL-5, IL-6, IL-7, IL-8, IL-9, IL-10, IL-11, IL-12, IL-13, interféron α, interféron β, interféron γ, GM-CSF, G-CSF, M-CSF et érythropoïétine.

11. Combinaison selon l'une quelconque des revendications 1 à 10, dans laquelle ladite paire de liaison à affinité élevée est choisie dans le groupe constitué par biotine/avidine, cytostatine/papaïne, val-phosphonate/carboxypeptidase A, protéine de liaison à la 4-chlorophylle a/b ribulose-1,5-diphosphate carboxylase (4CABP/RuBisCo) et hormone diurétique de sphinx du tabac/récepteur d'hormone diurétique de sphinx du tabac.

12. Combinaison selon l'une quelconque des revendications 1 à 10, dans laquelle ladite paire de liaison à affinité élevée est une paire de liaison antigène/anticorps.

13. Combinaison selon l'une quelconque des revendications 1 à 12, dans laquelle ledit véhicule d'apport de gène est une construction de vecteur rétroviral.

14. Combinaison selon la revendication 13, dans laquelle ladite construction de vecteur rétroviral est construite à partir d'un virus choisi dans le groupe constitué par virus de leucose aviaire, virus de leucémie bovine, virus de leucémie murine, virus induisant un foyer cellulaire de vison, virus de sarcome murin, virus de réticulo-endothéliose, virus de leucémie du gibbon, virus de la leucémie de Mason Pfizer et virus du sarcome de Rous.

15. Combinaison selon la revendication 13, dans laquelle ladite construction de vecteur rétroviral est construite à partir d'un virus de leucémie murine choisi dans le groupe constitué par virus de sarcome d'Harvey, Abelson, Friend, Graffi, Gross, Kirsten, virus de la leucémie murine de Moloney et virus de Rauscher.

16. Combinaison selon l'une quelconque des revendications 1 à 12, dans laquelle ledit véhicule d'apport de gène est choisi dans le groupe constitué par vecteurs poliovirus, vecteurs rhinovirus, vecteurs poxvirus, vecteurs poxvirus de canari, vecteurs virus de la vaccine, vecteurs virus de la grippe, vecteurs adénovirus, vecteurs parvovirus, vecteurs viraux adénoassociés, vecteurs herpèsvirus, vecteurs SV40, vecteurs HIV, vecteurs virus de la rougeole, vecteurs astrovirus, vecteurs coronavirus, et vecteurs viraux de Sindbis.

17. Combinaison selon l'une quelconque des revendications 1 à 12, dans laquelle ledit véhicule d'apport de gène est choisi dans le groupe constitué par acides nucléiques condensés à des polycations, ADN nu et lignées cellulaires productrices.

18. Combinaison selon l'une quelconque des revendications 1 à 17, dans laquelle ledit véhicule d'apport de gène contient une séquence hétérologue.

19. Combinaison selon la revendication 18, dans laquelle ladite séquence hétérologue est un gène codant une protéine cytotoxique.

20. Combinaison selon la revendication 19, dans laquelle ladite protéine cytotoxique est choisie dans le groupe constitué par ricine, abrine, toxine diphtérique, toxine cholérique, gélonine, mitogène de phytolaque, protéine antivirale, tritine, toxine de Shigella et exotoxine A de Pseudomonas.

21. Combinaison selon la revendication 18, dans laquelle ladite séquence hétérologue est une séquence antisens.

22. Combinaison selon la revendication 18, dans laquelle ladite séquence hétérologue code une molécule accessoire immune.

23. Combinaison selon la revendication 22, dans laquelle ladite molécule accessoire immune est choisie dans le groupe constitué par interféron α, interféron β, IL-1, IL-3, IL-4, IL-5, IL-6, IL-7, IL-8, IL-9, IL-10, IL-11 et IL-13.

24. Combinaison selon la revendication 22, dans laquelle ladite molécule accessoire immune est choisie dans le groupe constitué par IL-2, IL-12, IL-15 et interféron gamma.

25. Combinaison selon la revendication 22, dans laquelle ladite molécule accessoire immune est choisie dans le groupe constitué par ICAM-1, ICAM-2, microglobuline-β, LFA3, et molécules HLA de classe I et HLA de classe II.

26. Combinaison selon la revendication 18, dans laquelle ladite séquence hétérologue code un produit de gène qui active un composé ne présentant que peu ou pas de cytotoxicité en un produit toxique.

27. Combinaison selon la revendication 26, dans laquelle ledit produit de gène est choisi dans le groupe constitué par HSVTK et VZVTK.

28. Combinaison selon la revendication 18, dans laquelle ladite séquence hétérologue est un ribozyme.

29. Combinaison selon la revendication 18, dans laquelle ladite séquence hétérologue est un gène de remplacement.

30. Combinaison selon la revendication 29, dans laquelle ledit gène de remplacement code une protéine choisie dans le groupe constitué par facteur VIII, ADA, HPRT, CFTCR et le récepteur LDL.

31. Combinaison selon la revendication 18, dans laquelle ladite séquence hétérologue code une portion immunogène d'un virus choisi dans le groupe constitué par HBV, HCV, HPV, EBV, FeLV, FIV et HIV.

32. Véhicule d'apport de gène qui présente un membre d'une paire de liaison à affinité élevée choisi parmi les paires de liaison biotine/avidine, cytostatine/papaïne, val-phosphonate/carboxypeptidase A, protéine de liaison à la 4-chlorophylle a/b ribulose-1,5-diphosphate carboxylase (4CABP/RuBisCo) et hormone diurétique de sphinx du tabac/récepteur d'hormone diurétique de sphinx du tabac.

33. Véhicule d'apport de gène tel que revendiqué dans la revendication 32, ledit véhicule d'apport de gène étant couplé par covalence audit membre de paire de liaison.

34. Véhicule d'apport de gène tel que revendiqué dans la revendication 32, qui contient ou exprime ledit membre de paire de liaison.

35. Véhicule d'apport de gène selon l'une quelconque des revendications 32 à 34, qui est une construction de vecteur rétroviral.

36. Véhicule d'apport de gène selon la revendication 35, qui est construit à partir d'un virus choisi dans le groupe constitué par virus de leucose aviaire, virus de leucémie bovine, virus de leucémie murine, virus induisant un foyer cellulaire de vison, virus de sarcome murin, virus de réticuloendothéliose, virus de leucémie du gibbon, virus de la leucémie de Mason Pfizer et virus du sarcome de Rous.

37. Véhicule d'apport de gène selon la revendication 35, dans lequel ladite construction de vecteur rétroviral est construite à partir d'un virus de leucémie murine choisi dans le groupe constitué par virus de sarcome d'Harvey, Abelson, Friend, Graffi, Gross, Kirsten, virus de la leucémie murine de Moloney et virus de Rauscher.

38. Véhicule d'apport de gène selon l'une quelconque des revendications 32 à 34, ledit véhicule d'apport de gène étant choisi dans le groupe constitué par vecteurs poliovirus, vecteurs rhinovirus, vecteurs poxvirus, vecteurs poxvirus de canari, vecteurs virus de la vaccine, vecteurs virus de la grippe, vecteurs adénovirus, vecteurs parvovirus, vecteurs viraux adénoassociés, vecteurs herpèsvirus, vecteurs SV40, vecteurs HIV, vecteurs virus de la rougeole, vecteurs coronavirus, vecteurs astrovirus, et vecteurs viraux de Sindbis.

39. Véhicule d'apport de gène selon l'une quelconque des revendications 32 à 34, ledit véhicule d'apport de gène étant choisi dans le groupe constitué par acides nucléiques condensés à des polycations, ADN nu et cellules productrices.

40. Véhicule d'apport de gène selon l'une quelconque des revendications 32 à 39, qui inclut une séquence hétérologue.

41. Véhicule d'apport de gène selon la revendication 40, dans lequel ladite séquence hétérologue est un gène codant une protéine cytotoxique.

42. Véhicule d'apport de gène selon la revendication 41, dans lequel ladite protéine cytotoxique est choisie dans le groupe constitué par ricine, abrine, toxine diphtérique, toxine cholérique, gélonine, mitogène de phytolaque, protéine antivirale, tritine, toxine de Shigella et exotoxine A de Pseudomonas.

43. Véhicule d'apport de gène selon la revendication 40, dans lequel ladite séquence hétérologue est une séquence antisens.

44. Véhicule d'apport de gène selon la revendication 40, dans lequel ladite séquence hétérologue code une molécule accessoire immune.

45. Véhicule d'apport de gène selon la revendication 44, dans lequel ladite molécule accessoire immune est choisie dans le groupe constitué par interféron α, interféron β, IL-1, IL-3, IL-4, IL-5, IL-6, IL-7, IL-8, IL-9, IL-10, IL-11 et IL-13.

46. Véhicule d'apport de gène selon la revendication 44, dans lequel ladite molécule accessoire immune est choisie dans le groupe constitué par IL-2, IL-12, IL-15 et interféron gamma.

47. Véhicule d'apport de gène selon la revendication 44, dans lequel ladite molécule accessoire immune est choisie dans le groupe constitué par ICAM-1, ICAM-2, microglobuline-β, LFA3, et molécules HLA de classe I et HLA de classe II.

48. Véhicule d'apport de gène selon la revendication 40, dans lequel ladite séquence hétérologue code un produit de gène qui active un composé ne présentant que peu ou pas de cytotoxicité en un produit toxique.

49. Véhicule d'apport de gène selon la revendication 48, dans lequel ledit produit de gène est choisi dans le groupe constitué par HSVTK et VZVTK.

50. Véhicule d'apport de gène selon la revendication 40, dans lequel ladite séquence hétérologue est un ribozyme.

51. Véhicule d'apport de gène selon la revendication 40, dans lequel ladite séquence hétérologue est un gène de remplacement.

52. Véhicule d'apport de gène selon la revendication 51, dans lequel ledit gène de remplacement code une protéine choisie dans le groupe constitué par facteur VIII, ADA, HPRT, CFTCR et le récepteur LDL.

53. Véhicule d'apport de gène selon la revendication 40, dans lequel ladite séquence hétérologue code une portion immunogène d'un virus choisi dans le groupe constitué par HBV, HCV, HPV, EBV, FeLV, FIV et HIV.


### SEQUENCE LISTING

(1) GENERAL INFORMATION:
   (i) APPLICANTS: Jolly, Douglas, J.
      Barber, Jack R.
      Respess, James G.
      Moore, Margaret
   (ii) TITLE OF INVENTION: Compositions and Methods for Targeting Gene Delivery Vehicles
   (iii) NUMBER OF SEQUENCES: 26
   (iv) CORRESPONDENCE ADDRESS:
      (A) ADDRESSEE: Seed & Berry
      (B) STREET: 6300 Columbia Center: 701 Fifth Avenue
      (C) CITY: Seattle
      (D) STATE: Washington
      (E) COUNTRY: USA
      (F) ZIP: 98104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE: 15-MAY-1995
      (C) CLASSIFICATION:
   (viii) ATTORNEY/AGENT INFORMATION:
      (A) NAME: McMasters, David D.
      (B) REGISTRATION NUMBER: 33.963
      (C) REFERENCE/DOCKET NUMBER: 930049.431PC
   (ix) TELECOMMUNICATION INFORMATION:
      (A) TELEPHONE: (206)622-4900
      (B) TELEFAX: (206)682-6031
(2) INFORMATION FOR SEQ ID NO:1:
   (i) SEQUENCE CHARACTERISTICS:
      (A) LENGTH: 8332 base pairs
      (B) TYPE: nucleic acid
      (C) STRANDEDNESS: single
      (D) TOPOLOGY: linear
   (xi) SEQUENCE DESCRIPTION: SEQ ID NO:1:
   GCGCCAGTCC TCCGATTGAC TGAGTCGCCC GGGTACCCGT GTATCCAATA AACCCTCTTG 60
   CAGTTGCATC CGACTTGTGG TCTCGCTGTT CCTTGGGAGG GTCTCCTCTG AGTGATTGAC 120
   TACCCGTCAG CGGGGGTCTT TCATTTGGGG GCTCGTCCGG GATCGGGAGA CCCCTGCCCA 180
   GGGACCACCG ACCCACCACC GGGAGGTAAG CTGGCCAGCA ACTTATCTGT GTCTGTCCGA 240
   TTGTCTAGTG TCTATGACTG ATTTTATGCG CCTGCGTCGG TACTAGTTAG CTAACTAGCT 300
   CTGTATCTGG CGGACCCGTG GTGGAACTGA CGAGTTCGGA ACACCCGGCC GCAACCCTGG 360
   GAGACGTCCC AGGGACTTCG GGGGCCGTTT TTGTGGCCCG ACCTGAGTCC AAAAATCCCG 420
   ATCGTTTTGG ACTCTTTGGT GCACCCCCCT TAGAGGAGGG ATATGTGGTT CTGGTAGGAG 480
   ACGAGAACCT AAAACAGTTC CCGCCTCCGT CTGAATTTTT GCTTTCGGTT TGGGACCGAA 540
   GCCGCGCCGC GCGTCTTGTC TGCTGCAGCA TCGTTCTGTG TTGTCTCTGT CTGACTGTGT 600
   TTCTGTATTT GTCTGAGAAT ATGGGCCAGA CTGTTACCAC TCCCTTAAGT TTGACCTTAG 660
   GTCACTGGAA AGATGTCGAG CGGATCGCTC ACAACCAGTC GGTAGATGTC AAGAAGAGAC 720
   GTTGGGTTAC CTTCTGCTCT GCAGAATGGC CAACCTTTAA CGTCGGATGG CCGCGAGACG 780
   GCACCTTTAA CCGAGACCTC ATCACCCAGG TTAAGATCAA GGTCTTTTCA CCTGGCCCGC 840
   ATGGACACCC AGACCAGGTC CCCTACATCG TGACCTGGGA AGCCTTGGCT TTTGACCCCC 900
   CTCCCTGGGT CAAGCCCTTT GTACACCCTA AGCCTCCGCC TCCTCTTCCT CCATCCGCCC 960
   CGTCTCTCCC CCTTGAACCT CCTCGTTCGA CCCCGCCTCG ATCCTCCCTT TATCCAGCCC 1020
   TCACTCCTTC TCTAGGCGCC AAACCTAAAC CTCAAGTTCT TTCTGACAGT GGGGGGCCGC 1080
   TCATCGACCT ACTTACAGAA GACCCCCCGC CTTATAGGGA CCCAAGACCA CCCCCTTCCG 1140
   ACAGGGACGG AAATGGTGGA GAAGCGACCC CTGCGGGAGA GGCACCGGAC CCCTCCCCAA 1200
   TGGCATCTCG CCTACGTGGG AGACGGGAGC CCCCTGTGGC CGACTCCACT ACCTCGCAGG 1260
   CATTCCCCCT CCGCGCAGGA GGAAACGGAC AGCTTCAATA CTGGCCGTTC TCCTCTTCTG 1320
   ACCTTTACAA CTGGAAAAAT AATAACCCTT CTTTTTCTGA AGATCCAGGT AAACTGACAG 1380
   CTCTGATCGA GTCTGTTCTC ATCACCCATC AGCCCACCTG GGACGACTGT CAGCAGCTGT 1440
   TGGGGACTCT GCTGACCGGA GAAGAAAAAC AACGGGTGCT CTTAGAGGCT AGAAAGGCGG 1500
   TGCGGGGCGA TGATGGGCGC CCCACTCAAC TGCCCAATGA AGTCGATGCC GCTTTTCCCC 1560
   TCGAGCGCCC AGACTGGGAT TACACCACCC AGGCAGGTAG GAACCACCTA GTCCACTATC 1620
   GCCAGTTGCT CCTAGCGGGT CTCCAAAACG CGGGCAGAAG CCCCACCAAT TTGGCCAAGG 1680
   TAAAAGGAAT AACACAAGGG CCCAATGAGT CTCCCTCGGC CTTCCTAGAG AGACTTAAGG 1740
   AAGCCTATCG CAGGTACACT CCTTATGACC CTGAGGACCC AGGGCAAGAA ACTAATGTGT 1800
   CTATGTCTTT CATTTGGCAG TCTGCCCCAG ACATTGGGAG AAAGTTAGAG AGGTTAGAAG 1860
   ATTTAAAAAA CAAGACGCTT GGAGATTTGG TTAGAGAGGC AGAAAAGATC TTTAATAAAC 1920
   GAGAAACCCC GGAAGAAAGA GAGGAACGTA TCAGGAGAGA AACAGAGGAA AAAGAAGAAC 1980
   GCCGTAGGAC AGAGGATGAG CAGAAAGAGA AAGAAAGAGA TCGTAGGAGA CATAGAGAGA 2040
   TGAGCAAGCT ATTGGCCACT GTCGTTAGTG GACAGAAACA GGATAGACAG GGAGGAGAAC 2100
   GAAGGAGGTC CCAACTCGAT CGCGACCAGT GTGCCTACTG CAAAGAAAAG GGGCACTGGG 2160
   CTAAAGATTG TCCCAAGAAA CCACGAGGAC CTCGGGGACC AAGACCCCAG ACCTCCCTCC 2220
   TGACCCTAGA TGACTAGGGA GGTCAGGGTC AGGAGCCCCC CCCTGAACCC AGGATAACCC 2280
   TCAAAGTCGG GGGGCAACCC GTCACCTTCC TGGTAGATAC TGGGGCCCAA CACTCCGTGC 2340
   TGACCCAAAA TCCTGGACCC CTAAGTGATA AGTCTGCCTG GGTCCAAGGG GCTACTGGAG 2400
   GAAAGCGGTA TCGCTGGACC ACGGATCGCA AAGTACATCT AGCTACCGGT AAGGTCACCC 2460
   ACTCTTTCCT CCATGTACCA GACTGTCCCT ATCCTCTGTT AGGAAGAGAT TTGCTGACTA 2520
   AACTAAAAGC CCAAATCCAC TTTGAGGGAT CAGGAGCTCA GGTTATGGGA CCAATGGGGC 2580
   AGCCCCTGCA AGTGTTGACC CTAAATATAG AAGATGAGCA TCGGCTACAT GAGACCTCAA 2640
   AAGAGCCAGA TGTTTCTCTA GGGTCCACAT GGCTGTCTGA TTTTCCTCAG GCCTGGGCGG 2700
   AAACCGGGGG CATGGGACTG GCAGTTCGCC AAGCTCCTCT GATCATACCT CTGAAAGCAA 2760
   CCTCTACCCC CGTGTCCATA AAACAATACC CCATGTCACA AGAAGCCAGA CTGGGGATCA 2820
   AGCCCCACAT ACAGAGACTG TTGGACCAGG GAATACTGGT ACCCTGCCAG TCCCCCTGGA 2880
   ACACGCCCCT GCTACCCGTT AAGAAACCAG GGACTAATGA TTATAGGCCT GTCCAGGATC 2940
   TGAGAGAAGT CAACAAGCGG GTGGAAGACA TCCACCCCAC CGTGCCCAAC CCTTACAACC 3000
   TCTTGAGCGG GCTCCCACCG TCCCACCAGT GGTACACTGT GCTTGATTTA AAGGATGCCT 3060
   TTTTCTGCCT GAGACTCCAC CCCACCAGTC AGCCTCTCTT CGCCTTTGAG TGGAGAGATC 3120
   CAGAGATGGG AATCTCAGGA CAATTGACCT GGACCAGACT CCCACAGGGT TTCAAAAACA 3180
   GTCCCACCCT GTTTGATGAG GCACTGCACA GAGACCTAGC AGACTTCCGG ATCCAGCACC 3240
   CAGACTTGAT CCTGCTACAG TACGTGGATG ACTTACTGCT GGCCGCCACT TCTGAGCTAG 3300
   ACTGCCAACA AGGTACTCGG GCCCTGTTAC AAACCCTAGG GAACCTCGGG TATCGGGCCT 3360
   CGGCCAAGAA AGCCCAAATT TGCCAGAAAC AGGTCAAGTA TCTGGGGTAT CTTCTAAAAG 3420
   AGGGTCAGAG ATGGCTGACT GAGGCCAGAA AAGAGACTGT GATGGGGCAG CCTACTCCGA 3480
   AGACCCCTCG ACAACTAAGG GAGTTCCTAG GGACGGCAGG CTTCTGTCGC CTCTGGATCC 3540
   CTGGGTTTGC AGAAATGGCA GCCCCCTTGT ACCCTCTCAC CAAAACGGGG ACTCTGTTTA 3600
   ATTGGGGCCC AGACCAACAA AAGGCCTATC AAGAAATCAA GCAAGCTCTT CTAACTGCCC 3660
   CAGCCCTGGG GTTGCCAGAT TTGACTAAGC CCTTTGAACT CTTTGTCGAC GAGAAGCAGG 3720
   GCTACGCCAA AGGTGTCCTA ACGCAAAAAC TGGGACCTTG GCGTCGGCCG GTGGCCTACC 3780
   TGTCCAAAAA GCTAGACCCA GTAGCAGCTG GGTGGCCCCC TTGCCTACGG ATGGTAGCAG 3840
   CCATTGCCGT ACTGACAAAG GATGCAGGCA AGCTAACCAT GGGACAGCCA CTAGTCATTC 3900
   TGGCCCCCCA TGCAGTAGAG GCACTAGTCA AACAACCCCC CGACCGCTGG CTTTCCAACG 3960
   CCCGGATGAC TCACTATCAG GCCTTGCTTT TGGACACGGA CCGGGTCCAG TTCGGACCGG 4020
   TGGTAGCCCT GAACCCGGCT ACGCTGCTCC CACTGCCTGA GGAAGGGCTG CAACACAACT 4080
   GCCTTGATAT CCTGGCCGAA GCCCACGGAA CCCGACCCGA CCTAACGGAC CAGCCGCTCC 4140
   CAGACGCCGA CCACACCTGG TACACGGATG GAAGCAGTCT CTTACAAGAG GGACAGCGTA 4200
   AGGCGGGAGC TGCGGTGACC ACCGAGACCG AGGTAATCTG GGCTAAAGCC CTGCCAGCCG 4260
   GGACATCCGC TCAGCGGGCT GAACTGATAG CACTCACCCA GGCCCTAAAG ATGGCAGAAG 4320
   GTAAGAAGCT AAATGTTTAT ACTGATAGCC GTTATGCTTT TGCTACTGCC CATATCCATG 4380
   GAGAAATATA CAGAAGGCGT GGGTTGCTCA CATCAGAAGG CAAAGAGATC AAAAATAAAG 4440
   ACGAGATCTT GGCCCTACTA AAAGCCCTCT TTCTGCCCAA AAGACTTAGC ATAATCCATT 4500
   GTCCAGGACA TCAAAAGGGA CACAGCGCCG AGGCTAGAGG CAACCGGATG GCTGACCAAG 4560
   CGGCCCGAAA GGCAGCCATC ACAGAGACTC CAGACACCTC TACCCTCCTC ATAGAAAATT 4620
   CATCACCCTA CACCTCAGAA CATTTTCATT ACACAGTGAC TGATATAAAG GACCTAACCA 4680
   AGTTGGGGGC CATTTATGAT AAAACAAAGA AGTATTGGGT CTACCAAGGA AAACCTGTGA 4740
   TGCCTGACCA GTTTACTTTT GAATTATTAG ACTTTCTTCA TCAGCTGACT CACCTCAGCT 4800
   TCTCAAAAAT GAAGGCTCTC CTAGAGAGAA GCCACAGTCC CTACTACATG CTGAACCGGG 4860
   ATCGAACACT CAAAAATATC ACTGAGACCT GCAAAGCTTG TGCACAAGTC AACGCCAGCA 4920
   AGTCTGCCGT TAAACAGGGA ACTAGGGTCC GCGGGCATCG GCCCGGCACT CATTGGGAGA 4980
   TCGATTTCAC CGAGATAAAG CCCGGATTGT ATGGCTATAA ATATCTTCTA GTTTTTATAG 5040
   ATACCTTTTC TGGCTGGATA GAAGCCTTCC CAACCAAGAA AGAAACCGCC AAGGTCGTAA 5100
   CCAAGAAGCT ACTAGAGGAG ATCTTCCCCA GGTTCGGCAT GCCTCAGGTA TTGGGAACTG 5160
   ACAATGGGCC TGCCTTCGTC TCCAAGGTGA GTCAGACAGT GGCCGATCTG TTGGGGATTG 5220
   ATTGGAAATT ACATTGTGCA TACAGACCCC AAAGCTCAGG CCAGGTAGAA AGAATGAATA 5280
   GAACCATCAA GGAGACTTTA ACTAAATTAA CGCTTGCAAC TGGCTCTAGA GACTGGGTGC 5340
   TCCTACTCCC CTTAGCCCTG TACCGAGCCC GCAACACGCC GGGCCCCCAT GGCCTCACCC 5400
   CATATGAGAT CTTATATGGG GCACCCCCGC CCCTTGTAAA CTTCCCTGAC CCTGACATGA 5460
   CAAGAGTTAC TAACAGCCCC TCTCTCCAAG CTCACTTACA GGCTCTCTAC TTAGTCCAGC 5520
   ACGAAGTCTG GAGACCTCTG GCGGCAGCCT ACCAAGAACA ACTGGACCGA CCGGTGGTAC 5580
   CTCACCCTTA CCGAGTCGGC GACACAGTGT GGGTCCGCCG ACACCAGACT AAGAACCTAG 5640
   AACCTCGCTG GAAAGGACCT TACACAGTCC TGCTGACCAC CCCCACCGCC CTCAAAGTAG 5700
   ACGGCATCGC AGCTTGGATA CACGCCGCCC ACGTGAAGGC TGCCGACCCC GGGGGTGGAC 5760
   CATCCTCTAG ACTGACATGG CGCGTTCAAC GCTCTCAAAA CCCCTTAAAA ATAAGGTTAA 5820
   CCCGCGAGGC CCCCTAATCC CCTTAATTCT TCTGATGCTC AGAGGGGTCA GTACTGCTTC 5880
   GCCCGGCTCC AGTCCTCATC AAGTCTATAA TATCACCTGG GAGGTAACCA ATGGAGATCG 5940
   GGAGACGGTA TGGGCAACTT CTGGCAACCA CCCTCTGTGG ACCTGGTGGC CTGACCTTAC 6000
   CCCAGATTTA TGTATGTTAG CCCACCATGG ACCATCTTAT TGGGGGCTAG AATATCAATC 6060
   CCCTTTTTCT TCTCCCCCGG GGCCCCCTTG TTGCTCAGGG GGCAGCAGCC CAGGCTGTTC 6120
   CAGAGACTGC GAAGAACCTT TAACCTCCCT CACCCCTCGG TGCAACACTG CCTGGAACAG 6180
   ACTCAAGCTA GACCAGACAA CTCATAAATC AAATGAGGGA TTTTATGTTT GCCCCGGGCC 6240
   CCACCGCCCC CGAGAATCCA AGTCATGTGG GGGTCCAGAC TCCTTCTACT GTGCCTATTG 6300
   GGGCTGTGAG ACAACCGGTA GAGCTTACTG GAAGCCCTCC TCATCATGGG ATTTCATCAC 6360
   AGTAAACAAC AATCTCACCT CTGACCAGGC TGTCCAGGTA TGCAAAGATA ATAAGTGGTG 6420
   CAACCCCTTA GTTATTCGGT TTACAGACGC CGGGAGACGG GTTACTTCCT GGACCACAGG 6480
   ACATTACTGG GGCTTACGTT TGTATGTCTC CGGACAAGAT CCAGGGCTTA CATTTGGGAT 6540
   CCGACTCAGA TACCAAAATC TAGGACCCCG CGTCCCAATA GGGCCAAACC CCGTTCTGGC 6600
   AGACCAACAG CCACTCTCCA AGCCCAAACC TGTTAAGTCG CCTTCAGTCA CCAAACCACC 6660
   CAGTGGGACT CCTCTCTCCC CTACCCAACT TCCACCGGCG GGAACGGAAA ATAGGCTGCT 6720
   AAACTTAGTA GACGGAGCCT ACCAAGCCCT CAACCTCACC AGTCCTGACA AAACCCAAGA 6780
   GTGCTGGTTG TGTCTAGTAG CGGGACCCCC CTACTACGAA GGGGTTGCCG TCCTGGGTAC 6840
   CTACTCCAAC CATACCTCTG CTCCAGCCAA CTGCTCCGTG GCCTCCCAAC ACAAGTTGAC 6900
   CCTGTCCGAA GTGACCGGAC AGGGACTCTG CATAGGAGCA GTTCCCAAAA CACATCAGGC 6960
   CCTATGTAAT ACCACCCAGA CAAGCAGTCG AGGGTCCTAT TATCTAGTTG CCCCTACAGG 7020
   TACCATGTGG GCTTGTAGTA CCGGGCTTAC TCCATGCATC TCCACCACCA TACTGAACCT 7080
   TACCACTGAT TATTGTGTTC TTGTCGAACT CTGGCCAAGA GTCACCTATC ATTCCCCCAG 7140
   CTATGTTTAC GGCCTGTTTG AGAGATCCAA CCGACACAAA AGAGAACCGG TGTCGTTAAC 7200
   CCTGGCCCTA TTATTGGGTG GACTAACCAT GGGGGGAATT GCCGCTGGAA TAGGAACAGG 7260
   GACTACTGCT CTAATGGCCA CTCAGCAATT CCAGCAGCTC CAAGCCGCAG TACAGGATGA 7320
   TCTCAGGGAG GTTGAAAAAT CAATCTCTAA CCTAGAAAAG TCTCTCACTT CCCTGTCTGA 7380
   AGTTGTCCTA CAGAATCGAA GGGGCCTAGA CTTGTTATTT CTAAAAGAAG GAGGGCTGTG 7440
   TGCTGCTCTA AAAGAAGAAT GTTGCTTCTA TGCGGACCAC ACAGGACTAG TGAGAGACAG 7500
   CATGGCCAAA TTGAGAGAGA GGCTTAATCA GAGACAGAAA CTGTTTGAGT CAACTCAAGG 7560
   ATGGTTTGAG GGACTGTTTA ACAGATCCCC TTGGTTTACC ACCTTGATAT CTACCATTAT 7620
   GGGACCCCTC ATTGTACTCC TAATGATTTT GCTCTTCGGA CCCTGCATTC TTAATCGATT 7680
   AGTCCAATTT GTTAAAGACA GGATATCAGT GGTCCAGGCT CTAGTTTTGA CTCAACAATA 7740
   TCACCAGCTG AAGCCTATAG AGTACGAGCC ATAGATAAAA TAAAAGATTT TATTTAGTCT 7800
   CCAGAAAAAG GGGGGAATGA AAGACCCCAC CTGTAGGTTT GGCAAGCTAG CTTAAGTAAC 7860
   GCCATTTTGC AAGGCATGGA AAAATACATA ACTGAGAATA GAGAAGTTCA GATCAAGGTC 7920
   AGGAACAGAT GGAACAGCTG AATATGGGCC AAACAGGATA TCTGTGGTAA GCAGTTCCTG 7980
   CCCCGGCTCA GGGCCAAGAA CAGATGGAAC AGCTGAATAT GGGCCAAACA GGATATCTGT 8040
   GGTAAGCAGT TCCTGCCCCG GCTCAGGGCC AAGAACAGAT GGTCCCCAGA TGCGGTCCAG 8100
   CCCTCAGCAG TTTCTAGAGA ACCATCAGAT GTTTCCAGGG TGCCCCAAGG ACCTGAAATG 8160
   ACCCTGTGCC TTATTTGAAC TAACCAATCA GTTCGCTTCT CGCTTCTGTT CGCGCGCTTC 8220
   TGCTCCCCGA GCTCAATAAA AGAGCCCACA ACCCCTCACT CGGGGCGCCA GTCCTCCGAT 8280
   TGACTGAGTC GCCCGGGTAC CCGTGTATCC AATAAACCCT CTTGCAGTTG CA 8332
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:2:
   GGTAACAGTC TGGCCCGAAT TCTCAGACAA ATACAG 36
2) INFORMATION FOR SEQ ID NO:3:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:3:
   CTGTATTTGT CTGAGAATTA AGGCTAGACT GTTACCAC 38
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:4:
   ATATATATAT ATCGATACCA TG 22
(2) INFORMATION FOR SEQ ID NO:5:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:5:
   GGCGCCAAAC CTAAAC 16
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (xi) SEQUENCE DESCRIPTION: SEQ ID NO:6:
   Ser Lys Asn Tyr Pro 5
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:7:
   ACCATCCTCT GGACGGACAT G 21
(2) INFORMATION FOR SEQ ID N0:8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:8:
(2) INFORMATION FOR SEQ ID N0:9:
   (i) SEQUENCE CHARACTERISTICS:
      (A) LENGTH: 449 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 20..439
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 140 amino acids
      (B) TYPE: amino acid
      (D) TOPOLOGY: linear
   (xi) SEQUENCE DESCRIPTION: SEQ ID NO:10:
   Met Gly Gln Thr Val Thr Thr Pro Leu Ser Leu Thr Leu Gly His Trp
   1 5 10 15
   Lys Asp Val Glu Arg Ile Ala His Asn Gln Ser Val Asp Cys Lys Lys
   20 25 30
   Arg Arg Trp Val Thr Phe Cys Ser Ala Glu Trp Pro Thr Phe Asn Val
   35 40 45
   Gly Trp Pro Arg Asp Gly Thr Phe Asn Arg Asp Leu Ile Thr Gln Val
   50 55 60
   Lys Ile Lys Val Phe Ser Pro Gly Pro His Gly His Pro Asp Gln Val
   65 70 75 80
   Pro Tyr Ile Val Thr Trp Glu Ala Leu Ala Phe Asp Pro Pro Pro Trp
   85 90 95
   Val Lys Pro Phe Val His Pro Lys Pro Pro Pro Pro Leu Pro Pro Ser
   100 105 110
   Ala Pro Ser Leu Pro Leu Glu Pro Pro Arg Ser Thr Pro Pro Arg Ser
   115 120 125
   Ser Leu Tyr Pro Ala Leu Thr Pro Ser Leu Gly Ala
   130 135 140
(2) INFORMATION FOR SEQ ID NO:11:
   (i) SEQUENCE CHARACTERISTICS:
      (A) LENGTH: 420 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..420
   (xi) SEQUENCE DESCRIPTION: SEQ ID NO:11:
   ATG GGC CAG ACT GTT ACC ACT CCC TTA AGT TTG ACC TTA GGT CAC TGG        48
   Met Gly Gln Thr Val Thr Thr Pro Leu Ser Leu Thr Leu Gly His Trp
     1               5                  10                  15
   AAA GAT GTC GAG CGG ATC GCT CAC AAC CAG TCG GTA GAT GTC AAG AAG        96
   Lys Asp Val Glu Arg Ile Ala His Asn Gln Ser Val Asp Val Lys Lys
                20                  25                  30
   AGA CGT TGG GTT ACC TTC TGC TCT GCA GAA TGG CCA ACC TTT AAC GTC       144
   Arg Arg Trp Val Thr Phe Cys Ser Ala Glu Trp Pro Thr Phe Asn Val
            35                  40                  45
   GGA TGG CCG CGA GAC GGC ACC TTT AAC CGA GAC CTC ATC ACC CAG GTT       192
   Gly Trp Pro Arg Asp Gly Thr Phe Asn Arg Asp Leu Ile Thr Gln Val
        50                  55                  60
   AAG ATC AAG GTC TTT TCA CCT GGC CCG CAT GGA CAC CCA GAC CAG GTC       240
   Lys Ile Lys Val Phe Ser Pro Gly Pro His Gly His Pro Asp Gln Val
    65                  70                  75                  80
   CCC TAC ATC GTG ACC TGG GAA GCC TTG GCT TTT GAC CCC CCT CCC TGG       288
   Pro Tyr Ile Val Thr Trp Glu Ala Leu Ala Phe Asp Pro Pro Pro Trp
                    85                  90                  95
   GTC AAG CCC TTT GTA CAC CCT AAG CCT CCG CCT CCT CTT CCT CCA TCC       336
   Val Lys Pro Phe Val His Pro Lys Pro Pro Pro Pro Leu Pro Pro Ser
               100                 105                 110
   GCC CCG TCT CTC CCC CTT GAA CCT CCT CGT TCG ACC CCG CCT CGA TCC       384
   Ala Pro Ser Leu Pro Leu Glu Pro Pro Arg Ser Thr Pro Pro Arg Ser
           115                 120                 125
   TCC CTT TAT CCA GCC CTC ACT CCT TCT CTA GGC GCC                       420
   Ser Leu Tyr Pro Ala Leu Thr Pro Ser Leu Gly Ala
       130                 135                 140
(2) INFORMATION FOR SEQ ID NO:12:
   (i) SEQUENCE CHARACTERISTICS:
      (A) LENGTH: 140 amino acids
      (B) TYPE: amino acid
      (D) TOPOLOGY: linear
   (xi) SEQUENCE DESCRIPTION: SEQ ID NO:12:
   Met Gly Gln Thr Val Thr Thr Pro Leu Ser Leu Thr Leu Gly His Trp
    1               5                  10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
           35                  40                  45
   Gly Trp Pro Arg Asp Gly Thr Phe Asn Arg Asp Leu Ile Thr Gln Val
       50                  55                  60
   Lys Ile Lys Val Phe Ser Pro Gly Pro His Gly His Pro Asp Gln Val
   65                  70                  75                  80
   Pro Tyr Ile Val Thr Trp Glu Ala Leu Ala Phe Asp Pro Pro Pro Trp
                   85                  90                  95
   Val Lys Pro Phe Val His Pro Lys Pro Pro Pro Pro Leu Pro Pro Ser
               100                 105                 110
   Ala Pro Ser Leu Pro Leu Glu Pro Pro Arg Ser Thr Pro Pro Arg Ser
           115                 120                 125
   Ser Leu Tyr Pro Ala Leu Thr Pro Ser Leu Gly Ala
       130                 135                 140
(2) INFORMATION FOR SEQ ID NO:13:
   (i) SEQUENCE CHARACTERISTICS:
      (A) LENGTH: 2001 base pairs
      (B) TYPE: nucleic acid
      (C) STRANDEDNESS: single
      (D) TOPOLOGY: linear
   (xi) SEQUENCE DESCRIPTION: SEQ ID NO:13:
   GGCCGACACC CAGAGTGGAC CATCCTCTGG ACGGACATGG CGCGTTCAAC GCTCTCAAAA    60
   CCCCCTCAAG ATAAGATTAA CCCGTGGAAG CCCTTAATAG TCATGGGAGT CCTGTTAGGA   120
   GTAGGGATGG CAGAGAGCCC CCATCAGGTC TTTAATGTAA CCTGGAGAGT CACCAACCTG   180
   ATGACTGGGC GTACCGCCAA TGCCACCTCC CTCCTGGGAA CTGTACAAGA TGCCTTCCCA   240
   AAATTATATT TTGATCTATG TGATCTGGTC GGAGAGGAGT GGGACCCTTC AGACCAGGAA   300
   CCGTATGTCG GGTATGGCTG CAAGTACCCC GCAGGGAGAC AGCGGACCCG GACTTTTGAC   360
   TTTTACGTGT GCCCTGGGCA TACCGTAAAG TCGGGGTGTG GGGGACCAGG AGAGGGCTAC   420
   TGTGGTAAAT GGGGGTGTGA AACCACCGGA CAGGCTTACT GGAAGCCCAC ATCATCGTGG   480
   GACCTAATCT CCCTTAAGCG CGGTAACACC CCCTGGGACA CGGGATGCTC TAAAGTTGCC   540
   TGTGGCCCCT GCTACGACCT CTCCAAAGTA TCCAATTCCT TCCAAGGGGC TACTCGAGGG   600
   GGCAGATGCA ACCCTCTAGT CCTAGAATTC ACTGATGCAG GAAAAAAGGC TAACTGGGAC   660
   GGGCCCAAAT CGTGGGGACT GAGACTGTAC CGGACAGGAA CAGATCCTAT TACCATGTTC 720
   TCCCTGACCC GGCAGGTCCT TAATGTGGGA CCCCGAGTCC CCATAGGGCC CAACCCAGTA 780
   TTACCCGACC AAAGACTCCC TTCCTCACCA ATAGAGATTG TACCGGCTCC ACAGCCACCT 840
   AGCCCCCTCA ATACCAGTTA CCCCCCTTCC ACTACCAGTA CACCCTCAAC CTCCCCTACA 900
   AGTCCAAGTG TCCCACAGCC ACCCCCAGGA ACTGGAGATA GACTACTAGC TCTAGTCAAA 960
   GGAGCCTATC AGGCGCTTAA CCTCACCAAT CCCGACAAGA CCCAAGAATG TTGGCTGTGC 1020
   TTAGTGTCGG GACCTCCTTA TTACGAAGGA GTAGCGGTCG TGGGCACTTA TACCAATCAT 1080
   TCCACCGCTC CGGCCAACTG TACGGCCACT TCCCAACATA AGCTTACCCT ATCTGAAGTG 1140
   ACAGGACAGG GCCTATGCAT GGGGGCAGTA CCTAAAACTC ACCAGGCCTT ATGTAACACC 1200
   ACCCAAAGCG CCGGCTCAGG ATCCTACTAC CTTGCAGCAC CCGCCGGAAC AATGTGGGCT 1260
   TGCAGCACTG GATTGACTCC CTGCTTGTCC ACCACGGTGC TCAATCTAAC CACAGATTAT 1320
   TGTGTATTAG TTGAACTCTG GCCCAGAGTA ATTTACCACT CCCCCGATTA TATGTATGGT 1380
   CAGCTTGAAC AGCGTACCAA ATATAAAAGA GAGCCAGTAT CATTGACCCT GGCCCTTCTA 1440
   CTAGGAGGAT TAACCATGGG AGGGATTGCA GCTGGAATAG GGACGGGGAC CACTGCCTTA 1500
   ATTAAAACCC AGCAGTTTGA GCAGCTTCAT GCCGCTATCC AGACAGACCT CAACGAAGTC 1560
   GAAAAGTCAA TTACCAACCT AGAAAAGTCA CTGACCTCGT TGTCTGAAGT AGTCCTACAG 1620
   AACCGCAGAG GCCTAGATTT GCTATTCCTA AAGGAGGGAG GTCTCTGCGC AGCCCTAAAA 1680
   GAAGAATGTT GTTTTTATGC AGACCACACG GGGCTAGTGA GAGACAGCAT GGCCAAATTA 1740
   AGAGAAAGGC TTAATCAGAG ACAAAAACTA TTTGAGACAG GCCAAGGATG GTTCGAAGGG 1800
   CTGTTTAATA GATCCCCCTG GTTTACCACC TTAATCTCCA CCATCATGGG ACCTCTAATA 1860
   GTACTCTTAC TGATCTTACT CTTTGGACCT TGCATTCTCA ATCGATTGGT CCAATTTGTT 1920
   AAAGACAGGA TCTCAGTGGT CCAGGCTCTG GTTTTGACTC AGCAATATCA CCAGCTAAAA 1980
   CCCATAGAGT ACGAGCCATG A 2001
(2) INFORMATION FOR SEQ ID NO:14:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:14:
   CTAGCTAGCT AG 12
(2) INFORMATION FOR SEQ ID NO:15:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (xi) SEQUENCE DESCRIPTION: SEQ ID NO:15:
   ATATATATAT ATCGATACCA TGGGGCAAAC CGTGACTACC CCTCTGTCCC TCACACTGGC 60
   CCAA 64
(2) INFORMATION FOR SEQ ID NO:16:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO:16:
   TTGATTATGG GCATTTCTTT CCACGTCCTT CCAATGGCCC AGTGTGAGGG A 51
(2) INFORMATION FOR SEQ ID NO:17:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:17:
   AGAATTGCCC ATAATCAAAG CGTGGACGTC AAAAAACGCA GGTGGGTGAC ATTTTGTAGC 60
   GCCGAGTGGC CC 72
(2) INFORMATION FOR SEQ ID NO:18:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:18:
   AAGTTCCATC CCTAGGCCAG CCAACATTGA ATGTGGGCCA CTCGGCGCTA CA 52
(2) INFORMATION FOR SEQ ID NO:19:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (xi) SEQUENCE DESCRIPTION: SEQ ID NO:19:
   GGCCTAGGGA GGAACTTTCA ATCGCGATCT GATTACTCAA GTGAAAATTA AAGTGTTCAG 60
   CCCCGGACCC C 71
(2) INFORMATION FOR SEQ ID NO:20:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:20:
   GTGACAATAT AAGGAACTTG ATCGGGATGG CCGTGGGGTC CGGGGCTGAA CA 52
(2) INFORMATION FOR SEQ ID NO:21:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:21:
   AGTTCCTTAT ATTGTCACAT CGGAGGCTCT CGCTTTCGAT CCACCACCTT GGGTGAAACC 60
   ATTCGTGCAT CC 72
(2) INFORMATION FOR SEQ ID N0:22:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:22
(2) INFORMATION FOR SEQ ID NO:23
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:23:
   CTCCCACCCA GCGCTCCTAG CCTGCCCTTG GAGCCCCCAC GAAGCACACC ACCCAGGAGC 60
   AGCTTGTACC CT 72
(2) INFORMATION FOR SEQ ID NO:24:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:24:
   GTTTAGGTTT GGCGCCGAGG CTGGGGGTCA GAGCAGGGTA CAAGCTGCTC CT 52
(2) INFORMATION FOR SEQ ID NO:25:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:25:
   ATATATATAT ATCGATACC 19
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:26:
   GTTTAGGTTT GGCGCCGAGG 20